# EUROPEAN PATENT SPECIFICATION

(11) **EP 2 734 626 B1**
(45) Date of publication and mention of the grant of the patent: **16.12.2020**
(21) Application number: 12815593.4
(22) Date of filing: 20.07.2012
(51) Int. Cl.: C11B 1/10, A23K 20/158, A23L 7/104, A23L 31/00, A23L 5/00, A61P 9/00, A61K 31/202, A61K 31/232, A21D 2/16, C12R 1/89, C11B 3/04, C11B 3/06, C11B 3/10, C12P 7/64, C11C 3/10, A23D 9/00, A23L 33/115

(54) **MICROBIAL OILS ENRICHED IN POLYUNSATURATED FATTY ACIDS**
IN MEHRFACH UNGESÄTTIGTEN FETTSÄUREN ANGEREICHERTE MIKROBIELLE ÖLE
HUILES MICROBIENNES ENRICHIES EN ACIDES GRAS POLYINSATURÉS

(30) Priority: 21.07.2011 US 201161510454 P
(43) Date of publication of application: 28.05.2014
(73) Proprietor: DSM IP Assets B.V., 6411 TE Heerlen (NL)
(72) Inventor: RAMAN, Krishna, Wilmington, Delaware 19810 (US)
(74) Representative: DSM Intellectual Property
(86) International application number: PCT/US2012/047730
(87) International publication number: WO 2013/013210

(56) References cited:
- WO-A2-2007/068997
- WO-A2-2012/109545
- US-A- 5 130 242
- US-A1- 2002 026 063
- US-A1- 2006 115 881
- US-A1- 2008 175 975
- US-A1- 2009 093 543
- US-A1- 2010 239 533
- US-A1- 2010 266 564
- US-B1- 6 395 778

## Description

### REFERENCE TO A SEQUENCE LISTING SUBMITTED ELECTRONICALLY

The content of the electronically submitted sequence listing ("sequence listing.txt", 5,074 bytes, created on July 20, 2011) filed with the application is incorporated herein by reference in its entirety.

### BACKGROUND OF THE INVENTION

### Field of the Invention

This application discloses microbial oils that are enriched in polyunsaturated fatty acids, their esters, their acid salts, their alcohols, and/or their aldehydes; compositions containing enriched microbial oils; and methods of making and using the enriched microbial oils.

The present invention is directed methods of using the oils.

### Background Art

Fatty acids are classified based on the length and saturation characteristics of the carbon chain. Fatty acids are termed short chain, medium chain, or long chain fatty acids based on the number of carbons present in the chain, are termed saturated fatty acids when no double bonds are present between the carbon atoms, and are termed unsaturated fatty acids when double bonds are present. Unsaturated long chain fatty acids are monounsaturated when only one double bond is present and are polyunsaturated when more than one double bond is present.

Polyunsaturated fatty acids (PUFAs) are classified based on the position of the first double bond from the methyl end of the fatty acid: omega-3 (n-3) fatty acids contain a first double bond at the third carbon, while omega-6 (n-6) fatty acids contain a first double bond at the sixth carbon. For example, docosahexaenoic acid ("DHA") is an omega-3 long chain polyunsaturated fatty acid (LC-PUFA) with a chain length of 22 carbons and 6 double bonds, often designated as "22:6 n-3." Other omega-3 LC-PUFAs include eicosapentaenoic acid ("EPA"), designated as "20:5 n-3," and omega-3 docosapentaenoic acid ("DPA n-3"), designated as "22:5 n-3." DHA and EPA have been termed "essential" fatty acids. Omega-6 LC-PUFAs include arachidonic acid ("ARA"), designated as "20:4 n-6," and omega-6 docosapentaenoic acid ("DPA n-6"), designated as "22:5 n-6."

Omega-3 fatty acids are biologically important molecules that affect cellular physiology due to their presence in cell membranes, regulate production and gene expression of biologically active compounds, and serve as biosynthetic substrates. Roche, H. M., Proc. Nutr. Soc. 58: 397-401 (1999). DHA, for example, accounts for approximately 15%-20% of lipids in the human cerebral cortex, 30%-60% of lipids in the retina, is concentrated in the testes and sperm, and is an important component of breast milk. Berge, J.P., and Barnathan, G.. Adv. Biochem. Eng. Biotechnol. 96:49-125 (2005). DHA accounts for up to 97% of the omega-3 fatty acids in the brain and up to 93% of the omega-3 fatty acids in the retina. Moreover, DHA is essential for both fetal and infant development as well as maintenance of cognitive functions in adults. *Id.* Because omega-3 fatty acids are not synthesized de novo in the human body, these fatty acids must be derived from nutritional sources.

Flaxseed oil and fish oils are considered good dietary sources of omega-3 fatty acids. Flaxseed oil contains no EPA, DHA, DPA, or ARA but rather contains linolenic acid (C18:3 n-3), a building block enabling the body to manufacture EPA. There is evidence, however, that the rate of metabolic conversion can be slow and variable, particularly among those with impaired health. Fish oils vary considerably in the type and level of fatty acid composition depending on the particular species and their diets. For example, fish raised by aquaculture tend to have a lower level of omega-3 fatty acids than those in the wild. Furthermore, fish oils carry the risk of containing environmental contaminants and can be associated with stability problems and a fishy odor or taste.

Thraustochytrids are microorganisms of the order Thraustochytriales. Thraustochytrids include members of the genus *Schizochytrium* and *Thraustochytrium* and have been recognized as an alternative source of omega-3 fatty acids, including DHA and EPA. *See* U.S. Patent No. 5,130,242. Oils produced from these marine heterotrophic microorganisms often have simpler polyunsaturated fatty acid profiles than corresponding fish or microalgal oils. Lewis, T.E., Mar. Biotechnol. 1: 580-587 (1999). Strains of thraustochytrid species have been reported to produce omega-3 fatty acids as a high percentage of the total fatty acids produced by the organisms. U.S. Patent No. 5,130,242; Huang, J. et al., J. Am. Oil. Chem. Soc. 78: 605-610 (2001); Huang, J. et al., Mar. Biotechnol. 5: 450-457 (2003). However, isolated thraustochytrids vary in the identity and amounts of LC-PUFAs produced, such that some previously described strains can have undesirable levels of omega-6 fatty acids and/or can demonstrate low productivity in culture. As such, a continuing need exists for the isolation of microorganisms demonstrating high productivity and desirable LC-PUFA profiles.

Furthermore, there is unmet need for enriched microbial oils that contain higher concentrations of polyunsaturated fatty acids, their esters, their aldehydes, their acid salts, and/or their alcohols, then are found in previous microbial oils.

WO 2012/109545 describes methods for pelletizing a microbial biomass, extracting a refined lipid composition from the pelletized biomass under supercritical conditions and distilling the refined lipid composition, at least once under short path distillation conditions, to obtain a lipid-containing fraction. Also disclosed are methods of making lipid-containing oil concentrates therefrom, by transesterifying and enriching the lipid-containing fraction.

US 2009/093543 describes engineered strains of the oleaginous yeast Yarrowia lipolytica capable of producing greater than 50 weight percent of eicosapentaenoic acid in the total oil fraction.

US 2008/175975 describes a method for producing a fatty acid composition which, based on the entire weight of the fatty acids and/or fatty acid derivatives contained in the fatty acid composition, contains at least 70.0% by weight of docosahexaenoic acid and/or docosahexaenoic alkyl ester.

### BRIEF SUMMARY OF THE INVENTION

The present invention is directed to an enriched microbial oil comprising at least 70% by weight of a combination of DHA and EPA, wherein the oil comprises at least 10% by weight EPA and wherein (i) the oil comprises at least 50% by weight DHA and at least 20% by weight EPA: or (ii) the oil comprises a triacylglycerol fraction, wherein at least 50% by weight of the fatty acids in the triacylglycerol fraction is DHA and at least 20% by weight of the fatty acids in the triacylglycerol fraction is EPA. In some embodiments, the DHA and EPA are in an ester form. For example, the DHA and EPA ester can be an ethyl ester.

The present invention is directed to a food, supplement, or pharmaceutical composition comprising an enriched microbial oil of the invention. The pharmaceutical composition can contain a pharmaceutically acceptable carrier.

In some embodiments, the enriched microbial oil is obtainable from an isolated microorganism of a *Schizochytrium* species.

In some embodiments, the oil of the invention is obtainable from an isolated microorganism of the species deposited under ATCC Accession No. PTA-10208, wherein the total fatty acids produced by the microorganism comprise more than 10% by weight EPA.

In some embodiments, the oil of the invention is obtainable from an isolated microorganism having the characteristics of the species deposited under ATCC Accession No. PTA-10208, wherein the total fatty acids produced by the microorganism comprise more than about 10% by weight EPA.

In some embodiments, the oil of the invention is obtainable from an isolated microorganism that produces a triacylglycerol fraction, wherein EPA content of the triacylglycerol fraction is at least about 12% by weight.

In some embodiments, the oil of the invention is obtainable from an isolated microorganism deposited under ATCC Accession No. PTA-10208.

In some embodiments, the oil of the invention is obtainable from an isolated microorganism deposited under ATCC Accession No. PTA-10209.

In some embodiments, the oil of the invention is obtainable from an isolated microorganism deposited under ATCC Accession No. PTA-10210.

In some embodiments, the oil of the invention is obtainable from an isolated microorganism deposited under ATCC Accession No. PTA-10211.

The present invention is directed to an oral dosage form comprising the enriched microbial oils of the invention. In some embodiments, the oral dosage comprises 100 mg to 3000 mg of omega-3 polyunsaturated fatty acids. In some embodiments, the oral dosage comprises 500 mg to 1000 mg of a combination of DHA and EPA. In some embodiments, the oral dosage form is a capsule. In some embodiments, the oral dosage form is a vegetarian capsule.

The present invention is directed to a method for treating a heart disease or a condition related thereto in a subject in need thereof, comprising administering to the subject an oil of the invention, or a mixture of the oils of the invention, and a pharmaceutically acceptable carrier.

The present invention is directed to the use of the oil of the invention, or mixtures of the oils of the invention, for the manufacture of a medicament for treatment of a heart disease or a condition related thereto.

The present invention is directed to the use of the oil of the invention, or mixtures of the oils of the invention, for the manufacture of a supplement for improving a heart condition or a condition related thereto, or maintaining a healthy heart condition or a condition related thereto.

The present invention is directed to a method for producing the enriched microbial oils of the invention from the biomass of a microorganism, comprising the steps of: a) heating and reacting the biomass in the presence of an alcohol and a base to produce an ester of a polyunsaturated fatty acid from the biomass; b) separating the biomass into a substantially liquid phase, enriched with polyunsaturated fatty acid, and a substantially solidphase; and c) purifying the substantially liquid phase by recovering a fraction comprising the ester of polyunsaturated fatty acid.

In some embodiments, the microorganism is an isolated microorganism of a *Schizochytrium* species.

In some embodiments, the microorganism is any one of isolated microorganisms of the species deposited under ATCC Accession Nos.: PTA-10212, PTA-10213, PTA-10214, PTA-10215, PTA-10208, PTA-10209, PTA-10210, PTA-10211, a mutant strain thereof, or a mixture thereof.

In some embodiments, the heating step a) is conducted from about 80 °C to 100 °C.

In some embodiments, the heating step a) is conducted at 90 °C.

In some embodiments, the base is potassium hydroxide.

In some embodiments, the alcohol is ethanol.

In some embodiments, the ester is an ethyl ester of the polyunsatured fatty acid.

In some embodiments, the polyunsatured fatty acid is a combination of DHA and EPA.

In some embodiments, the separating step b) is conducted by centrifugation or filtration.

In some embodiments, the purifying step c) is conducted by urea adduction crystallization in ethanol.

In some embodiments, the purifying step c) is conducted by vacuum molecular fractional distillation.

There is further disclosed an isolated microorganism of the species deposited under ATCC Accession No. PTA-10212.

There is further disclosed an isolated microorganism having the characteristics of the species deposited under ATCC Accession No. PTA-10212.

There is further disclosed an isolated microorganism comprising an 18s rRNA comprising a polynucleotide sequence of SEQ ID NO: 1 or a polynucleotide sequence having at least 94% identity to SEQ ID NO:1.

There is further disclosed an isolated microorganism comprising an 18s rRNA polynucleotide sequence that has at least 94% identity to an 18s rRNA polynucleotide sequence of the microorganism deposited under ATCC Accession No. PTA-10212.

There is further disclosed an isolated microorganism of the species deposited under ATCC Accession No. PTA-10208, wherein the total fatty acids produced by the microorganism comprises more than about 10% by weight eicosapentaenoic acid.

There is further disclosed an isolated microorganism having the characteristics of the species deposited under ATCC Accession No. PTA-10208, wherein the total fatty acids produced by the microorganism comprises more than about 10% by weight eicosapentaenoic acid.

There is further disclosed an isolated microorganism that produces a triacylglycerol fraction, wherein eicosapentaenoic acid content of the triacylglycerol fraction is at least about 12% by weight.

In some embodiments, the isolated microorganism is a mutant strain.

There is further disclosed an isolated microorganism deposited under ATCC Accession No. PTA-10212, PTA-10213, PTA-10214, PTA-10215, PTA-10208, PTA-10209, PTA-10210, or PTA-10211.

There is further disclosed a biomass comprising any of the microorganisms of the invention or mixtures thereof.

There is further disclosed an isolated biomass, wherein at least about 20% by weight of a dry cell weight of the biomass are fatty acids, wherein more than about 10% by weight of fatty acids is eicosapentaenoic acid, and wherein the fatty acids comprise less than about 5% by weight each of arachidonic acid and docosapentaenoic acid n-6. In some embodiments, at least about 25% by weight of the fatty acids is docosahexaenoic acid.

There is further disclosed an isolated biomass comprising triacylglycerol, wherein at least about 12% by weight of triacylglycerol is eicosapentaenoic acid.

There are further disclosed any of the isolated biomasses as described herein wherein the fatty acids further comprise less than about 5% by weight each of oleic acid, linoleic acid, linolenic acid, eicosenoic acid, and erucic acid.

There is further disclosed an isolated culture comprising any of the microorganisms of the invention or mixtures thereof.

The present invention is directed to a food product, cosmetic, or pharmaceutical composition for a non-human animal or human, comprising any of the microorganisms or biomasses of the invention or mixtures thereof.

There is further disclosed a microbial oil comprising at least about 20% by weight eicosapentaenoic acid and less than about 5% by weight each of arachidonic acid, docosapentaenoic acid n-6, oleic acid, linoleic acid, linolenic acid, eicosenoic acid, erucic acid, and stearidonic acid. In some embodiments, the microbial oil further comprises at least about 25% by weight docosahexaenoic acid.

There is further disclosed a microbial oil comprising a triacylglycerol fraction of at least about 10% by weight, wherein at least about 12% by weight of the fatty acids in the triacylglycerol fraction is eicosapentaenoic acid, wherein at least about 25% by weight of the fatty acids in the triacylglycerol fraction is docosahexaenoic acid, and wherein less than about 5% by weight of the fatty acids in the triacylglycerol fraction is arachidonic acid.

The present invention is directed to a food product, cosmetic, or pharmaceutical composition for a non-human animal or human, comprising any of the microbial oils of the invention. In some embodiments, the food product is an infant formula. In some embodiments, the infant formula is suitable for premature infants. In some embodiments, the food product is a milk, a beverage, a therapeutic drink, a nutritional drink, or a combination thereof. In some embodiments, the food product is an additive for the non-human animal or human food. In some embodiments, the food product is a nutritional supplement. In some embodiments, the food product is an animal feed. In some embodiments, the animal feed is an aquaculture feed. In some embodiments, the animal feed is a domestic animal feed, a zoological animal feed, a work animal feed, a livestock feed, or a combination thereof.

There is further disclosed a method for producing a microbial oil comprising omega-3 fatty acids, the method comprising: growing any of the isolated microorganisms of the invention or mixtures thereof in a culture to produce an oil comprising omega-3 fatty acids. In some embodiments, the method further comprises extracting the oil.

There is further disclosed a method for producing a microbial oil comprising omega-3 fatty acids, the method comprising extracting an oil comprising omega-3 fatty acids from any of the biomasses of the invention. In some embodiments, the microbial oil is extracted using an organic solvent extraction process, for example hexane extraction. In some embodiments, the microbial oil is extracted using a solventless extraction process.

There is further disclosed a microbial oil produced by a method as disclosed herein.

There is further disclosed a method for producing a biomass of the invention, comprising: growing any of the isolated microorganisms of the invention or mixtures thereof in a culture to produce a biomass.

There is further disclosed a biomass produced by a method of the invention.

There is further disclosed a method for producing a mutant strain of the invention, comprising: mutagenizing any of the microorganisms of the invention, and isolating the mutant strain.

There is further disclosed the use of any of the isolated microorganisms, biomasses, or microbial oils as described herein. or mixtures thereof, for the manufacture of a medicament for treatment of inflammation or a condition related thereto.

There is further disclosed the use of any of the isolated microorganisms, biomasses, or microbial oils of the inventionas described herein. or mixtures thereof, for treatment of inflammation or a condition related thereto.

There is further disclosed any of the isolated microorganisms, biomasses, or microbial oils as described herein, or mixtures thereof, for use in treatment of inflammation or a condition related thereto.

There is further disclosed a method for treating inflammation or a condition related thereto in a subject in need thereof, comprising administering to the subject any of the isolated microorganisms, biomasses, or microbial oils as described herein, or mixtures thereof, and a pharmaceutically acceptable carrier.

### BRIEF DESCRIPTION OF THE DRAWINGS

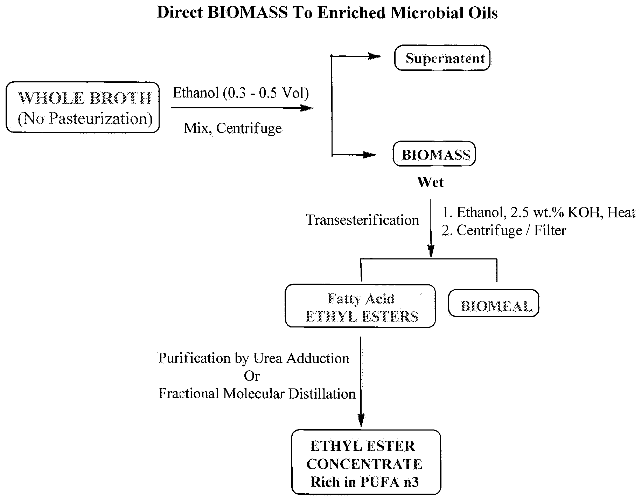

FIG. 1 is a schematic diagram of an exemplary embodiment of a biomass separation process and a microbial oil enrichment process according to the present disclosure.

### DETAILED DESCRIPTION OF THE INVENTION

### Microorganisms

Disclosed herein are isolated microorganisms and strains derived therefrom. A strain that is "derived" from an isolated microorganism of the invention can be a natural or artificial derivative such as, for example, a mutant, variant, or recombinant strain. The term "isolated" as used herein does not necessarily reflect the extent to which an isolate has been purified, but indicates isolation or separation from a native form or native environment. An isolate can include, but is not limited to, an isolated microorganism, an isolated biomass, an isolated culture, an isolated microbial oil, and an isolated sequence (such as an isolated polynucleotide sequence disclosed herein). The term "microorganism" as used herein includes, but is not limited to, the terms "microalgae," "thraustochytrid," and taxonomic classifications associated with any of the deposited microorganisms described herein. The terms "Thraustochytriales," "thraustochytrid," *"Schizochytrium,"* and *"Thraustochytrium"* as used in reference to any of the microorganisms of the invention, including the deposited microorganisms described herein, are based on present taxonomic classifications including available phylogenetic information and are not intended to be limiting in the event that the taxonomic classifications are revised after the filing date of the present application.

There is further disclosed an isolated microorganism of the species deposited under ATCC Accession No. PTA-10212. The isolated microorganism associated with ATCC Accession No. PTA-10212 is also known herein as *Thraustochytrium sp.* ATCC PTA-10212. The isolated microorganism associated with ATCC Accession No. PTA-10212 was deposited under the Budapest Treaty on July 14, 2009 at the American Type Culture Collection, Patent Depository, 10801 University Boulevard, Manassas, VA 20110-2209. There is further disclosed an isolated strain deposited under ATCC Accession No. PTA-10212.

There is further disclosed an isolated microorganism having the characteristics of the species deposited under ATCC Accession No. PTA-10212 or a strain derived therefrom. The characteristics of the species deposited under ATCC Accession No. PTA-10212 can include its growth and phenotypic properties (examples of phenotypic properties include morphological and reproductive properties), its physical and chemical properties (such as dry weights and lipid profiles), its gene sequences, and combinations thereof, in which the characteristics distinguish the species over previously identified species. There is further disclosed an isolated microorganism having the characteristics of the species deposited under ATCC Accession No. PTA-10212, wherein the characteristics include an 18s rRNA comprising the polynucleotide sequence of SEQ ID NO:1 or a polynucleotide sequence having at least 94%, 95%, 96%, 97%, 98%, or 99% identity to SEQ ID NO:1, the morphological and reproductive properties of the species deposited under ATCC Accession No. PTA-10212, and the fatty acid profiles of the species deposited under ATCC Accession No. PTA-10212. In some embodiments, isolated microorganisms disclosed herein have phenotypic properties substantially identical to those of the microorganism deposited under ATCC Accession No. PTA-10212. In some embodiments, isolated microorganisms disclosed herein have growth properties substantially identical to those of the microorganism deposited under ATCC Accession No. PTA-10212. There is further disclosed an isolated microorganism comprising an 18s rRNA comprising the polynucleotide sequence of SEQ ID NO:1 or a polynucleotide sequence having at least 94%, 95%, 96%, 97%, 98%, or 99% identity to SEQ ID NO:1. There is further disclosed an isolated microorganism comprising an 18s rRNA polynucleotide sequence that has at least 94% identity to the 18s rRNA polynucleotide sequence of the microorganism deposited under ATCC Accession No. PTA-10212.

There is further disclosed a mutant strain of the microorganism deposited under ATCC Accession No. PTA-10212. In further embodiments, the mutant strain is a strain deposited under ATCC Accession No. PTA-10213, PTA-10214, or PTA-10215. The microorganisms associated with ATCC Accession Nos. PTA-10213, PTA-10214, and PTA-10215 were deposited under the Budapest Treaty on July 14, 2009 at the American Type Culture Collection, Patent Depository, 10801 University Boulevard, Manassas, VA 20110-2209.

There is further disclosed an isolated microorganism of the species deposited under ATCC Accession No. PTA-10208. The isolated microorganism associated with ATCC Accession No. PTA-10208 is also known herein as *Schizochytrium sp.* ATCC PTA-10208. The microorganism associated with ATCC Accession No. PTA-10208 was deposited under the Budapest Treaty on July 14, 2009 at the American Type Culture Collection, Patent Depository, 10801 University Boulevard, Manassas, VA 20110-2209 There is further disclosed an isolated strain deposited under ATCC Accession No. PTA-10208.

There is further disclosed an isolated microorganism of the species deposited under ATCC Accession No. PTA-10208, wherein the total fatty acids produced by the microorganism comprises more than about 10%, more than about 11%, more than about 12%, more than about 13%, more than about 14%, more than about 15%, more than about 16%, more than about 17%, more than about 18%, more than about 19%, or more than about 20% by weight EPA. There is further disclosed an isolated microorganism of the species deposited under ATCC Accession No. PTA-10208, wherein the total fatty acids produced by the microorganism comprises about 10% to about 55%, about 10% to about 50%, about 10% to about 45%, about 10% to about 40%, about 10% to about 35%, about 10% to about 30%, about 15% to about 55%, about 15% to about 50%, about 15% to about 45%, about 15% to about 40%, about 15% to about 35%, about 15% to about 30%, about 20% to about 55%, about 20% to about 50%, about 20% to about 45%, about 20% to about 40%, about 20% to about 35%, or about 20% to about 30% by weight EPA.

There is further disclosed an isolated microorganism having the characteristics of the species deposited under ATCC Accession No. PTA-10208, wherein the total fatty acids produced by the microorganism comprises more than about 10% by weight eicosapentaenoic acid. The characteristics of the microorganism deposited under ATCC Accession No. PTA-10208 include its growth and phenotypic properties (examples of phenotypic properties include morphological and reproductive properties), its physical and chemical properties (such as dry weights and lipid profiles), its gene sequences, and combinations thereof, in which the characteristics distinguish the species over previously identified species. There is further disclosed an isolated microorganism having the characteristics of the species deposited under ATCC Accession No. PTA-10212, wherein the characteristics include an 18s rRNA comprising the polynucleotide sequence of SEQ ID NO:2, the morphological and reproductive properties of the species deposited under ATCC Accession No. PTA-10208, and the fatty acid profiles of the species deposited under ATCC Accession No. PTA-10208. In some embodiments, isolated microorganisms as disclosed herein have physical and chemical properties substantially identical to those of the microorganism deposited under ATCC Accession No. PTA-10208.

There is further disclosed a mutant strain of the microorganism deposited under ATCC Accession No. PTA-10208. In further embodiments, the mutant strain is a strain deposited under ATCC Accession No. PTA-10209, PTA-10210, or PTA-10211. The microorganisms associated with ATCC Accession Nos. PTA-10209, PTA-10210, and PTA-10211 were deposited under the Budapest Treaty on September 25, 2009 at the American Type Culture Collection, Patent Depository, 10801 University Boulevard, Manassas, VA 20110-2209.

There is further disclosed an isolated microorganism disclosed herein that produces a triacylglycerol fraction, wherein the EPA content of the triacylglycerol fraction is at least about 12%, at least about 13%, at least about 14%, at least about 15%, at least about 16%, at least about 17%, at least about 18%, at least about 19%, or at least about 20% by weight. There is further disclosed an isolated microorganism that produces a triacylglycerol fraction, wherein the EPA content of the triacylglycerol fraction is about 12% to about 55%, about 12 % to about 50%, about 12% to about 45%, about 12% to about 40%, about 12% to about 35%, about 12% to about 30%, about 15% to about 45%, about 15% to about 40%, about 15% to about 35%, about 15% to about 30%, or about 20% to about 30% by weight.

There is further disclosed a mutant, variant, or recombinant of an isolated microorganism disclosed herein that produces a triacylglycerol fraction, wherein the EPA content of the triacylglycerol fraction is at least about 10%, at least about 11%, at least about 12%, at least about 13%, at least about 14%, at least about 15%, at least about 16%, at least about 17%, at least about 18%, at least about 19%, or at least about 20% by weight. There is further disclosed a mutant, variant, or recombinant of an isolated microorganism disclosed herein that produces a triacylglycerol fraction, wherein the EPA content of the triacylglycerol fraction is about 12% to about 55%, about 12% to about 50%, about 12% to about 45%, about 12% to about 40%, about 12% to about 35%, about 12% to about 30%, about 15% to about 55%, about 15% to about 50%, about 15% to about 45%, about 15% to about 40%, about 15% to about 35%, about 15% to about 30%, about 20% to about 55%, about 20% to about 50%, about 20% to about 45%, about 20% to about 40%, about 20% to about 35%, or about 20% to about 30% by weight. Mutant strains can be produced by well-known procedures. Common procedures include irradiation, treatment at high temperatures, and treatment with a mutagen. Variant strains can be other naturally occurring isolates and/or sub-isolates of the species described herein. Recombinant strains can be produced by any well-known methods in molecular biology for the expression of exogenous genes or alteration of endogenous gene function or expression. In some embodiments, the mutant, variant, or recombinant strain produces a higher amount of omega-3 fatty acids, particularly EPA, than the wild-type strain. In some embodiments, the mutant, variant, or recombinant strain produces a lower amount of one or more fatty acids, such as lower amounts of DHA, ARA, DPA n-6, or combinations thereof. In some embodiments, the mutant, variant, or recombinant strain produces a larger dry cell weight per liter of culture than the wild-type strain. Such mutant, variant, or recombinant strains are examples of strains derived from an isolated microorganism as disclosed herein.

In some embodiments, an isolated microorganism as disclosed herein, including mutants, variants, and recombinants thereof, comprises a fatty acid profile in one or more fractions isolated from the microorganism. The one or more fractions isolated from the microorganism include the total fatty acid fraction, the sterol esters fraction, the triacylglycerol fraction, the free fatty acid fraction, the sterol fraction, the diacylglycerol fraction, the polar fraction (including the phospholipid fraction), and combinations thereof. The fatty acid profile for a specific fraction can include any of the fatty acid profiles associated with the specific fraction as disclosed herein.

There is further disclosed a method of producing a mutant comprising mutagenizing any of the microorganisms as disclosed herein and isolating the mutant strain.

### Cultures and Isolated Biomasses

There is further disclosed a culture comprising one or more isolated microorganisms of the invention. Various fermentation parameters for inoculating, growing, and recovering microflora, such as microalgae and thraustochytrids, are known in the art. *See,* e.g., U.S. Patent No. 5,130,242. Liquid or solid media can contain natural or artificial sea water. Carbon sources for heterotrophic growth include, but are not limited to, glucose, fructose, xylose, saccharose, maltose, soluble starch, molasses, fucose, glucosamine, dextran, fats, oils, glycerol, sodium acetate, and mannitol. Nitrogen sources include, but are not limited to, peptone, yeast extract, polypeptone, malt extract, meat extract, casamino acid, corn steep liquor, organic nitrogen sources, sodium glutamate, urea, inorganic nitrogen sources, ammonium acetate, ammonium sulfate, ammonium chloride, and ammonium nitrate.

A typical media for growth of the microorganism deposited under ATCC Accession No. PTA-10212 is shown in Table 1:

**Table 1: PTA-10212 Vessel Media**

| Ingredient | concentration | | ranges |
|---|---|---|---|
| Na₂SO₄ | g/L | 31.0 | 0-50, 15-45, or 25-35 |
| NaCl | g/L | 0.625 | 0-25, 0.1-10, or 0.5-5 |
| KCl | g/L | 1.0 | 0-5, 0.25-3, or 0.5-2 |
| MgSO₄·7H₂O | g/L | 5.0 | 0-10, 2-8, or 3-6 |
| (NH₄)₂SO₄ | g/L | 0.44 | 0-10, 0.25-5, or 0.05-3 |
| MSG·1H₂O | g/L | 6.0 | 0-10, 4-8, or 5-7 |
| CaCl₂ | g/L | 0.29 | 0.1-5, 0.15-3, or 0.2-1 |
| T 154 (yeast extract) | g/L | 6.0 | 0-20, 0.1-10, or 1-7 |
| KH₂PO₄ | g/L | 0.8 | 0.1-10, 0.5-5, or 0.6-1.8 |

| Post autoclave (Metals) | | | |
|---|---|---|---|
| Citric acid | mg/L | 3.5 | 0.1-5000, 10-3000, or 3-2500 |
| FeSO₄·7H₂O | mg/L | 10.30 | 0.1-100, 1-50, or 5-25 |
| MnCl₂·4H₂O | mg/L | 3.10 | 0.1-100, 1-50, or 2-25 |
| ZnSO₄·7H₂O | mg/L | 3.10 | 0.01-100, 1-50, or 2-25 |
| COCl₂·6H₂O | mg/L | 0.04 | 0-1,0.001-0.1, or 0.01-0.1 |
| Na₂MoO₄·2H₂O | mg/L | 0.04 | 0.001-1, 0.005-0.5, or 0.01-0.1 |
| CuSO₄·5H₂O | mg/L | 2.07 | 0.1-100, 0.5-50, or 1-25 |
| NiSO₄·6H₂O | mg/L | 2.07 | 0.1-100, 0.5-50, or 1-25 |

| Post autoclave (Vitamins) | | | |
|---|---|---|---|
| Thiamine | mg/L | 9.75 | 0.1-100, 1-50, or 5-25 |
| Vitamin B12 | mg/L | 0.16 | 0.01-100, 0.05-5, or 0.1-1 |
| Ca½-pantothenate | mg/L | 2.06 | 0.1-100, 0.1-50, or 1-10 |
| Biotin | mg/L | 3.21 | 0.1-100, 0.1-50, or 1-10 |

| Post autoclave (Carbon) | | | |
|---|---|---|---|
| Glycerol | g/L | 30.0 | 5-150, 10-100, or 20-50 |

| Nitrogen Feed. | | | |
|---|---|---|---|
| Ingredient | Concentration | | |
| MSG.1H₂O | g/L | 17 | 0-150, 10-100, or 15-50 |
| Typical cultivation conditions would include the following: | | | |
| pH | about 6.5 - about 9.5, about 6.5 - about 8.0, or about 6.8 - about 7.8; | | |
| temperature: | about 15 - about 30 degrees Celsius, about 18 - about 28 degrees Celsius, or about 21 to about 23 degrees Celsius; | | |
| dissolved oxygen: | about 0.1 - about 100% saturation, about 5 - about 50% saturation, or about 10 - about 30% saturation; and/or | | |
| glycerol controlled @: | about 5 - about 50 g/L, about 10 - about 40 g/L, or about 15 - about 35 g/L. | | |

In some embodiments, the microorganism deposited under ATCC Accession No. PTA-10212, or a mutant, variant, or recombinant thereof, grows heterotrophically on glycerol as the carbon source but does not grow on glucose as the carbon source.

A typical media for growth of the microorganism deposited under ATCC Accession No. PTA-10208 is shown in Table 2:

**Table 2: PTA-10208 Vessel Media**

| Ingredient | concentration | | ranges |
|---|---|---|---|
| Na₂SO₄ | g/L | 8.8 | 0-25, 2-20, or 3-10 |
| NaCl | g/L | 0.625 | 0-25, 0.1-10, or 0.5-5 |
| KCl | g/L | 1.0 | 0-5, 0.25-3, or 0.5-2 |
| MgSO₄·7H₂O | g/L | 5.0 | 0-10, 2-8, or 3-6 |
| (NH₄)₂SO₄ | g/L | 0.42 | 0-10, 0.25-5, or 0.05-3 |
| CaCl₂ | g/L | 0.29 | 0.1-5, 0.15-3, or 0.2-1 |
| T 154 (yeast extract) | g/L | 1.0 | 0-20, 0.1-10, or 0.5-5 |
| KH₂PO₄ | g/L | 1.765 | 0.1-10, 0.5-5, or 1-3 |

| Post autoclave (Metals) | | | |
|---|---|---|---|
| Citric acid | mg/L | 46.82 | 0.1-5000, 10-3000, or 40-2500 |
| FeSO₄·7H₂O | mg/L | 10.30 | 0.1-100, 1-50, or 5-25 |
| MnCl₂·4H₂O | mg/L | 3.10 | 0.1-100, 1-50, or 2-25 |
| ZnSO₄·7H₂O | mg/L | 9.3 | 0.01-100, 1-50, or 2-25 |
| COCl₂·6H₂O | mg/L | 0.04 | 0-1, 0.001-0.1, or 0.01-0.1 |
| Na₂MoO₄·2H₂O | mg/L | 0.04 | 0.001-1, 0.005-0.5, or 0.01-0.1 |
| CuSO₄·5H₂O | mg/L | 2.07 | 0.1-100, 0.5-50, or 1-25 |
| NiSO₄·6H₂O | mg/L | 2.07 | 0.1-100, 0.5-50, or 1-25 |

| Post autoclave (Vitamins) | | | |
|---|---|---|---|
| Thiamine | mg/L | 9.75 | 0.1-100, 1-50, or 5-25 |
| Ca½-pantothenate | mg/L | 3.33 | 0.1-100, 0.1-50, or 1-10 |
| Biotin | mg/L | 3.58 | 0.1-100, 0.1-50, or 1-10 |

| Post autoclave (Carbon) | | | |
|---|---|---|---|
| Glucose | g/L | 30.0 | 5-150, 10-100, or 20-50 |
| Nitrogen Feed: | | | |

| Ingredient | Concentration | | |
|---|---|---|---|
| NH₄OH | mL/L | 23.6 | 0-150, 10-100, or 15-50 |
| Typical cultivation conditions would include the following: | | | |
| pH | about 6.5 - about 8.5, about 6.5 - about 8.0, or about 7.0 - about 8.0; | | |
| temperature: | about 17 - about 30 degrees Celsius, about 20 - about 28 degrees Celsius, or about 22 to about 24 degrees Celsius; | | |
| dissolved oxygen: | about 2 - about 100% saturation, about 5 - about 50% saturation, or about 7 - about 20% saturation; and/or | | |
| glucose controlled @: 20 | about 5 - about 50 g/L, about 10 - about 40 g/L, or about - about 35 g/L. | | |

In some embodiments, the culture medium comprises at least about 0.1%, at least about 0.5%, at least about 1%, at least about 1.5%, at least about 2%, at least about 5%, at least about 7%, at least about 10%, at least about 20%, at least about 30%, at least about 40%, at least about 50%, at least about 60%, at least about 70%, at least about 80%, or at least about 90% dissolved oxygen, as a percentage of saturation level. In some embodiments, the culture medium comprises about 0.1% to about 2%, about 0.1% to about 5%, about 0.1% to about 10%, about 0.1% to about 20%, about 0.1% to about 30%, about 0.1% to about 50%, about 0.1% to about 100%, about 5% to about 10%, about 5% to about 20%, about 5% to about 30%, about 5% to about 50%, about 5% to about 100%, about 7% to about 10%, about 7% to about 20%, about 7% to about 30%, about 7% to about 50%, about 7% to about 100%, about 10% to about 20%, about 10% to about 30%, about 10% to about 50%, about 10% to about 100%, about 20% to about 30%, about 20% to about 50%, or about 20% to about 100% dissolved oxygen, as a percentage of saturation level.

There is further disclosed an isolated biomass of a microorganism as disclosed herein. An isolated biomass as disclosed herein is a harvested cellular biomass obtained by any conventional method for the isolation of a biomass, such as described in U.S. Patent No. 5,130,242 and U.S. Appl. Publ. No. 2002/0001833.

In some embodiments, the dry cell weight of the biomass isolated from each liter of culture is at least about 10 g, at least about 15 g, at least about 20 g, at least about 25 g, at least about 30 g, at least about 50 g, at least about 60 g, at least about 70 g, at least about 80 g, at least about 100 g, at least about 120 g, at least about 140 g, at least about 160 g, at least about 180 g, or at least about 200 g after growing for about 6 days to about 8 days at about 15°C to about 30°C in a culture medium of about pH 6.5 to about 9.5 comprising sources of carbon, nitrogen, and nutrients, and about 950 ppm to about 8500 ppm chloride ions. In some embodiments, the dry cell weight of the biomass isolated from each liter of culture is at least about 10 g, at least about 15 g, at least about 20 g, at least about 25 g, at least about 30 g, at least about 50 g, at least about 60 g, at least about 70 g, at least about 80 g, at least about 100 g, at least about 120 g, at least about 140 g, at least about 160 g, at least about 180 g, or at least about 200 g after growing for about 6 days, about 7 days, or about 8 days at about 15°C, about 16°C, about 17°C, at about 18°C, at about 19°C, at about 20°C, at about 21°C, at about 22°C, at about 23°C, at about 24°C, at about 25°C, at about 26°C, at about 27°C, at about 28°C, at about 29°C, or at about 30°C in a culture medium of about pH 6.5, about pH 7, about pH 7.5, about pH 8.0, about pH 8.5, about pH 9, or about pH 9.5 comprising sources of carbon, nitrogen, and nutrients, and about 950 ppm to about 8500 ppm chloride ions. In some embodiments, the dry cell weight of the biomass isolated from each liter of culture is about 10 g to about 200 g after growing for about 6 days to about 8 days at about 15°C to about 30°C in a culture medium of about pH 6.5 to about pH 9.5 comprising sources of carbon, nitrogen, and nutrients, and about 950 ppm to about 8500 ppm chloride ions. In some embodiments, the dry cell weight of the biomass isolated from each liter of culture is about 10 g to about 200 g, about 10 g to about 100 g, about 10 g to about 50 g, about 15 g to about 200 g, about 15 g to about 100 g, about 15 g to about 50 g, about 20 g to about 200 g, about 20 g to about 100 g, about 20 g to about 50 g, about 50 g to about 200 g, or about 50 g to about 100 g after growing for about 6 days, about 7 days, or about 8 days at about 15°C, about 16°C, about 17°C, at about 18°C, at about 19°C, at about 20°C, at about 21°C, at about 22°C, at about 23°C, at about 24°C, at about 25°C, at about 26°C, at about 27°C, at about 28°C, at about 29°C, or at about 30°C in a culture medium of about pH 6.5, about pH 7, about pH 7.5, about pH 8.0, about pH 8.5, about pH 9, or about pH 9.5 comprising sources of carbon, nitrogen, and nutrients, and about 950 ppm to about 8500 ppm chloride ions. In some embodiments, the isolated culture does not contain polyvinylpyrrolidone.

In some embodiments, the isolated culture has an omega-3 fatty acid productivity of at least about 0.2 g/L/day, at least about 0.3 g/L/day, at least about 0.4 g/L/day, at least about 0.5 g/L/day, at least about 1 g/L/day, at least about 1.2 g/L/day, at least about 1.5 g/L/day, at least about 1.7 g/L/day, at least about 2 g/L/day, at least about 3 g/L/day, at least about 3.5 g/L/day, at least about 4 g/L/day, at least about 4.5 g/L/day, at least about 5 g/L/day, at least about 6 g/L/day, or at least about 8 g/L/day after growing for about 6 days, about 7 days, or about 8 days at about 15°C to about 30°C in a culture medium of about pH 6.5 to about pH 8.5 or about pH 6.5 to about pH 9.5 comprising sources of carbon, nitrogen, and nutrients, and about 950 ppm to about 8500 ppm chloride ions. In some embodiments, the isolated culture has an omega-3 fatty acid productivity of about 0.2 g/L/day to about 20 g/L/day, about 0.4 g/L/day to about 20 g/L/day, about 0.4 g/L/day to about 2 g/L/day, about 1 g/L/day to about 2 g/L/day, about 1 g/L/day to about 20 g/L/day, about 2 g/L/day to about 15 g/L/day, about 2 g/L/day to about 10 g/L/day, about 3 g/L/day to about 10 g/L/day, about 4 g/L/day to about 9 g/L/day, about 4 g/L/day to about 8 g/L/day, about 4 g/L/day to about 7 g/L/day, or about 4 g/L/day to about 6 g/L/day after growing for about 6 days, about 7 days, or about 8 days at about 15°C to about 30°C in a culture medium of about pH 6.5 to about pH 9.5 comprising sources of carbon, nitrogen, and nutrients, and about 950 ppm to about 8500 ppm chloride ions.

In some embodiments, the isolated culture comprises an EPA productivity of at least about 0.2 g/L/day, at least about 0.3 g/L/day, at least about 0.4 g/L/day, at least about 0.5 g/L/day, at least about 0.6 g/L/day, at least about 0.7 g/L/day, at least about 0.8 g/L/day, at least about 0.9 g/L/day, at least about 1 g/L/day, at least about 1.2 g/L/day, at least about 1.5 g/L/day, at least about 1.7 g/L/day, at least about 2 g/L/day, at least about 3 g/L/day, at least about 4 g/L/day, or at least about 5 g/L/day after growing for about 6 days, about 7 days, or about 8 days at about 15°C to about 30°C in a culture medium of about pH 6.5 to about pH 8.5 or about pH 6.5 to about pH 9.5 comprising sources of carbon, nitrogen, and nutrients, and about 950 ppm to about 8500 ppm chloride ions. In some embodiments, the EPA productivity is about 0.2 g/L/day to about 5 g/L/day, about 0.2 g/L/day to about 4 g/L/day, about 0.2 g/L/day to about 3 g/L/day, about 0.2 g/L/day to about 2 g/L/day, about 0.2 g/L/day to about 1 g/L/day, about 0.2 g/L/day to about 0.8 g/L/day, about 0.2 g/L/day to about 0.7 g/L/day, about 1 g/L/day to about 5 g/L/day, about 1 g/L/day to about 4 g/L/day, about 1 g/L/day to about 3 g/L/day, or about 1 g/L/day to about 2 g/L/day after growing for about 6 days, about 7 days, or about 8 days at about 15°C to about 30°C in a culture medium of about pH 6.5 to about pH 8.5 or about pH 6.5 to about pH 9.5 comprising sources of carbon, nitrogen, and nutrients, and about 950 ppm to about 8500 ppm chloride ions. In some embodiments, any of the aforementioned EPA productivities are associated with any of the aforementioned omega-3 fatty acid productivities. In some embodiments, the culture further comprises a DHA productivity of about 0 g/L/day to about 5 g/L/day, about 0 g/L/day to about 4 g/L/day, about 0 g/L/day to about 3 g/L/day, about 0 g/L/day to about 2 g/L/day, about 0 g/L/day to about 1 g/L/day, about 0.2 g/L/day to about 5 g/L/day, about 0.2 g/L/day to about 4 g/L/day, about 0.2 g/L/day to about 3 g/L/day, about 0.2 g/L/day to about 2 g/L/day, about 0.2 g/L/day to about 1 g/L/day, about 1 g/L/day to about 5 g/L/day, about 2 g/L/day to about 5 g/L/day, about 2 g/L/day to about 4 g/L/day, or about 2 g/L/day to about 3 g/L/day. In some embodiments, the DHA productivity is less than about 5 g/L/day, less than about 4 g/L/day, less than about 3 g/L/day, less than about 2 g/L/day, less than about 1 g/L/day, less than about 0.5 g/L/day, less than about 0.2 g/L/day, or about 0 g/L/day.

In some embodiments, the fermentation volume (volume of culture) is at least about 2 liters, at least about 10 liters, at least about 50 liters, at least about 100 liters, at least about 200 liters, at least about 500 liters, at least about 1000 liters, at least about 10,000 liters, at least about 20,000 liters, at least about 50,000 liters, at least about 100,000 liters, at least about 150,000 liters, at least about 200,000 liters, or at least about 250,000 liters. In some embodiments, the fermentation volume is about 2 liters to about 300,000 liters, about 2 liters, about 10 liters, about 50 liters, about 100 liters, about 200 liters, about 500 liters, about 1000 liters, about 10,000 liters, about 20,000 liters, about 50,000 liters, about 100,000 liters, about 150,000 liters, about 200,000 liters, about 250,000 liters, or about 300,000 liters.

There is further disclosed an isolated biomass comprising a fatty acid profile as disclosed herein. In some embodiments, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80% of the dry cell weight of the biomass are fatty acids. In some embodiments, greater than about 20%, greater than about 25%, greater than about 30%, greater than about 35%, greater than about 40%, greater than about 45%, greater than about 50%, greater than about 55%, or greater than about 60% of the dry cell weight of the biomass are fatty acids. In some embodiments, about 20% to about 55%, about 20% to about 60%, about 20% to about 70%, about 20% to about 80%, about 30% to about 55%, about 30% to about 70%, about 30% to about 80%, about 40% to about 60%, about 40% to about 70%, about 40% to about 80%, about 50% to about 60%, about 50% to about 70%, about 50% to about 80%, about 55% to about 70%, about 55% to about 80%, about 60% to about 70%, or about 60% to about 80% by weight of the dry cell weight of the biomass are fatty acids. In some embodiments, the biomass comprises more than about 10%, at least about 12%, at least about 15%, at least about 20%, at least about 25%, at least about 30%, about least about 35%, at least about 40%, or at least about 45% by weight of the fatty acids as EPA. In some embodiments, the biomass comprises about 10% to about 55%, about 12% to about 55%, about 15% to about 55%, about 20% to about 55%, about 20% to about 40%, or about 20% to about 30% by weight of the fatty acids as EPA. In some embodiments, the biomass comprises a triacylglycerol fraction, wherein at least about 12%, at least about 13%, at least about 14%, at least about 15%, at least about 16%, at least about 17%, at least about 18%, at least about 19%, or at least about 20% by weight of the triacylglycerol fraction is EPA. In some embodiments, the biomass comprises a triacylglycerol fraction, wherein the EPA content of the triacylglycerol fraction is from at least about 12% to about 55%, about 12% to about 50%, about 12% to about 45%, at least about 12% to about 40%, at least about 12% to about 35%, or at least about 12% to about 30%, about 15% to about 55%, about 15% to about 50%, about 15% to about 45%, about 15% to about 40%, about 15% to about 35%, about 15% to about 30%, about 20% to about 55%, about 20% to about 50%, about 20% to about 45%, at least about 20% to about 40%, at least about 20% to about 35%, or about 20% to about 30% by weight. In some embodiments, at least about 20%, at least about 25%, at least about 30%, at least about 35%, at least about 40%, at least about 50%, or at least about 60% by weight of the dry cell weight of the biomass is DHA. In some embodiments, about 20% to about 60%, about 25% to about 60%, about 25% to about 50%, about 25% to about 45%, about 30% to about 50%, or about 35% to about 50% by weight of the dry cell weight of the biomass is DHA. In some embodiments, the biomass comprises about 10% or less, about 9% or less, about 8% or less, about 7% or less, about 6% or less, about 5% or less, about 4% or less, about 3% or less, about 2% or less, or about 1% or less by weight of the fatty acids as DHA. In some embodiments, the biomass comprises about 1% to about 10%, about 1% to about 5%, about 2% to about 5%, about 3% to about 5%, or about 3% to about 10% by weight of the fatty acids as DHA. In some embodiments, the biomass is substantially free of DHA. In some embodiments, the biomass comprises about 0.1% to less than about 5%, about 0.1% to about 4%, about 0.1% to about 3%, about 0.1% to about 2%, about 0.2% to less than about 5%, about 0.2% to about 4%, about 0.2% to about 3%, about 0.2% to about 2%, about 0.3% to about 2%, about 0.1% to about 0.5%, about 0.2% to about 0.5%, about 0.1% to about 0.4%, about 0.2% to about 0.4%, about 0.5% to about 2%, about 1% to about 2%, about 0.5% to about 1.5%, or about 1% to about 1.5% by weight of the fatty acids as ARA. In some embodiments, the biomass comprises less than about 5%, about 4% or less, about 3% or less, about 2% or less, about 1.5% or less, about 1% or less, about 0.5% or less, about 0.4% or less, about 0.3% or less, about 0.2% or less, or about 0.1% or less by weight of the fatty acids as ARA. In some embodiments, the biomass is substantially free of ARA. In some embodiments, the biomass comprises about 0.4% to about 2%, about 0.4% to about 3%, about 0.4% to about 4%, about 0.4% to about 5%, about 0.4% to less than about 5%, about 0.5% to about 1%, about 0.5% to about 2%, about 0.5% to about 3%, about 0.5% to about 4%, about 0.5% to about 5%, about 0.5% to less than about 5%, about 1% to about 2%, about 1% to about 3%, about 1% to about 4%, about 1% to about 5%, or about 1% to less than about 5% by weight of the fatty acids as DPA n-6. In some embodiments, the biomass comprises about 5% or less, less than about 5%, about 4% or less, about 3% or less, about 2% or less, about 1% or less, about 0.75% or less, about 0.6% or less, or about 0.5% or less by weight of the fatty acids as DPA n-6. In some embodiments, the biomass is substantially free of DPA n-6. In some embodiments, the biomass comprises fatty acids with about 5% or less, less than about 5%, about 4% or less, about 3% or less, or about 2% or less by weight of oleic acid (18:1 n-9), linoleic acid (18:2 n-6), linolenic acid (18:3 n-3), eicosenoic acid (20:1 n-9), erucic acid (22:1 n-9), or combinations thereof.

The characteristics of an isolated biomass as disclosed herein are associated with endogenous or native properties of the isolated biomass rather than exogenously introduced materials. In some embodiments, the isolated biomass does not contain polyvinylpyrrolidone or is not isolated from a culture containing polyvinylpyrrolidone.

There is further disclosed a method of producing a biomass. In some embodiments, the method for producing a biomass of the invention comprises growing any of the isolated microorganisms of the invention or mixtures thereof in a culture to produce a biomass. There is further disclosed a biomass produced by the method.

### Microbial Oils

The invention is directed to a microbial oil comprising a fatty acid profile of the invention. A microbial oil of the invention is a "crude oil" or a "refined oil" comprising a triacylglycerol fraction of at least about 35% by weight. A "crude oil" is an oil that is extracted from the biomass of the microorganism without further processing. A "refined oil" is an oil that is obtained by treating a crude oil with standard processing of refining, bleaching, and/or deodorizing. *See,* e.g., U.S. Patent No. 5,130,242. A microbial oil also includes a "final oil" as described herein, which is a refined oil that has been diluted with a vegetable oil. In some embodiments, a final oil is a refined oil that has been diluted with high oleic sunflower oil. The term "microbial" as used herein includes, but is not limited to, the terms "microalgal," "thraustochytrid," and taxonomic classifications associated with any of the deposited microorganisms described herein. The terms "Thraustochytriales," "thraustochytrid," *"Schizochytrium,"* and *"Thraustochytrium"* as used in reference to any of the microbial oils of the deposited microorganisms described herein are based on present taxonomic classifications including available phylogenetic information and are not intended to be limiting in the event that the taxonomic classifications are revised after the filing date of the present application.

In some embodiments, a fatty acid as described herein can be a fatty acid ester. In some embodiments, a fatty acid ester includes an ester of an omega-3 fatty acid, omega-6 fatty acid, and combinations thereof. In some embodiments, the fatty acid ester is a DHA ester, an EPA ester, or a combination thereof. In some embodiments, an oil or fraction thereof as described herein is esterified to produce an oil or fraction thereof comprising fatty acid esters. The term "ester" refers to the replacement of the hydrogen in the carboxylic acid group of the fatty acid molecule with another substituent. Typical esters are known to those in the art, a discussion of which is provided by Higuchi, T. and V. Stella in Pro-drugs as Novel Delivery Systems, Vol. 14, A.C.S. Symposium Series, Bioreversible Carriers in Drug Design, Ed. Edward B. Roche, American Pharmaceutical Association, Pergamon Press, 1987, and Protective Groups in Organic Chemistry, McOmie ed., Plenum Press, New York, 1973. Examples of esters include methyl, ethyl, propyl, butyl, pentyl, t-butyl, benzyl, nitrobenzyl, methoxybenzyl, benzhydryl, and trichloroethyl. In some embodiments, the ester is a carboxylic acid protective ester group, esters with aralkyl (e.g., benzyl, phenethyl), esters with lower alkenyl (e.g., allyl, 2-butenyl), esters with lower-alkoxy-lower-alkyl (e.g., methoxymethyl, 2-methoxyethyl, 2-ethoxyethyl), esters with lower-alkanoyloxy-lower-alkyl (e.g., acetoxymethyl, pivaloyloxymethyl, 1-pivaloyloxyethyl), esters with lower-alkoxycarbonyl-lower-alkyl (e.g., methoxycarbonylmethyl, isopropoxycarbonylmethyl), esters with carboxy-lower alkyl (e.g., carboxymethyl), esters with lower-alkoxycarbonyloxy-lower-alkyl (e.g., 1-(ethoxycarbonyloxy)ethyl, 1-(cyclohexyloxycarbonyloxy)ethyl), esters with carbamoyloxy-lower alkyl (e.g., carbamoyloxymethyl), and the like. In some embodiments, the added substituent is a linear or cyclic hydrocarbon group, e.g., a C1-C6 alkyl, C1-C6 cycloalkyl, C1-C6 alkenyl, or C1-C6 aryl ester. In some embodiments, the ester is an alkyl ester, e.g., a methyl ester, ethyl ester or propyl ester. In some embodiments, the ester substituent is added to the free fatty acid molecule when the fatty acid is in a purified or semi-purified state. Alternatively, the fatty acid ester is formed upon conversion of a triacylglycerol to an ester.

The present invention is directed to methods of producing microbial oils. In some embodiments, the method comprises growing any of the isolated microorganisms as disclosed herein or mixtures thereof in a culture to produce a microbial oil comprising omega-3 fatty acids. In some embodiments, the method further comprises extracting the microbial oil. In some embodiments, the method comprises extracting a microbial oil comprising omega-3 fatty acids from any of the biomasses as disclosed herein or mixtures thereof. In some embodiments, the method comprises heterotrophically growing the isolated microorganism, wherein the culture comprises a carbon source as described herein. The microbial oil can be extracted from a freshly harvested biomass or can be extracted from a previously harvested biomass that has been stored under conditions that prevent spoilage. Known methods can be used to culture a microorganism as disclosed herein, to isolate a biomass from the culture, to extract a microbial oil from the biomass, and to analyze the fatty acid profile of oils extracted from the biomass. *See,* e.g., U.S. Patent No. 5,130,242. The invention is directed to a microbial oil produced by any of the methods of the invention.

In some embodiments, the microbial oil is extracted by an enzyme extraction method. In some embodiments, the microbial oil is extracted by a mechanical extraction method. In some embodiments, the mechanical extraction method comprises one or more of: (1) processing a pasteurized fermentation broth through a homogenizer to assist in cell lysis and release of oil from cells; (2) adding isopropyl alcohol to the fermentation broth following homogenization to break the oil and water emulsion; (3) centrifuging the mixture to recover the oil phase; and (4) drying under vacuum with addition of antioxidants. In some embodiments, the crude oil is purified. In some embodiments, purification of the crude oil comprises one or more of: (1) pumping the crude oil into a refining tank and heating the oil, followed by adding an acid solution with mixing; (2) adding a caustic solution to the oil after acid treatment; (3) reheating the crude oil and then centrifuging to separate the heavy phase from the refined oil; (4) removing the remaining polar compounds, trace metals, and oxidation products from the refined oil by using, for example, acid, TriSyl®, clay, and/or filtration; (5) chill filtering the bleached oil to further remove high melting point components from the oil to achieve the desired level of clarity; (6) heating the oil, after which the oil is then cooled and held for a period of time causing the high melting triglycerides and waxes to crystallize; (7) adding a filter aid to the chilled oil and then removing crystallized solids by filtration; (8) using a deodorizer after chill filtration, operated under high temperature and vacuum, to remove, for example, peroxides and any remaining low molecular weight compounds that can cause off-odor and flavors; (9) transferring the oil to the deodorizer feed tank, deaerating, and deodorizing, for example, in a packed column deodorizer; and (10) cooling, for example, under a nitrogen blanket at the end of the deodorization cycle and adding suitable antioxidants to the deodorized oil to provide oxidative stability.

In some embodiments, the microbial oil comprises a sterol esters fraction of about 0%, at least about 0.1%, at least about 0.2%, at least about 0.5%, at least about 1%, at least about 1.5%, at least about 2%, or at least about 5% by weight. In some embodiments, the microbial oil comprises a sterol esters fraction of about 0% to about 1.5%, about 0% to about 2%, about 0% to about 5%, about 1% to about 1.5%, about 0.2% to about 1.5%, about 0.2% to about 2%, or about 0.2% to about 5% by weight. In some embodiments, the microbial oil comprises a sterol esters fraction of about 5% or less, about 4% or less, about 3% or less, about 2% or less, about 1% or less, about 0.5% or less, about 0.3% or less, about 0.2% or less, about 0.5% or less, about 0.4% or less, about 0.3% or less, or about 0.2% or less by weight.

In some embodiments, the microbial oil comprises a triacylglycerol fraction of at least about 35%, at least about 40%, at least about 45%, at least about 50%, at least about 55%, at least about 60%, at least about 65%, at least about 70%, at least about 75%, at least about 80%, at least about 85%, or at least about 90% by weight. In some embodiments, the microbial oil comprises a triacylglycerol fraction of about 35% to about 98%, about 35% to about 90%, about 35% to about 80%, about 35% to about 70%, about 35% to about 70%, about 35% to about 65%, about 40% to about 70%, about 40% to about 65%, about 40% to about 55%, about 40% to about 50%, about 65% to about 95%, about 75% to about 95%, about 75% to about 98%, about 80% to about 95%, about 80% to about 98%, about 90% to about 96%, about 90% to about 97%, about 90% to about 98%, about 90%, about 95%, about 97%, or about 98% by weight.

In some embodiments, the microbial oil comprises a diacylglycerol fraction of at least about 10%, at least about 11%, at least about 12%, at least about 13%, at least about 14%, at least about 15%, at least about 16%, at least about 17%, at least about 18%, at least about 19%, or at least about 20% by weight. In some embodiments, the microbial oil comprises a diacylglycerol fraction of about 10% to about 45%, about 10% to about 40%, about 10% to about 35%, about 10% to about 30%, about 15% to about 40%, about 15% to about 35%, or about 15% to about 30% by weight. In some embodiments, the microbial oil comprises a 1,2-diacylglycerol fraction of at least about 0.2%, at least about 0.3%, at least about 0.4%, at least about 0.5%, at least about 1%, at least about 5%, at least about 10%, at least about 11%, at least about 12%, at least about 13%, at least about 14%, at least about 15%, at least about 16%, at least about 17%, at least about 18%, at least about 19%, or at least about 20% by weight. In some embodiments, the microbial oil comprises a diacylglycerol fraction of about 0.2% to about 45%, about 0.2% to about 30%, about 0.2% to about 20%, about 0.2% to about 10%, about 0.2% to about 5%, about 0.2% to about 1%, about 0.2% to about 0.8%, about 0.4% to about 45%, about 0.4% to about 30%, about 0.4% to about 20%, about 0.4% to about 10%, about 0.4% to about 5%, about 0.4% to about 1%, about 0.4% to about 0.8%, about 0.5% to about 1%, about 0.5% to about 0.8%, about 10% to about 45%, about 10% to about 40%, about 10% to about 35%, about 10% to about 30%, about 15% to about 40%, about 15% to about 35%, about 15% to about 30%, or about 15% to about 25% by weight. In some embodiments, the microbial oil comprises a 1,3-diacylglycerol fraction of at least about 0.1%, at least about 0.2%, at least about 0.5%, at least about 1%, at least about 2%, at least about 2.5 %, or at least about 3% by weight. In some embodiments, the microbial oil comprises a sterol fraction of at least about 0.3%, at least about 0.4%, at least about 0.5%, at least about 1%, at least about 1.5%, at least about 2%, or at least about 5% by weight.

In some embodiments, the microbial oil comprises a sterol fraction of about 0.3% to about 5%, about 0.3% to about 2%, about 0.3% to about 1.5%, about 0.5% to about 1.5%, about 1% to about 1.5%, about 0.5% to about 2%, about 0.5% to about 5%, about 1% to about 2%, or about 1% to about 5% by weight. In some embodiments, the microbial oil comprises a sterol fraction of about 5% or less, about 4% or less, about 3% or less, about 2% or less, about 1.5% or less, or about 1% or less by weight.

In some embodiments, the microbial oil comprises a phospholipid fraction of at least about 2%, at least about 5%, or at least about 8% by weight. In some embodiments, the microbial oil comprises a phospholipid fraction of about 2% to about 25%, about 2% to about 20%, about 2% to about 15%, about 2% to about 10%, about 5% to about 25%, about 5% to about 20%, about 5% to about 20%, about 5% to about 10%, or about 7% to about 9% by weight. In some embodiments, the microbial oil comprises a phospholipid fraction of less than about 20%, less than about 15%, less than about 10%, less than about 9%, or less than about 8% by weight. In some embodiments, the microbial oil is substantially free of phospholipids. In some embodiments, the microbial oil comprises unsaponifiables of less than about 2%, less than about 1.5%, less than about 1%, or less than about 0.5% by weight of the oil. The lipid classes present in the microbial oil, such as a triacylglycerol fraction, can be separated by flash chromatography and analyzed by thin layer chromatography (TLC), or separated and analyzed by other methods known in the art.

In some embodiments, the microbial oil and/or one or more fractions thereof selected from the triacylglycerol fraction, the free fatty acid fraction, the sterol fraction, the diacylglycerol fraction, and combinations thereof, comprises at least about 5%, at least about 10%, more than about 10%, at least about 12%, at least about 13%, at least about 14%, at least about 15%, at least about 16%, at least about 17%, at least about 18%, at least about 19%, at least about 20%, at least about 25%, at least about 30%, about least about 35%, at least about 40%, or at least about 45% by weight EPA. In some embodiments, the microbial oil and/or one or more fractions thereof selected from the triacylglycerol fraction, the free fatty acid fraction, the sterol fraction, the diacylglycerol fraction, and combinations thereof, comprises about 5% to about 55%, about 5% to about 50%, about 5% to about 45%, about 5% to about 40%, about 5% to about 35%, about 5% to about 30%, about 10% to about 55%, about 10% to about 50%, about 10% to about 45%, about 10% to about 40%, about 10% to about 35%, about 10% to about 30%, at least about 12% to about 55%, at least about 12% to about 50%, at least about 12% to about 45%, at least about 12% to about 40%, at least about 12% to about 35%, or at least about 12% to about 30%, about 15% to about 55%, about 15% to about 50%, about 15% to about 45%, about 15% to about 40%, about 15% to about 35%, about 15% to about 30%, about 15% to about 25%, about 15% to about 20%, about 20% to about 55%, about 20% to about 50%, about 20% to about 45%, about 20% to about 40%, or about 20% to about 30% by weight EPA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises at least about 5%, at least about 10%, at least about 15%, at least about 20%, at least about 25%, at least about 30%, at least about 35%, at least about 40%, at least about 50%, or at least about 60% by weight DHA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 5% to about 60%, about 5% to about 55%, about 5% to about 50%, about 5% to about 40%, about 10% to about 60%, about 10% to about 50%, about 10% to about 40%, about 20% to about 60%, about 25% to about 60%, about 25% to about 50%, about 25% to about 45%, about 30% to about 50%, about 35% to about 50%, or about 30% to about 40% by weight DHA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 10% or less, about 9% or less, about 8% or less, about 7% or less, about 6% or less, about 5% or less, about 4% or less, about 3% or less, about 2% or less, or about 1% or less by weight DHA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 1% to about 10%, about 1% to about 5%, about 2% to about 5%, about 3% to about 5%, or about 3% to about 10% by weight of the fatty acids as DHA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, is substantially free of DHA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 0.1% to about 5%, about 0.1% to less than about 5%, about 0.1% to about 4%, about 0.1% to about 3%, about 0.1% to about 2%, about 0.2% to about 5%, about 0.2% to less than about 5%, about 0.2% to about 4%, about 0.2% to about 3%, about 0.2% to about 2%, about 0.3% to about 2%, about 0.1% to about 0.5%, about 0.2% to about 0.5%, about 0.1% to about 0.4%, about 0.2% to about 0.4%, about 0.5% to about 2%, about 1% to about 2%, about 0.5% to about 1.5%, or about 1% to about 1.5% by weight ARA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 5% or less, less than about 5%, about 4% or less, about 3% or less, about 2% or less, about 1.5% or less, about 1% or less, about 0.5% or less, about 0.4% or less, about 0.3% or less, about 0.2% or less, or about 0.1% or less by weight ARA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, is substantially free of ARA. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 0.4% to about 2%, about 0.4% to about 3%, about 0.4% to about 4%, about 0.4% to about 5%, about 0.4% to less than about 5%, about 0.5% to about 1%, about 0.5% to about 2%, about 0.5% to about 3%, about 0.5% to about 4%, about 0.5% to about 5%, about 0.5% to less than about 5%, about 1% to about 2%, about 1% to about 3%, about 1% to about 4%, about 1% to about 5%, or about 1% to less than about 5% by weight DPA n-6. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises about 5%, less than about 5%, about 4% or less, about 3% or less, about 2% or less, about 1% or less, about 0.75% or less, about 0.6% or less, or about 0.5% or less by weight DPA n-6. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, is substantially free of DPA n-6. In some embodiments, the microbial oil and/or one or more fractions thereof selected from the triacylglycerol fraction, the diacylglycerol fraction, the sterol fraction, the sterol esters fraction, the free fatty acids fraction, the phospholipid fraction, and combinations thereof, comprises fatty acids with about 5% or less, less than about 5%, about 4% or less, about 3% or less, or about 2% or less by weight of oleic acid (18:1 n-9), linoleic acid (18:2 n-6), linolenic acid (18:3 n-3), eicosenoic acid (20:1 n-9), erucic acid (22:1 n-9), stearidonic acid (18:4 n-3), or combinations thereof.

The triacylglycerol molecule contains 3 central carbon atoms (C(*sn-1*)H₂R1*-*(*sn-*2)H₂R2-C(*sn*-3)H₂R3), allowing for formation of different positional isomers. In some embodiments, the microbial oil comprises a triacylglycerol fraction in which at least about 2%, at least about 3%, at least about 5%, at least about 10%, at least about 15%, at least about 20%, at least about 30%, at least about 35%, or at least about 40% of the triacylglycerols in the triacylglycerol fraction contain DHA at two positions in the triacylglycerol (di-substituted DHA) selected from any two of the *sn-1, sn-2,* and *sn-3* positions, based on the relative area percent of peaks on an HPLC chromatograph. In some embodiments, the microbial oil comprises a triacylglycerol fraction in which about 2% to about 55%, about 2% to about 50%, about 2% to about 45%, about 2% to about 40%, about 2% to about 35%, about 2% to about 30%, about 2% to about 25%, about 5% to about 55%, about 5% to about 50%, about 5% to about 45%, about 5% to about 40%, about 5% to about 35%, about 5% to about 30%, about 5% to about 25%, about 10% to about 55%, about 10% to about 50%, about 10% to about 45%, about 10% to about 40%, about 10% to about 35%, about 10% to about 30%, about 10% to about 25%, about 10% to about 20%, about 20% to about 40%, about 20% to about 35%, or about 20% to about 25% of the triacylglycerols in the triacylglycerol fraction contain EPA at two positions in the triacylglycerol selected from any two of the *sn-1, sn-2,* or *sn-3* positions, based on the relative area percent of peaks on an HPLC chromatograph. In some embodiments, the microbial oil comprises a triacylglycerol fraction in which at least about 0.5%, at least about 1%, at least about 1.5%, or at least about 2% of the triacylglycerols in the triacylglycerol fraction contain DHA at all of the *sn-1, sn-2,* and *sn-3* positions (trisubstituted DHA), based on the relative area percent of peaks on an HPLC chromatograph. In some embodiments, the microbial oil comprises a triacylglycerol fraction in which about 0.5% to about 5%, about 0.5% to about 3%, about 0.5% to about 2.5%, about 0.5% to about 2%, about 1% to about 5%, about 1% to about 3%, or about 1% to about 2% of the triacylglycerols in the triacylglycerol fraction contain DHA at all of the *sn-1, sn-2,* and *sn-3* positions, based on the relative area percent of peaks on an HPLC chromatograph. In some embodiments, the microbial oil comprises a triacylglycerol fraction in which at least about 10%, at least about 15%, at least about 20%, at least about 25%, at least about 30%, at least about 35%, at least about 40%, at least about 45%, at least about 50%, at least about 55%, or at least about 60% of the triacylglycerols in the triacylglycerol fraction contain DHA at one position in the triacylglycerol selected from any one of the *sn-1, sn-2,* or *sn-3* positions, based on the relative area percent of peaks on an HPLC chromatograph. In some embodiments, the microbial oil comprises a triacylglycerol fraction in which about 10% to about 80%, about 10% to about 70%, about 10% to about 60%, about 15% to about 80%, about 15% to about 75%, about 15% to about 70%, about 15% to about 65%, about 15% to about 60%, about 35% to about 80%, about 35% to about 75%, about 35% to about 65%, about 35% to about 60%, about 40% to about 80%, about 40% to about 75%, about 40% to about 70%, about 40% to about 65%, about 40% to about 60%, or about 40% to about 55% of the triacylglycerols in the triacylglycerol fraction contain DHA at one position in the triacylglycerol selected from any one of the *sn-1, sn-2,* and *sn-3* positions, based on the relative area percent of peaks on an HPLC chromatograph.

### Compositions

There are further disclosed compositions comprising a microorganism as disclosed herein, an isolated biomass disclosed herein, a microbial oil of the invention, or combinations thereof.

A microorganism, biomass as disclosed herein, or microbial oil of the invention can be further chemically or physically modified or processed based on the requirements of the composition by any known technique.

Microorganism cells or biomasses can be dried prior to use in a composition by methods including, but not limited to, freeze drying, air drying, spray drying, tunnel drying, vacuum drying (lyophilization), and a similar process. Alternatively, a harvested and washed biomass can be used directly in a composition without drying. *See,* e.g., U.S. Patent Nos. 5,130,242 and 6,812,009, each.

Microbial oils of the invention can be used as starting material to more efficiently produce a product enriched in a fatty acid such as EPA. For example, the microbial oils of the invention can be subjected to various purification techniques known in the art, such as distillation or urea adduction, to produce a higher potency product with higher concentrations of EPA or another fatty acid. The microbial oils of the invention can also be used in chemical reactions to produce compounds derived from fatty acids in the oils, such as esters and salts of EPA or another fatty acid.

A composition of the invention can include one or more excipients. As used herein, "excipient" refers to a component, or mixture of components, that is used in a composition of the present invention to give desirable characteristics to the composition, including foods as well as pharmaceutical, cosmetic, and industrial compositions. An excipient of the present invention can be described as a "pharmaceutically acceptable" excipient when added to a pharmaceutical composition, meaning that the excipient is a compound, material, composition, salt, and/or dosage form which is, within the scope of sound medical judgment, suitable for contact with tissues of human beings and non-human animals without excessive toxicity, irritation, allergic response, or other problematic complications over the desired duration of contact commensurate with a reasonable benefit/risk ratio. In some embodiments, the term "pharmaceutically acceptable" means approved by a regulatory agency of the Federal or a state government or listed in the U.S. Pharmacopeia or other generally recognized international pharmacopeia for use in animals, and more particularly in humans. Various excipients can be used. In some embodiments, the excipient can be, but is not limited to, an alkaline agent, a stabilizer, an antioxidant, an adhesion agent, a separating agent, a coating agent, an exterior phase component, a controlled-release component, a solvent, a surfactant, a humectant, a buffering agent, a filler, an emollient, or combinations thereof. Excipients in addition to those discussed herein can include excipients listed in, though not limited to, Remington: The Science and Practice of Pharmacy, 21st ed. (2005). Inclusion of an excipient in a particular classification herein (e.g., "solvent") is intended to illustrate rather than limit the role of the excipient. A particular excipient can fall within multiple classifications.

Compositions of the invention include, but are not limited to, food products, pharmaceutical compositions, cosmetics, and industrial compositions.

In some embodiments, the composition is a food product. A food product is any food for non-human animal or human consumption, and includes both solid and liquid compositions. A food product can be an additive to animal or human foods. Foods include, but are not limited to, common foods; liquid products, including milks, beverages, therapeutic drinks, and nutritional drinks; functional foods; supplements; nutraceuticals; infant formulas, including formulas for pre-mature infants; foods for pregnant or nursing women; foods for adults; geriatric foods; and animal foods.

In some embodiments, a microorganism, biomass, as disclosed herein or microbial oil of the invention can be used directly as or included as an additive within one or more of: an oil, shortening, spread, other fatty ingredient, beverage, sauce, dairy-based or soy-based food (such as milk, yogurt, cheese and ice-cream), a baked good, a nutritional product, e.g., as a nutritional supplement (in capsule or tablet form), a vitamin supplement, a diet supplement, a powdered drink, and a finished or semi-finished powdered food product. In some embodiments, the nutritional supplement is in the form of a vegetarian capsule that is not formed from and does not contain any components from an animal source.

A partial list of food compositions that can include a microbial oil of the invention includes, but is not limited to, soya based products (milks, ice creams, yogurts, drinks, creams, spreads, whiteners); soups and soup mixes; doughs, batters, and baked food items including, for example, fine bakery wares, breakfast cereals, cakes, cheesecakes, pies, cupcakes, cookies, bars, breads, rolls, biscuits, muffins, pastries, scones, croutons, crackers, sweet goods, snack cakes, pies, granola/snack bars, and toaster pastries; candy; hard confectionery; chocolate and other confectionery; chewing gum; liquid food products, for example milks, energy drinks, infant formula, carbonated drinks, teas, liquid meals, fruit juices, fruit-based drinks, vegetable-based drinks; multivitamin syrups, meal replacers, medicinal foods, and syrups; powdered beverage mixes; pasta; processed fish products; processed meat products; processed poultry products; gravies and sauces; condiments (ketchup, mayonnaise, etc.); vegetable oil-based spreads; dairy products; yogurt; butters; frozen dairy products; ice creams; frozen desserts; frozen yogurts; semisolid food products such as baby food; puddings and gelatin desserts; processed and unprocessed cheese; pancake mixes; food bars including energy bars; waffle mixes; salad dressings; replacement egg mixes; nut and nut-based spreads; salted snacks such as potato chips and other chips or crisps, corn chips, tortilla chips, extruded snacks, popcorn, pretzels, potato crisps, and nuts; and specialty snacks such as dips, dried fruit snacks, meat snacks, pork rinds, health food bars and rice/corn cakes.

In some embodiments, a microbial oil of the invention can be used to supplement infant formula. Infant formula can be supplemented with a microbial oil of the invention alone or in combination with a physically refined oil derived from an arachidonic acid (ARA)-producing microorganism. An ARA-producing microorganism, for example, is *Mortierella alpina* or *Mortierella* sect. *schmuckeri.* Alternatively, infant formulas can be supplemented with a microbial oil of the invention in combination with an oil rich in ARA, including ARASCO® (Martek Biosciences, Columbia, MD).

In some embodiments, the composition is an animal feed. An "animal" includes non-human organisms belonging to the kingdom Animalia, and includes, without limitation, aquatic animals and terrestrial animals. The term "animal feed" or "animal food" refers to any food intended for non-human animals, whether for fish; commercial fish; ornamental fish; fish larvae; bivalves; mollusks; crustaceans; shellfish; shrimp; larval shrimp; artemia; rotifers; brine shrimp; filter feeders; amphibians; reptiles; mammals; domestic animals; farm animals; zoo animals; sport animals; breeding stock; racing animals; show animals; heirloom animals; rare or endangered animals; companion animals; pet animals such as dogs, cats, guinea pigs, rabbits, rats, mice, or horses; primates such as monkeys (e.g., cebus, rhesus, African green, patas, cynomolgus, and cercopithecus), apes, orangutans, baboons, gibbons, and chimpanzees; canids such as dogs and wolves; felids such as cats, lions, and tigers; equids such as horses, donkeys, and zebras; food animals such as cows, cattle, pigs, and sheep; ungulates such as deer and giraffes; or rodents such as mice, rats, hamsters and guinea pigs; and so on. An animal feed includes, but is not limited to, an aquaculture feed, a domestic animal feed including pet feed, a zoological animal feed, a work animal feed, a livestock feed, and combinations thereof.

In some embodiments, the composition is a feed or feed supplement for any animal whose meat or products are consumed by humans, such as any animal from which meat, eggs, or milk is derived for human consumption. When fed to such animals, nutrients such as LC-PUFAs can be incorporated into the flesh, milk, eggs or other products of such animals to increase their content of these nutrients.

In some embodiments, the composition is a spray-dried material that can be crumbled to form particles of an appropriate size for consumption by zooplankton, artemia, rotifers, and filter feeders. In some embodiments, the zooplankton, artemia, or rotifers fed by the composition are in turn fed to fish larvae, fish, shellfish, bivalves, or crustaceans.

In some embodiments, the composition is a pharmaceutical composition. Suitable pharmaceutical compositions include, but are not limited to, an anti-inflammatory composition, a drug for treatment of coronary heart disease, a drug for treatment of arteriosclerosis, a chemotherapeutic agent, an active excipient, an osteoporosis drug, an anti-depressant, an anti-convulsant, an anti-Helicobacter pylori drug, a drug for treatment of neurodegenerative disease, a drug for treatment of degenerative liver disease, an antibiotic, a cholesterol lowering composition, and a triacylglycerol lowering composition. In some embodiments, the composition is a medical food. A medical food includes a food that is in a composition to be consumed or administered externally under the supervision of a physician and that is intended for the specific dietary management of a condition, for which distinctive nutritional requirements, based on recognized scientific principles, are established by medical evaluation.

In some embodiments, the microbial oil can be formulated in a dosage form. Dosage forms can include, but are not limited to, tablets, capsules, cachets, pellets, pills, powders and granules, and parenteral dosage forms, which include, but are not limited to, solutions, suspensions, emulsions, and dry powders comprising an effective amount of the microbial oil. It is also known in the art that such formulations can also contain pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. Administration forms can include, but are not limited to, tablets, dragees, capsules, caplets, and pills, which contain the microbial oil and one or more suitable pharmaceutically acceptable carriers.

For oral administration, the microbial oil can be combined with pharmaceutically acceptable carriers well known in the art. Such carriers enable the microbial oils of the invention to be formulated as tablets, pills, dragees, capsules, liquids, gels, syrups, slurries, suspensions and the like, for oral ingestion by a subject to be treated. In some embodiments, the dosage form is a tablet, pill or caplet. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl cellulose, sodium carboxymethyl cellulose, and polyvinylpyrrolidone (PVP). If desired, disintegrating agents can be added, such as, but not limited to, the cross-linked polyvinylpyrrolidone, agar, or alginic acid or a salt thereof such as sodium alginate. Pharmaceutical preparations that can be used orally include, but are not limited to, push-fit capsules made of gelatin, as well as soft, sealed capsules made of gelatin and a plasticizer, such as glycerol or sorbitol. In some embodiments, the dosage form is a vegetarian dosage form, in which the dosage form is not formed from and does not contain any components from an animal source. In some embodiments, the vegetarian dosage form is a vegetarian capsule.

In some embodiments, the dosage form has a target fill weight of 200 mg to 1500 mg, 250 mg to 1250 mg, 300 mg to 1100 mg, or 350 mg to 1000 mg. In some embodiments, the dosage form has a target fill weight of 350 mg, 500 mg, 750 mg, 800 mg, 900 mg, or 1000 mg. The actual fill weight of the dosage form can deviate from the target fill weight by plus or minus 5%.

In some embodiments, an oil is provided comprising 120 mg to 220 mg EPA per one gram of oil and from 240 mg to 450 mg DHA per one gram of oil. In some embodiments, an oil is provided comprising 200 mg to 350 mg EPA per one gram of oil and from 500 mg to 700 mg DHA per one gram of oil. In some embodiments, an oil is provided comprising 250 mg to 300 mg EPA per one gram of oil and from 550 mg to 650 mg DHA per one gram of oil. In some embodiments, an oil is provided comprising 300 mg to 700 mg EPA per one gram of oil. In some embodiments, an oil is provided comprising 400 mg to 650 mg, or 500 mg to 600 mg EPA per one gram of oil. In some embodiments, the oral dosage form is a vegetarian capsule comprising modified cornstarch, carrageenan, glycerin, sorbitol, purified water, beta-caroten and caramel powder. In some embodiments, the capsule can be formulated with an oil comprised of 100 mg, 200 mg, 300 mg, 400 mg, 500 mg, 600 mg, 700 mg, 800 mg, or 950 mg of a combination of DHA and EPA.

In some embodiments, the composition is a cosmetic. Cosmetics include, but are not limited to, emulsions, creams, lotions, masks, soaps, shampoos, washes, facial creams, conditioners, make-ups, bath agents, and dispersion liquids. Cosmetic agents can be medicinal or non-medicinal.

In some embodiments, the composition is an industrial composition. In some embodiments, the composition is a starting material for one or more manufactures. A manufacture includes, but is not limited to, a polymer; a photographic photosensitive material; a detergent; an industrial oil; or an industrial detergent. For example, U.S. Patent No. 7,259,006 describes use of DHA-containing fat and oil for production of behenic acid and production of photographic sensitive materials using behenic acid.

### Methods of Using the Compositions

In some embodiments, the compositions can be used in the treatment of a condition in humans or non-human animals. In some embodiments, the compositions can be used for nutrition in humans or non-human animals.

The terms "treat" and "treatment" refer to both therapeutic treatment and prophylactic or preventative measures, wherein the object is to prevent or slow down (lessen) an undesired physiological condition, disease, or disorder, or to obtain beneficial or desired clinical results. For purposes of this invention, beneficial or desired clinical results include, but are not limited to, alleviation or elimination of the symptoms or signs associated with a condition, disease, or disorder; diminishment of the extent of a condition, disease, or disorder; stabilization of a condition, disease, or disorder, (i.e., where the condition, disease, or disorder is not worsening); delay in onset or progression of the condition, disease, or disorder; amelioration of the condition, disease, or disorder; remission (whether partial or total and whether detectable or undetectable) of the condition, disease, or disorder; or enhancement or improvement of a condition, disease, or disorder. Treatment includes eliciting a clinically significant response without excessive side effects. Treatment also includes prolonging survival as compared to expected survival if not receiving treatment.

In some embodiments, the composition is used to treat a condition, disease, or disorder such as acne, acute inflammation, age related maculopathy, allergy, Alzheimer's, arthritis, asthma, atherosclerosis, autoimmune disease, blood lipid disorder, breast cysts, cachexia, cancer, cardiac restenosis, cardiovascular diseases, chronic inflammation, coronary heart disease, cystic fibrosis, degenerative disorder of the liver, diabetes, eczema, gastrointestinal disorder, heart disease, high triacylglycerol levels, hypertension, hyperactivity, immunological diseases, inhibiting tumor growth, inflammatory conditions, intestinal disorders, kidney dysfunction, leukemia, major depression, multiple sclerosis, neurodegenerative disorder, osteoarthritis, osteoporosis, peroxisomal disorder, preeclampsia, preterm birth, psoriasis, pulmonary disorder rheumatoid arthritis, risk of heart disease, or thrombosis.

In some embodiments, the composition is used to increase the length of gestation of a fetus in the third trimester.

In some embodiments, the composition is used to control blood pressure.

In some embodiments, the composition is used to improve or maintain cognitive function.

In some embodiments, the composition is used to improve or maintain memory.

The composition or dosage form can be administered into the body of a subject by any route compatible with the composition or dosage form. A substance is considered to be "administered" if the substance is introduced into the body of the subject by the subject, or if another person, a machine, or a device introduces the substance into the body of the subject. "Administering," therefore, includes, e.g., self-administration, administration by others, and indirect administration. The term "continuous" or "consecutive," as used herein in reference to "administration," means that the frequency of administration is at least once daily. Note, however, that the frequency of administration can be greater than once daily and still be "continuous" or "consecutive," e.g., twice or even three times daily, as long as the dosage levels as specified herein are not exceeded. The means and methods for administration are known in the art and an artisan can refer to various pharmacologic references for guidance. For example, "Modern Pharmaceutics," Banker & Rhodes, Informa Healthcare, USA, 4th ed. (2002); and "Goodman & Gilman's The Pharmaceutical Basis of Therapeutics," McGraw-Hill Companies, Inc., New York, 10th ed. (2001) can be consulted.

By "subject," "individual," or "patient" is meant any subject, whether human or non-human, for whom diagnosis, prognosis, therapy, or administration of the composition or dosage form is desired. Mammalian subjects include, but are not limited to, humans; domestic animals; farm animals; zoo animals; sport animals; pet animals such as dogs, cats, guinea pigs, rabbits, rats, mice, or horses; primates such as monkeys (e.g., cebus, rhesus, African green, patas, cynomolgus, and cercopithecus), apes, orangutans, baboons, gibbons, and chimpanzees; canids such as dogs and wolves; felids such as cats, lions, and tigers; equids such as horses, donkeys, and zebras; food animals such as cows, cattle, pigs, and sheep; ungulates such as deer and giraffes; rodents such as mice, rats, hamsters and guinea pigs; and so on. The term subject also encompasses model animals, e.g., disease model animals. In some embodiments, the term subject includes valuable animals, either economically or otherwise, e.g., economically important breeding stock, racing animals, show animals, heirloom animals, rare or endangered animals, or companion animals. In certain embodiments, the subject is a human subject. In certain embodiments, the subject is a non-human subject.

The composition can be administered as a "nutritional amount," "therapeutically effective amount," a "prophylactically effective amount," a "therapeutic dose," or a "prophylactic dose." A "nutritional amount" refers to an amount effective, at dosages and for periods of time necessary, to achieve a desired nutritional result. A nutritional result can be, e.g., increased levels of a desirable fatty acid component in a subject. A "therapeutically effective amount" or "therapeutic dose" refers to an amount effective, at dosages and for periods of time necessary, to achieve a desired therapeutic result. A therapeutic result can be, e.g., lessening of symptoms, prolonged survival, improved mobility, and the like. A therapeutic result need not be a "cure." A "prophylactically effective amount" or "prophylactic dose" refers to an amount effective, at dosages and for periods of time necessary, to achieve the desired prophylactic result. Typically, since a prophylactic dose is used in subjects prior to or at an earlier stage of disease, a prophylactically effective amount will be less than a therapeutically effective amount for treatment of an advanced stage of disease.

Various dosage amounts of the composition, dosage form, or pharmaceutical composition can be administered to a subject, based on the amount of EPA or other fatty acid component of the microorganism, biomass, or microbial oil to be administered to the subject. The terms "daily dosage," "daily dosage level," and "daily dosage amount" refer herein to the total amount of EPA or other fatty acid component administered per day (per 24 hour period). Thus, for example, administration of EPA to a subject at a daily dosage of 2 mg means that the subject receives a total of 2 mg of EPA on a daily basis, whether the EPA is administered as a single dosage form comprising 2 mg EPA, or alternatively, four dosage forms comprising 0.5 mg EPA each (for a total of 2 mg EPA). In some embodiments, the daily amount of EPA is administered in a single dosage form, or in two dosage forms. The dosage forms of the present invention can be taken in a single application or multiple applications. For example, if four tablets are taken daily, each tablet comprising 0.5 mg EPA, then all four tablets can be taken once daily, or 2 tablets can be taken twice daily, or 1 tablet can be taken every 6 hours. In some embodiments, the daily dosage is about 100 mg to about 15 g of EPA. In some embodiments, the daily dosage is about 0.5 mg to about 250 mg, about 100 mg to about 250 mg, about 100 mg to about 500 mg, about 100 mg to about 1 g, about 1 g to about 2.5 g, about 1 g to about 5 g, about 1 g to about 10 g, about 1 g to about 15 g, about 5 g to about 10 g, about 5 g to about 15 g, about 10 g to about 15 g, about 100 mg to about 10 g, about 100 mg to about 5 g, or about 100 mg to about 2.5 g of EPA, DHA, or a combination thereof. In some embodiments, the composition is a dosage form that comprises about 0.5 mg to about 250 mg, 100 mg to about 250 mg, about 0.5 mg to about 500 mg, about 100 mg to about 500 mg, about 0.5 mg to about 1 g, or about 100 mg to about 1 g of EPA, DHA, or a combination thereof per dosage form.

In some embodiments, the daily dosage of omega-3 polyunsaturated fatty acids (including DHA and/or EPA) is 100 mg to 3000 mg, 200 mg to 2000 mg, 300 mg to 1500 mg, 400 mg to 1250 mg, or 500 mg to 1000 mg. In some embodiments, the daily dosage of a combination of DHA and EPA is 500 mg to 1000 mg. In some embodiments, the daily dosage of a combination of DHA and EPA is 100 mg, 250 mg, 500 mg, 750 mg, 1000 mg, 1500 mg, 2000 mg, 2500 mg, or 3000 mg.

Administration of the compositions or dosage forms of the present invention can be achieved using various regimens. For example, in some embodiments, administration occurs daily on consecutive days, or alternatively, occurs every other day (bi-daily). Administration can occur on one or more days.

Administration of the compositions and dosage forms can be combined with other regimens used for treatment of the condition. For example, the method of the present invention can be combined with diet regimens (e.g., low carbohydrate diets, high protein diets, high fiber diets, etc.), exercise regimens, weight loss regimens, smoking cessation regimens, or combinations thereof. The method of the present invention can also be used in combination with other pharmaceutical products in the treatment of the condition. The compositions or dosage forms of the present invention can be administered before or after other regimens or pharmaceutical products.

### Kits Comprising the Compositions

There are further disclosed kits or packages containing one or more units of a composition of the invention. Kits or packages can include units of a food product, pharmaceutical composition, cosmetic, or industrial composition comprising the microorganism, biomass as disclosed herein, or microbial oil of the invention, or combinations thereof. Kits or packages can also include an additive comprising the microorganism, biomass as disclosed herein, or microbial oil of the invention, or combinations thereof for preparation of a food, cosmetic, pharmaceutical composition, or industrial composition.

In some embodiments, the kit or package contains one or more units of a pharmaceutical composition to be administered according to the methods of the present invention. The kit or package can contain one dosage unit, or more than one dosage unit (i.e., multiple dosage units). If multiple dosage units are present in the kit or package, the multiple dosage units can be optionally arranged for sequential administration.

The kits as disclosed herein can optionally contain instructions associated with the units or dosage forms of the kits. Such instructions can be in a form prescribed by a governmental agency regulating the manufacture, use or sale of pharmaceutical products, which notice reflects approval by the agency of the manufacture, use or sale for human administration to treat a condition or disorder. The instructions can be in any form which conveys information on the use of the units or dosage forms in the kit according to the methods of the invention. For example, the instructions can be in the form of printed matter, or in the form of a pre-recorded media device.

In the course of examination of a patient, a medical professional can determine that administration of one of the methods of the present invention is appropriate for the patient, or the physician can determine that the patient's condition can be improved by the administration of one of the methods of the present invention. Prior to prescribing any regimen, the physician can counsel the patient, for example, on the various risks and benefits associated with the regimen. The patient can be provided full disclosure of all known and suspected risks associated with the regimen. Such counseling can be provided verbally, as well as in written form. In some embodiments, the physician can provide the patient with literature materials on the regimen, such as product information, educational materials, and the like.

Further disclosed are methods of educating consumers about the methods of treatment, the method comprising distributing the dosage forms with consumer information at a point of sale. In some embodiments, the distribution will occur at a point of sale having a pharmacist or healthcare provider.

The term "consumer information" can include, but is not limited to, an English language text, non-English language text, visual image, chart, telephone recording, website, and access to a live customer service representative. In some embodiments, consumer information will provide directions for use of the dosage forms according to the methods of the present invention, appropriate age use, indication, contraindications, appropriate dosing, warnings, telephone number or website address. In some embodiments, the method further comprises providing professional information to relevant persons in a position to answer consumer questions regarding use of the disclosed regimens according to the methods of the present invention. The term "professional information" includes, but is not limited to, information concerning the regimen when administered according to the methods of the present invention that is designed to enable a medical professional to answer costumer questions.

A "medical professional," includes, for example, a physician, physician assistant, nurse, nurse practitioner, pharmacist and customer service representative.

### Enriched Microbial Oils

The invention is directed to enriched or concentrated microbial oils having higher concentrations of polyunsaturated fatty acids, their esters, salts, alcohols, and/or aldehydes. The invention is directed to an enriched microbial oil comprising at least 70% by weight of a combination of DHA and EPA, wherein the oil comprises at least 10% by weight EPA, based on the weight of the oil. In some embodiments, the enriched microbial oil comprises at least 75% by weight, at least 80% by weight, or at least 85% by weight of a combination of DHA and EPA, wherein the oil comprises at least 10% by weight EPA, based on the weight of the oil. In some embodiments, the enriched microbial oil comprises at least 70% by weight, at least 75% by weight, at least 80% by weight, or at least 85% by weight of a combination of DHA and EPA, wherein the oil comprises at least 10% by weight, at least 15% by weight, at least 20% by weight, at least 25% by weight, or at least 30% by weight EPA, based on the weight of the oil.

In some embodiments, the present invention is directed to an enriched microbial oil comprising at least 50% by weight DHA and at least 20% by weight EPA, based on the weight of the oil. In some embodiments, the enriched microbial oil comprises at least 55% by weight, at least 60% by weight, at least 65% by weight, or at least 70% by weight DHA, based on the weight of the oil. In some embodiments, the enriched microbial oil comprises at least 25% by weight, at least 30% by weight, or at least 35% by weight EPA, based on the weight of the oil.

In some embodiments, the enriched microbial oil of the invention comprises 50% to 70%, 55% to 65%, or 53% to 63% by weight DHA, based on the weight of the oil. In some embodiments, the enriched microbial oil of the invention comprises 20% to 35%, 23% to 33%, or 25% to 30% by weight EPA, based on the weight of the oil.

There is further disclosed an enriched microbial oil comprising at least 30% by weight EPA, based on the weight of the oil. In some embodiments, the oil comprises at least 35%, at least 40%, at least 45%, at least 50%, at least 55%, at least 60%, at least 65%, or at least 70% by weight EPA, based on the weight of the oil. In some embodiments, the oil comprises 30% to 75% by weight, 35% to 70% by weight, 40% to 65% by weight, or 45% to 60% by weight, or 50% to 60% by weight EPA, based on the weight of the oil.

In some embodiments, the oils of the invention, including the enriched microbial oils of the invention, are obtained from a single source. In some embodiments, the oils of the invention are obtained from microorganisms of the same species. In some embodiments, the oils of the invention are obtained from a single strain of microorganism. In some embodiments, the oils of the invention are obtained from a single isolate or sub-isolate of a strain of microorganism.

In some embodiments, the DHA and EPA are in a triacylglycerol form. In some embodiments, the DHA and EPA are in an ester form. For example, the DHA and EPA ester can be an ethyl ester.

The present invention is directed to a food, supplement, or medicament comprising an enriched microbial oil of the invention.

In some embodiments, the enriched microbial oil is obtainable from an isolated microorganism of a *Schizochytrium* species.

In some embodiments, the oil of the invention is obtainable from an isolated microorganism of the species deposited under ATCC Accession No. PTA-10208, wherein the total fatty acids produced by the microorganism comprise more than 10% by weight EPA. In some embodiments, the oil of the invention is obtainable from an isolated microorganism of the species deposited under ATCC Accession No. PTA-10208, wherein the total fatty acids produced by the microorganism comprises more than 11%, more than 12%, more than 13%, more than 14%, more than 15%, more than 16%, more than 17%, more than 18%, more than 19%, or more than 20% by weight EPA.

In some embodiments, the oil of the invention is obtainable from an isolated microorganism having the characteristics of the species deposited under ATCC Accession No. PTA-10208, wherein the total fatty acids produced by the microorganism comprise more than about 10% by weight EPA. In some embodiments, the oil of the invention is obtainable from an isolated microorganism having the characteristics of the species deposited under ATCC Accession No. PTA-10208, wherein the total fatty acids produced by the microorganism comprises more than 11%, more than 12%, more than 13%, more than 14%, more than 15%, more than 16%, more than 17%, more than 18%, more than 19%, or more than 20% by weight EPA.

In some embodiments, the oil of the invention is obtainable from an isolated microorganism that produces a triacylglycerol fraction, wherein EPA content of the triacylglycerol fraction is at least 12% by weight. In some embodiments, the oil of the invention is obtainable from an isolated microorganism that produces a triacylglycerol fraction, wherein the EPA content of the triacylglycerol fraction is at least 13%, at least 14%, at least 15%, at least 16%, at least 17%, at least 18%, at least 19%, or at least 20% by weight. In some embodiments, the oil of the invention is obtainable from an isolated microorganism that produces a triacylglycerol fraction, wherein the EPA content of the triacylglycerol fraction is 12% to 55%, 12% to 50%, 12% to 45%, 12% to 40%, 12% to 35%, 12% to 30%, 15% to 45%, 15% to 40%, 15% to 35%, 15% to 30%, or 20% to 30% by weight.

In some embodiments, the oil of the invention is obtainable from an isolated microorganism deposited under ATCC Accession No. PTA-10208.

In some embodiments, the oil of the invention is obtainable from an isolated microorganism deposited under ATCC Accession No. PTA-10209.

In some embodiments, the oil of the invention is obtainable from an isolated microorganism deposited under ATCC Accession No. PTA-10210.

In some embodiments, the oil of the invention is obtainable from an isolated microorganism deposited under ATCC Accession No. PTA-10211.

### Antioxidants

The oils of the invention can optionally include an antioxidant. Examples of antioxidant(s) that, individually or in any combination, can be optionally included in any oil of this disclosure include: ascorbic acid, an ester of ascorbic acid, a salt of ascorbic acid, glutathione, a carotene, beta-carotene, vitamin E, alpha-tocopherol, beta-tocopherol, gamma-tocopherol, delta-tocopherol, alpha-tocotrienol, beta-tocotrienol, gamma-tocotrienol, delta-tocotrienol, ubiquinol, ascorbic acid and/or ubiquinone.

Vitamin E, for purposes of this application, is defined as alpha-tocopherol, beta-tocopherol, gamma-tocopherol, delta-tocopherol, alpha-tocotrienol, beta-tocotrienol, gamma-tocotrienol, delta-tocotrienol, or a combination thereof.

The antioxidant(s) can optionally be present in any oil of this disclosure such that the amount of these, in total, ranges for example from 0.001 % by weight to 5% by weight, including all values and subranges therebetween as if explicitly written out, based on the total weight of the oil. The total amount of antioxidant(s) can be, for example, 0.01 % by weight, 0.01 % by weight, 0.1 % by weight, 1 % by weight, 2 % by weight, 3 % by weight, or 4 % by weight, based on the total weight of the oil.

These antioxidant(s), individually or in any combination, can be optionally excluded from any oil of this disclosure.

### Aldehydes and Alcohols

An aldehyde contains the functional group: -C(O)H.

An alcohol contains the functional group: -OH.

### Acids

An acid contains the functional group: -C(O)OH.

Acids of high interest in this application are fatty acids. Fatty acids of particular interest include omega-3 fatty acids, omega-6 fatty acids, and combinations thereof. Fatty acids of very particular interest include DHA, EPA, or a combination of these.

### Acid Salts

An acid salt contains the functional group: -C(O)O⁻ M⁺, wherein -C(O)O⁻ is an anion and M⁺ is a cation. M⁺ can be an inorganic cation or an organic cation. The number of -C(O)O⁻ anion groups associated with the cation can be adjusted to meet the cation valence. For example, M⁺ can be any cation of a metal from Group I or Group II of the Periodic Table of the Elements. Specific examples of inorganic cations include Na⁺, K⁺, Li⁺, Rb⁺, Cs⁺, Fr⁺, Ca²⁺, Mg²⁺, Be²⁺, Sr²⁺, Ba²⁺, Ra²⁺, and ammonium. Cations can also be organic. For example, organic cations can be protonated, or nitrogen quaternized, amino acids such as: arginine, histidine, lysine, aspartic acid, glutamic acid, serine, threonine, asparagine, glutamine, cysteine, glycine, proline, alanine, isoleucine, leucine, methionine, phenylalanine, glycine, valine, phenylalanine, tryptophan, and tyrosine. The protonated or quaternized amino acids can be racemic or of the D or L configuration.

Acid salts of high interest in this application are fatty acid salts. Fatty acid salts of particular interest include salts of an omega-3 fatty acid, salts of an omega-6 fatty acid, and combinations thereof. Fatty acid salts of very particular interest include a DHA salt, an EPA salt, or a combination of these.

### Esters

In some embodiments, a fatty acid ester includes an ester of an omega-3 fatty acid, omega-6 fatty acid, and combinations thereof. In some embodiments, the fatty acid ester is a DHA ester, an EPA ester, or a combination thereof. In some embodiments, an oil or fraction thereof as described herein is esterified to produce an oil or fraction thereof comprising fatty acid esters. The term "ester" refers to the replacement of the hydrogen in the carboxylic acid group of the fatty acid molecule with another substituent. Typical esters are known to those in the art, a discussion of which is provided by Higuchi, T. and V. Stella in Pro-drugs as Novel Delivery Systems, Vol. 14, A.C.S. Symposium Series, Bioreversible Carriers in Drug Design, Ed. Edward B. Roche, American Pharmaceutical Association, Pergamon Press, 1987, and Protective Groups in Organic Chemistry, McOmie ed., Plenum Press, New York, 1973. Examples of esters include methyl, ethyl, propyl, butyl, pentyl, t-butyl, benzyl, nitrobenzyl, methoxybenzyl, benzhydryl, and trichloroethyl. In some embodiments, the ester is a carboxylic acid protective ester group, esters with aralkyl (e.g., benzyl, phenethyl), esters with lower alkenyl (e.g., allyl, 2-butenyl), esters with lower-alkoxy-lower-alkyl (e.g., methoxymethyl, 2-methoxyethyl, 2-ethoxyethyl), esters with lower-alkanoyloxy-lower-alkyl (e.g., acetoxymethyl, pivaloyloxymethyl, 1-pivaloyloxyethyl), esters with lower-alkoxycarbonyl-lower-alkyl (e.g., methoxycarbonylmethyl, isopropoxycarbonylmethyl), esters with carboxy-lower alkyl (e.g., carboxymethyl), esters with lower-alkoxycarbonyloxy-lower-alkyl (e.g., 1-(ethoxycarbonyloxy)ethyl, 1-(cyclohexyloxycarbonyloxy)ethyl), esters with carbamoyloxy-lower alkyl (e.g., carbamoyloxymethyl), and the like. In some embodiments, the added substituent is a linear or cyclic hydrocarbon group, e.g., a C1-C6 alkyl, C1-C6 cycloalkyl, C1-C6 alkenyl, or C1-C6 aryl ester. In some embodiments, the ester is an alkyl ester, e.g., a methyl ester, ethyl ester or propyl ester. In some embodiments, the ester substituent is added to the free fatty acid molecule when the fatty acid is in a purified or semi-purified state. Alternatively, the fatty acid ester is formed upon conversion of a triacylglycerol to an ester.

Esters of high interest in this application are fatty acid esters. Fatty acid esters of particular interest include esters of an omega-3 fatty acid, esters of an omega-6 fatty acid, and combinations thereof. Fatty acid esters of very particular interest include a DHA ester, an EPA ester, or a combination thereof.

Esters can be glyceride esters (i.e., esters formed from acids and glycerol). Glyceride esters can take the form of mono-acyl glycerides, di-acyl glycerides, and / or tri-acyl glycerides. Glyceride esters of high interest in this application are poly-unsaturated fatty acid containing glyceride esters. Glyceride esters of particular interest include glyceride esters of an omega-3 fatty acid, an omega-6 fatty acid, and combinations thereof. Glyceride esters of very particular interest include a DHA containing glyceride ester, an EPA containing glyceride ester, or a combination thereof.

### Enrichment Techniques to Produce Enriched Microbial Oils

Various methods are known in the art for increasing the concentration of polyunsaturated fatty acids in a composition. Examples of such methods are disclosed, for example, in U.S. Patent No. 7,588,791; U.S. Patent No. 6,528,669; U.S. Patent No. 6,395,778; U.S. Application Publication No. 2011/0098356; and U.S. Application Publication No. 2010/0130608.

In the present invention, any concentrating, reacting, and/or purifying technique can be combined with any other concentrating, reacting, and/or purifying technique to produce microbial oils enriched in: polyunsaturated fatty acids, their esters, their salts, aldehydes thereof and/or alcohols thereof. The enrichment techniques can be used in any order and combination. In some embodiments, a crude or refined microbial oil can be used as the starting material for producing the enriched microbial oils of the invention. For example, simple winterization of a crude or refined microbial oil can produce a first enriched microbial oil that is enriched in polyunsaturated fatty acid containing tri-acyl glycerides. The first enriched microbial oil can, for example, then undergo base catalyzed tri-acyl glyceride hydrolysis to produce a second enriched microbial oil containing and enriched in polyunsaturated fatty acids. The second enriched microbial oil can, for example, then be fractionally distilled under reduced pressure to further concentrate the polyunsaturated fatty acids, thereby producing a third enriched microbial oil. The third enriched microbial oil can then, for example, be further enriched in polyunsaturated fatty acid content via use of high performance liquid chromatography to produce a fourth microbial oil enriched in polyunsaturated fatty acids. In a preferred embodiment, a biomass can be used as the starting material for producing the enriched microbial oils of the invention. Biomass as used here refers to an at least partially dewatered algal culture broth.

Tri-acyl glycerides are preferred materials from which fatty acids (and in particular polyunsaturated fatty acids), their salts, their aldehydes, their alcohols, and /or their non-glyceride esters are made. The tri-acyl glycerides can be present in crude microbial oil or in a refined microbial oil. The tri-acyl glycerides can be concentrated from a crude or refined microbial oil prior to conducting any chemical reactions which result in obtaining fatty acids (and in particular polyunsaturated fatty acids), their salts, their aldehydes, their alcohols, and / or their non-glyceride esters.

The content of the tri-acyl glycerides in the crude or refined microbial oil can range, for example, from 10 % to 99 % by weight, including all values and subranges therebetween as if explicitly written out, based on the total weight of the crude or refined microbial oil. For example, the tri-acyl glyceride content of the crude or refined microbial oil can be 15 %, 20 %, 25 %, 30 %, 35 %, 40 %, 45 %, 50 % , 60 %, 70 %, 80 %, 90%, 95 %, or 99 % by weight, based on the weight of the microbial oil.

The crude or refined microbial oil can be manipulated to increase the polyunsaturated fatty acid containing tri-acyl glyceride content therein by, for example, simple winterization.

### Simple Winterization

In simple winterization, the crude or refined microbial oil is cooled in the absence of solvent, and a solid fraction precipitates from the cooled oil. The solid fraction contains tri-acyl glycerides that contain predominantly saturated fatty acids attached thereto via ester linkages. The liquid fraction contains tri-acyl glycerides that contain predominantly poly-unsaturated fatty acids attached thereto via ester linkages. After cooling, the liquid fraction is separated from the solid fraction by, for example, decantation and / or filtration and /or via centrifugation followed by decantation and / or filtration. Evaporation, where appropriate, can also be affected. If carried out, centrifugation can be, for example, at from 1,000 - 4,000 g, including all values and subranges therebetween as if explicitly written out.

In the simple winterization, the cooling temperature can range, for example, from 0°C to -120°C, including all values and subranges therebetween as if explicitly written out. For example, the cooling temperature can be -5°C, -10°C, -20°C, -30°C, -40°C, - 50°C, -60°C, -70°C, -80°C, -90°C, -100°C, -110°C, or -120°C. The cooling time can range from 1 minute to 5 days, including all values and subranges therebetween as if explicitly written out. The cooling time can be, for example, 5 minutes, 10 minutes, 20 minutes, 30 minutes, 40 minutes, 50 minutes, 60 minutes, 2 hours, 3 hours, 4 hours, 5 hours, 6 hours, 7 hours, 8 hours, 9 hours, 10 hours, 11 hours, 12 hours, 24 hours, 2 days, 3 days, or 4 days.

After simple winterization, the content of tri-acyl glycerides (containing poly-unsaturated fatty acids attached thereto via ester linkages) in the simple winterized oil is enhanced, and can, for example, range from 20 % to 99.9 % by mass, including all values and subranges therebetween as if explicitly written out, based on the total mass of the simple winterized oil. For example, the poly-unsaturated fatty acid containing tri-acyl glyceride content of the simple winterized oil can be 25 %, 30 %, 35 %, 40 %, 45 %, 50 %, 55 %, 60 %, 65 %, 70 %, 75 %, 80%, 90 %, 95 %, 99 %, or 99.9 % by mass, based on the total mass of the simple winterized oil.

The poly-unsaturated fatty acid content in the tri-acyl glycerides of the simple winterized oil can range, for example, from 30 % of all tri-acyl glyceride ester bonds (e.g., 30 % of the tri-acyl glyceride ester bonds are to poly-unsaturated fatty acids) to 100 % of all tri-acyl glyceride ester bonds, including all values and subranges therebetween as if explicitly written out. For example, the poly-unsaturated fatty acid content of the tri-acyl glycerides can be 25 %, 30 %, 35 %, 40 %, 45 %, 50 %, 55 %, 60 %, 65 %, 70 %, 80 %, 90%, or 95% of all tri-acyl glyceride ester bonds in the tri-acyl glycerides in the simple winterized oil.

The crude or refined microbial oil can be manipulated to increase the poly-unsaturated fatty acid containing tri-acyl glyceride content therein by, for example, complex winterization.

### Complex Winterization

In complex winterization, the crude or refined microbial oil is cooled in the presence of a solvent, and a solid fraction precipitates from the cooled oil. The solid fraction contains tri-acyl glycerides that contain predominantly saturated fatty acids attached thereto via ester linkages. The liquid fraction contains tri-acyl glycerides that contain predominantly poly-unsaturated fatty acids attached thereto via ester linkages. After cooling, the liquid fraction is separated from the solid fraction by, for example, decantation and / or filtration and /or via centrifugation followed by decantation and / or filtration. Evaporation, where appropriate, can also be affected. If carried out, centrifugation can be, for example, at 1,000 - 4,000 g, including all values and subranges therebetween as if explicitly written out.

The solvent can be, for example, ethanol, methanol, propane, butane, butyl acetate, ethyl acetate, carbon dioxide, supercritical carbon dioxide, acetone, dinitrogen monoxide, hexane, ethyl methyl ketone, isopropanol, dichloromethane, or any combination of these solvents.

When two solvents are used together, the volume / volume ratio of the two solvents can range from 5:95 to 95:5, including all values and subranges therebetween as if explicitly written out, where the total combined solvent volume is 100%. For example, the volume / volume ratio can be 10:90, 20:80, 30:70, 40:60, 50:50, 60:40, 70:30, 80:20 or 90:10.

The ratio of solvent to oil can range, for example, from 1 g of oil : 1 mL of solvent to 1 g of oil : 100 mL of solvent, including all values and subranges therebetween as if explicitly written out. For example, the ratio of oil to solvent can be 1 g : 2 mL, 1g : 3 mL, 1 g : 4 mL, 1 g : 5 mL, 1 g : 6 mL, 1g : 7 mL, 1 g : 8 mL, 1 g : 9 mL, 1 g : 10 mL, 1 g : 20 mL, 1 g : 30 mL, 1 g : 40 mL, 1 g : 50 mL, 1 g: 60 mL, 1 g : 70 mL, 1 g : 80 mL, or 1 g : 90 mL.

In the complex winterization, the cooling temperature can range, for example, from 0°C to -120°C, including all values and subranges therebetween as if explicitly written out. For example, the cooling temperature can be -5°C, -10°C, -20°C, -30°C, - 40°C, -50°C, -60°C, -70°C, -80°C, -90°C, -100°C, -110°C, or -120°C. The cooling time can range from 1 minute to 5 days, including all values and subranges therebetween as if explicitly written out. The cooling time can be, for example, 5 minutes, 10 minutes, 20 minutes, 30 minutes, 40 minutes, 50 minutes, 60 minutes, 2 hours, 3 hours, 4 hours, 5 hours, 6 hours, 7 hours, 8 hours, 9 hours, 10 hours, 11 hours, 12 hours, 24 hours, 2 days, 3 days, or 4 days.

After complex winterization, and solvent removal, the content of tri-acyl glycerides (containing poly-unsaturated fatty acids attached thereto via ester linkages) in the complex winterized oil is enhanced, and can, for example, range from 20 % to 99.9 % by mass, including all values and subranges therebetween as if explicitly written out, based on the total mass of the complex winterized oil. For example, the poly-unsaturated fatty acid containing tri-acyl glyceride content of the complex winterized oil can be 25 %, 30 %, 35 %, 40 %, 45 %, 50 %, 55 %, 60 %, 65 %, 70 %, 75 %, 80 % , 90 %, 95 %, 99 %, or 99.9 % by mass, based on the total mass of the complex winterized oil.

The poly-unsaturated fatty acid content in the tri-acyl glycerides of the complex winterized oil can range, for example, from 20 % to 100 % of all tri-acyl glyceride ester bonds (e.g., 20 % to 100 % of all tri-acyl glyceride ester bonds are bonded to poly-unsaturated fatty acids), including all values and subranges therebetween as if explicitly written out. For example, 25 %, 30 %, 35 %, 40 %, 45 %, 50 %, 55 %, 60 %, 60 %, 70 %, 80 %, 90 %, 95 %, or 99% of the ester bonds in the simple winterized oil can be bonded to poyl-unsaturated fatty acid(s).

The content of U.S. Patent No. 7,588,791 is referred to in its entirety.

### Hydrolysis

Fatty acids, and particularly poly-unsaturated fatty acids, can be obtained by hydrolyzing tri-acyl glycerides and / or di-acyl glycerides and / or mono-acyl glycerides that contain fatty acids, particularly poly-unsaturated fatty acids. The tri-acyl glycerides are contained in a crude or refined microbial oil which can have optionally undergone simple winterization and / or complex winterization. The hydrolysis can be affected with a base such as a metal hydroxide, for example, sodium hydroxide, potassium hydroxide, lithium hydroxide, calcium hydroxide, magnesium hydroxide, cesium hydroxide, or any combination of these. The ratio of base (e.g. metal hydroxide) to oil, on a weight : weight basis, can range from 1 : 1 to 1000 : 1, including all values and subranges therebetween as if explicitly written out. For example, a weight : weight ratio of base to oil can be: 2 : 1, 3 : 1, 4 : 1, 5 : 1, 6 : 1, 7 : 1, 8 : 1, 9 : 1, 10 : 1, 20 : 1, 30 : 1, 40 : 1, 50 : 1, 60 : 1, 70 : 1, 80 : 1, 90 : 1, 100 : 1, 200 : 1, 300 : 1, 400 : 1, 500 : 1, 500 : 1, 700 : 1, 800: 1, or 900 : 1. The hydrolysis can be run in water, and optionally employing a co-solvent that can be wholly, partially, or not miscible at all with the water. Co - solvents can include, but are not limited to, diethyl ether, methanol, ethanol, hexane, pentane, ethylacetate, N, N-dimethyl formamide, dimethylsulfoxide, and / or a crown ether. The crown ether can be, for example, 12-crown-4, 15-crown-5, 18-crown-6, dibenzo-18-crown-6, diaza-18-crown 6, or a combination of these.

The hydrolysis reaction can be run at a temperature ranging from 10°C to 80°C, including all values and subranges therebetween as if explicitly written out. The hydrolysis reaction can be run, for example at 15°C, at 20°C, at 30°C, at 40°C, at 50°C, at 60°C, or at 70°C.

The hydrolysis reaction can be done for a time period ranging from 1 hour to 1 week, including all subranges and values therebetween as if explicitly written out. For example, the hydrolysis reaction can be run for 2 hours, 4 hours, 6 hours, 8 hours, 10 hours, 12 hours, 14 hours, 16 hours, 18 hours, 20 hours, 22 hours, 24 hours, 2 days, 3 days, 4 days, 5 days, or 6 days.

Workup of the hydrolysis reaction can be done in any number of ways known to those of skill in the art. If the hydrolysis reaction is worked up with a proton source (that can be diluted with water before commencing workup), for example ammonium chloride, or a mineral acid, free acid molecules of hydrolyzed fatty acid will result. Mineral acids include hydrochloric acid, sulfuric acid, and / or phosphoric acid.

Alternatively, the fatty acid salts formed in the hydrolysis reaction can be isolated by concentration, for example, by removal of water (and optionally co-solvents) from the hydrolysis reaction. Solvent removal from the fatty acid or fatty acid salt containing reaction can be affected under reduced atmospheric pressure, for example, in a rotary evaporator or a lyophilizer or a TurboVap®. The reduced atmospheric pressure can be affected by, for example, an aspirator or vacuum pump.

The reduced pressure can range from 0.0001 atmospheres to 0.9 atmospheres, including all values and subranges therebetween as if explicitly written out. An atmosphere is abbreviated "atm" and is equivalent to 101,325 Pa. The reduced pressure can be, for example, 0.001 atm, 0.01 atm, 0.1 atm, 0.2 atm, 0.3 atm, 0.4 atm, 0.5 atm, 0.6 atm, 0.7 atm, or 0.8 atm.

### Ester Formation / Transesterification

Fatty acid esters, in particular poly-unsaturated fatty acid esters, can be made in ways that are known to one of ordinary skill in the art.

For example, tri-acyl glycerides, di-acyl glycerides, and /or mono-acyl glycerides that contain fatty acids, particularly poly-unsaturated fatty acids, can be reacted with an alcohol in the presence of an acid or a base to produce esters. The disclosure of U.S. Patent Application No. 12/163,555, that published as U.S. Patent Application Pre-Grant Publication No. 2009/0023808.

Alcohols can include, for example, C₁-C₆ alkyl alcohols, for example, ethanol, methanol, n-propanol, iso-propanol, n-butanol, iso-butanol, sec-butanol, tert-butanol, n-pentanol, and n-hexanol. The alcohol can be used as the reaction solvent and co-reactant, either alone or with a co-solvent. The amount of the alcohol can range from 25 % to 50 % by weight of the reaction mixture, including all values and subranges therebetween as if explicitly written out. For example, the amount of alcohol in the reaction mixture can be 30%, 35%, 40% or 45% by weight of the reaction mixture.

The base can be, for example, a metal alkyloxide. Metal alkyloxides include sodium ethoxide, sodium methoxide, sodium n-propoxide, sodium iso-propoxide, sodium n-butoxide, sodium iso-butoxide, sodium sec-butoxide, sodium, tert-butoxide, sodium n-pentoxide, sodium n-hexoxide, lithium ethoxide, lithium methoxide, lithium n-propoxide, lithium iso-propoxide, lithium n-butoxide, lithium iso-butoxide, lithium sec-butoxide, lithium, tert-butoxide, lithium n-pentoxide, lithium n-hexoxide, potassium ethoxide, potassium methoxide, potassium n-propoxide, potassium iso-propoxide, potassium n-butoxide, potassium iso-butoxide, potassium sec-butoxide, potassium, tert-butoxide, potassium n-pentoxide, and /or potassium n-hexoxide.

In some situations, the base can be made by adding sodium metal, potassium metal, or lithium metal to an alcoholic solution.

In some situations, the base can be made by adding a metal hydride, such as lithium hydride, sodium hydride, or potassium hydride, to an alcoholic solution.

The ratio of base to oil, on a weight: weight basis can, for example, range from 1 : 1 to 1000 : 1, including all values and subranges therebetween as if explicitly written out. For example, the ratio of base to oil, on a weight to weight basis, can be 2 : 1, 3 : 1, 4 : 1, 5 : 1, 6 : 1, 7 : 1, 8 : 1, 9 : 1, 10 : 1, 20 : 1, 30 : 1, 40 : 1, 50 : 1, 60 : 1, 70 : 1, 80 : 1, 90 : 1, 100 : 1, 200 : 1, 300 : 1, 400 : 1, 500 ; 1, 600 : 1, 700 : 1, 800 : 1, or 900 : 1.

The esterification reaction can be run at a temperature ranging from 10 °C to 100 °C, including all values and subranges therebetween as if explicitly written out. For example, the esterification reaction can be run at 20 °C, 30 °C, 40 °C, 50 °C, 60 °C, 70 °C, 80 °C, or 90 °C.

The esterification reaction can be run open to the atmosphere, or under an inert atmosphere such as nitrogen or argon.

Workup and isolation of the fatty acid esters can be done in ways known to one of ordinary skill, for example, by extraction with an organic solvent and / or water. The organic solvent can be, for example, pentane, hexane, di-ethyl ether, ethyl acetate, or a combination of these. The water can optionally contain other substances such as sodium bicarbonate, sodium carbonate, ammonium chloride and / or dilute mineral acid.

### Reduction

Additionally, fatty acid esters can be reduced to form alcohols or aldehydes of the fatty acids.

For example, the tri-acyl glycerides can be treated with reducing reagents such as LiBH₄, optionally in the presence of MeOH and /or diglyme; LiBH₄ in the presence of trimethylsilyl chloride; NaBH₄ in the presence of boron tri fluoride etherate; NaBH₄ in the presence of the AlCl₃ or TiCl₄ or H₂SO₄; Zn(BH4)₂; iBuAlH; LiAlH₄ optionally in the presence of AlCl₃; and / or NaAlH₄. General reagents to achieve the reduction are described in Larock, R. C., Comprehensive Organic Transformations, 2nd Edition, (1999).

The reduction can be run, for example, at a temperature ranging from -78°C to 80°C, including all values and subranges therebetween as if explicitly written out. For example, the reduction can be performed at -70°C, -60°C, -50°C, -40°C, -30°C, -20°C, - 10°C, 0°C, 10°C, 20°C, 30°C, 40°C, 50°C, 60°C, or 70°C.

The reduction is preferably performed under an inert atmosphere such as nitrogen or argon.

The choice of reaction solvent can vary but can include, for example, tetrahydrofuran, diethyl ether, hexane, pentane, tetrahydropyran, or any combination of these. The solvent is preferably inert to the reducing reagent. The solvent is preferably anhydrous.

The ratio of the reducing reagent to the tri-acyl glyceride, on a mol : mol basis, can range 0.33 : 1 to 50 : 1, including all values, ranges, and subranges therebetween as if explicitly written out. For example, the ratio of reducing reagent to tri-acyl glyceride on a mol : mol basis can be 0.5 : 1, 0.6 : 1, 0.7 : 1, 0.8 : 1, 0.9 : 1, 1 : 1, 2 : 1, 3 : 1, 4 : 1, 5 : 1, 6 : 1, 7 : 1, 8 : 1, 9 : 1, 10 : 1, 20 : 1, 30 : 1, 40 : 1, or 50 : 1.

### Oxidation

The alcohols of the fatty acids resulting from the reduction can be converted to aldehydes by conducting an oxidation. The oxidizing agent can be, for example, dimethyl sulfoxide with a pyridine-sulfur trioxide complex and di-isopropyl ethyl amine; C₆F₅SeO₂H; tetrapropylammonium perruthenate; and / or the Dess-Martin periodinane reagent ("DMP"). General reagents to achieve the reduction are described in Larock, R. C., Comprehensive Organic Transformations, 2nd Edition, (1999).

The oxidation can be run, for example, at a temperature ranging from -78°C to 80°C, including all values and subranges therebetween as if explicitly written out. For example, the oxidation can be performed at -70°C, -60°C, -50°C, -40°C, -30°C, -20°C, - 10°C, 0°C, 10°C, 20°C, 30°C, 40°C, 50°C, 60°C, or 70°C.

The oxidation is preferably performed under an inert atmosphere such as nitrogen or argon.

The choice of reaction solvent can vary but can include, for example, tetrahydrofuran, diethyl ether, hexane, pentane, tetrahydropyran, or any combination of these. The solvent is preferably inert to the oxidizing reagent. The solvent is preferably anhydrous, although water can be added at any point during the oxidation.

The ratio of the oxidizing reagent to the alcohol, on a mol : mol basis, can range 0.33 : 1 to 50 : 1, including all values, ranges, and subranges therebetween as if explicitly written out. For example, the ratio of oxidizing reagent to alcohol on a mol : mol basis can be 0.5 : 1, 0.6 : 1, 0.7 : 1, 0.8 : 1, 0.9 : 1, 1 : 1, 2 : 1, 3 : 1, 4 : 1, 5 : 1, 6 : 1, 7 : 1, 8 : 1, 9 : 1, 10 : 1, 20 : 1, 30 : 1, 40 : 1, or 50 : 1.

The poly-unsaturated fatty acids, esters thereof, salts thereof, aldehydes made therefrom, or alcohols made therefrom can be enriched by purification.

### HPLC Purification / Enrichment

For example, poly-unsaturated fatty acids, salts thereof, esters thereof, aldehydes formed therefrom, and/or alcohols formed therefrom (collectively "the desired product(s)") formed therefrom, can be purified by high-performance liquid phase chromatography using, for example, a reverse phase column. The reverse phase column can be, for example, a C8 column, a C18 column, a C19 column, a C20 column, a C21 column, or a C22 column.

The desired product(s) can be eluted with a mobile phase that is a single solvent, a solvent mixture, or with a solvent gradient employing two solvents. Solvents include, but are not limited to, acetonitrile, methanol, ethanol, n-propanol, i-propanol, acetone, ethyl acetate, water, water with trace amounts (e.g., 0.001% - 0.1% volume / volume) of trifluoroacetic acid or formic acid, hexane, pentane, and combinations thereof. The desired products can also be eluted with high pressure or supercritical carbon dioxide.

The desired product(s) can be detected as they exit the column, for example, by uv-visible, diode arrays, refractive index and/or fluorescence detectors.

The desired product(s) can be collected in fractions (e.g., in test tubes) in a fraction collector, and then can be concentrated by solvent removal, under reduced pressure, or alternatively by blowing an inert atmosphere over the collected factions, to yield an oil enriched in the desired product(s).

### Chromatotron® Purification / Enrichment

Another way to concentrate the desired product(s) in the oil is to purify the desired product(s) with a Chromatotron®. The Chromatotron® is a preparative, centrifugally accelerated, radial, thin-layer chromatograph. The crude oil is loaded on the Chromatotron®, optionally in the presence of one or more solvents such as dimethyl sulfoxide, N,N-dimethyl formamide, acetone, ethyl acetate, hexane, methanol, ethanol, propanol, tetrahydrofuran, diethyl ether, pentane, dichloromethane, chloroform, and tetrahydropyran. The radial plate of the Chromatotron® can be coated with a stationary phase separating medium that can be silica gel, alumina, and / or silica gel impregnated with silver nitrate.

The desired product(s) can be collected in fractions (e.g., in test tubes) and then can be concentrated by solvent removal, under reduced pressure, or alternatively by blowing an inert atmosphere over the collected factions, to yield an oil enriched in the desired product(s).

### Column Chromatography

Another way to concentrate the desired product(s) in the oil is to purify the desired products with column chromatography. A column is packed with a stationary phase such as silica gel, alumina, and / or silica gel impregnated with silver nitrate, and wetted with a solvent or a mixture of solvents. The oil containing the desired product(s) is loaded onto the column, and eluted with one or more mobile phase solvents such as dimethyl sulfoxide, N, N-dimethyl formamide, ethyl acetate, hexane, methanol, acetone, ethanol, propanol, tetrahydrofuran, diethyl ether, pentane, dichloromethane, chloroform, and tetrahydropyran.

The desired product(s) can be collected in fractions (e.g., in test tubes) and then can be concentrated by solvent removal, under reduced pressure, or alternatively by blowing an inert atmosphere over the collected factions, to yield an oil enriched in the desired product(s).

### Vacuum Molecular Fractional Distillation / Enrichment

Another way to concentrate the desired product(s) in the oil is to purify the desired product(s) with fractional distillation. The oil is placed in a heating flask, and the flask is heated, optionally under reduced pressure. In one example, the desired product(s) convert to vapor phase when their boiling points are reached and pass through a fractionating column, are condensed in a condenser, and collected in a receiving flask. In an alternative example, the desired product(s) stay in the heating flask and impurities distill away from the desired product(s).

The fractional distillation can be run at a temperature ranging from, for example, 40°C to 500°C, including all values and subranges therebetween as if explicitly written out. For example, the fractional distillation can be run at 50°C, 60°C, 70°C, 80°C, 90°C, 100°C, 110°C, or 120°C, 130°C, 140°C, 150°C, 160°C, 170°C, 180°C, 190°C, 200°C, 210°C, 220°C, 230°C, or 240°C, 250°C, 260°C, 270°C, 280°C, 290°C, 300°C, 310°C, 320°C, 330°C, 340°C, 350°C, or 360°C, 370°C, 380°C, 390°C, 400°C, 410°C, 420°C, 430°C, 440°C, 450°C, 460°C, 470°C, 480°C, or 490°C.

The fractional distillation is preferably conducted under reduced pressure. The reduced pressure can range from 0.0001 atmospheres to 0.9 atmospheres, including all values and subranges therebetween as if explicitly written out. An atmosphere is abbreviated "atm" and is equivalent to 101,325 Pa. The reduced pressure can be, for example, 0.001 atm, 0.01 atm, 0.1 atm, 0.2 atm, 0.3 atm, 0.4 atm, 0.5 atm, 0.6 atm, 0.7 atm, or 0.8 atm.

### Urea Adduct Crystallization

Another way to concentrate the desired product(s) in the oil is to purify the desired product(s) with urea precipitation and /or urea adduct crystallization. In this, the oil containing the desired product(s) is dissolved in a solvent, for example methanol. The solvent, for example methanol, is warmed, for example, to a range of 40°C to 70°C, including all values and subranges therebetween as if explicitly written out. For example, the methanol solution of the oil containing the desired products can be heated to 45°C, 50°C, 55°C, 60°C, or 65°C, or 70°C. Urea is then dissolved in the heated solvent oil mixture. The amount of urea, on a basis of 100 mg of oil, can range from 1 g to 6 g of urea, including all values and subranges therebetween as if explicitly written out. For example, per 100 mg of oil, the amount of urea can be 1.5 g, 2.0 g, 2.5 g., 3.0 g, 3.5 g., 4.0 g, 4.5 g, 5.0 g, or 5.5 g. The resulting solution is cooled, for example to 0°C, or 5°C, or 10°C, or 15°C, or 20°C, and after a period of time elapses, for example, 1 hour, or 2 hours, or 3 hours, or 4 hours, or 5 hours, or 6 hours, or 7 hours, or 8 hours, the resulting precipitate / solution combination is centrifuged. Post centrifugation, the solvent, for example methanol, is removed, and the desired product(s) are extracted from the precipitate by using, for example, pentane, hexane, and / or heptane. More than one extraction, for example, 2, 3, 4 or 5 extractions can be performed. The resulting pentane, hexane and /or heptane extracts are concentrated to remove the pentane, hexane and /or heptane thereby yielding oils enriched in the desired product(s).

The contents of U.S. Application Nos. 12/729,013 and 61/296,456 are referred to herein in their entireties.

### Enrichment of Microbial Oil from Biomass

Another aspect of the methods and systems described herein is the ability to extract and purify microbial oils from a microbial biomass. The methods disclosed herein of extraction of microbial oils from microbial biomass comprise a customized process of mixing and heating the biomass with an alcohol and a base to produce esters of polyunsaturated fatty acids. The biomass is separated into a substantially liquid phase and a substantially solid phase. The substantially liquid phase is further purified by recovering a fraction comprising the esters of polyunsaturated fatty acids.

The systems and methods disclosed herein can start with wet biomass, reducing the drying and dewatering costs. It is known in the art to extract oil from dried algal mass by using hexane as a solvent. This process is energy intensive. The use of heat to dry and hexane to extract produces product of lower quality as this type of processing causes lipid degradation. Thus, extract and purify microbial oils from a microbial biomass provides a cost-effective alternative to the traditional methods.

Traditionally, microbial oils are purified and enriched from crude oil instead of directly from biomass. Biomass was not used as a starting material for microbial oil extraction because it was difficult to produce. It was difficult to separate algal biomass from algal fermentation broth. Algal fermentation broth is a tough emulsion in a form like thick milk. This makes it difficult to separate biomass from algal fermentation broth, if not feasible. The surfactants generated during the fermentation process are generally responsible for the emulsification. It was considered inefficient to extract oil from the biomass of fermentation broth because of the low recovery rate of oil due to emulsion. As a result, the starting material for microbial oils extraction is crude oil instead of biomass.

Surprisingly, the novel biomass separation process disclosed in the present invention results in over 90% recovery of oil. The small amount of lipids in the remaining biomass (or called biomeal or spent biomass) remain useful as feed.

The proposed biomass separation process involves breaking the emulsion by adding alcohol to algal broth to breach emulsion. In a preferred embodiment, the alcohol used in the reaction is ethanol. In another embodiment, the ethanol is 0.3-1.0 in volume of the starting algal broth. In another embodiment, the ethanol is 0.3-0.5 in volume of the starting algal broth. After mixing the alcohol with algal broth, the mixture is centrifuged to separate into a biomass phase and a supernatant phase. The biomass phase is further processed to produce enriched microbial oils. FIG. 1 provides a schematic overview of the steps involved in exemplary embodiments of methods used in the separation of biomass from algal broth, and the enrichment of microbial oil from biomass, which will be described below.

In some embodiments of the invention, a method of extracting oil from an algal biomass comprised of reacting the biomass with an alcohol and a base catalyst to produce esters of polyunsaturated fatty acids.

Alcohols suitable for use in the present invention include any lower alkyl alcohol containing from 1-6 carbon atoms (i.e., a C₁₋₆ alkyl alcohol). In certain embodiments, the alcohol is ethanol or methanol. In these embodiments, the polyunsaturated fatty acids esters produced are a methyl ester and an ethyl ester of the polyunsaturated fatty acids, respectively. In the process of the present invention, the alcohol typically comprises between 2 to 5 times of the weight of the biomass. In some embodiments, the alcohol comprises about 4 times of the weight of the biomass. In certain embodiments, the biomass and the base can be added to either pure ethanol or pure methanol. In general, the amount of alcohol used may vary with the solubility of the oil or composition containing triglycerides having polyunsaturated fatty acids residues in the alcohol.

Any base known in the art to be suitable for use as a reactant may be used in the present invention. Bases of the formula RO-M, wherein M is a metal element, and R is a hydrogen or a C₁₋₆ alkyl alcohol, are particularly suited for the present invention. Examples of suitable bases include potassium hydroxide, sodium hydroxide and sodium ethoxide. In some embodiments, the base is potassium hydroxide. In processes of the present invention, the base is typically added in an amount of between about 5 wt % equivalents of the biomass to the reaction step with the composition and the alcohol. In certain embodiments, the base is typically added in an amount of about 2.5 wt % equivalents of the biomass to the reaction step with the composition and the alcohol.

The biomass comprising triglycerides having polyunsaturated fatty acid residues, the alcohol, and the base are reacted together at a temperature and for an amount of time that allows the production of an ester between the fatty acid residues and the alcohol. Suitable reaction times and temperatures may be determined by one of skill in the art to produce an ester. Without intending to be bound by theory, fatty acids including PUFAs are believed to be cleaved from the glycerol backbone of the triglyceride and esters of each fatty acid are formed during the step of reacting. In certain embodiments, the step of reacting the composition in the presence of an alcohol and a base is performed at a temperature from about 60° C to about 120° C, from about 70° C to about 110° C, from about 75° C to about 100° C, or from about 80° C to about 90° C. In further embodiments, the step of reacting the composition in the presence of an alcohol and a base is performed at a temperature of about 75° C, 80° C, 85° C, 90° C, or 95° C. In some embodiments, the step of reacting the composition in the presence of an alcohol and a base is performed for a time from about 2 hours to about 12 hours, from about 3 hours to about 11 hours, from about 4 hours to about 10 hours, from about 5 hours to about 9 hours, or from about 6 hours to about 8 hours. In certain embodiments, the step of reacting the composition in the presence of an alcohol and a base is performed for about 5.5, 6, 6.5, 7, 7.5, 8, 8.5, 9, 9.5, or 10 hours.

In one embodiment, the step of reacting the oil composition, alcohol and base may be conducted by refluxing the components to produce the PUFA esters. In additional embodiments, the step of reacting the oil composition may be carried out at a temperature that does not result in the refluxing of the reaction components. For example, carrying out the step of reacting the oil composition under pressures greater than atmospheric pressure can increase the boiling point of the solvents present in the reaction mixture. Under such conditions, the reaction can occur at a temperature at which the solvents would boil at atmospheric pressure, but would not result in the refluxing of the reaction components. In some embodiments, the reaction is conducted at a pressure from about 5 to about 20 pounds per square inch (psi); from about 7 to about 15 psi; or from about 9 to about 12 psi. In certain embodiments, the reaction is conducted at a pressure of about 7, 8, 9, 10, 11, or 12 psi. Reactions conducted under pressure may be carried out at the reaction temperatures listed above. In some embodiments, reactions conducted under pressure may be carried out at about 70° C, 75° C, 80° C, 85° C, or 90° C.

The reaction mixture comprising PUFA esters can be further processed to obtain the PUFA esters from the mixture. In one embodiment, PUFA esters can be separated from the reaction mixture by distilling the composition to recover a fraction comprising the ester of the polyunsaturated fatty acid. In this manner, a targeted fraction of the reaction mixture including PUFA esters of interest can be separated from the reaction mixture and recovered. Details of this method can be found in earlier part of this application, in the subsection entitled "Vacuum Molecular Fractional Distillation / Enrichment." In another embodiment, the PUFA esters are subjected to a urea crystallization step. When urea crystallizes in a solution containing PUFA esters (e.g., esters of DHA) and saturated fatty acid esters formed by the transesterification of a glyceride source using the techniques discussed above, a precipitate forms that comprises the urea and at least a portion of the saturated fatty acid esters. This precipitate, however, comprises a substantially lesser traction of the PUFA esters than the initial reaction mixture. The bulk of the PUFA esters instead remain in solution and can therefore be easily separated from the precipitated saturated fatty acid esters. Details of this method can be found in earlier part of this application, in the subsection entitled "Urea Adduct Crystallization."

The microalgae species that can be used with the enrichment method described in the present invention is not limited to *Schizochytrium sp.* In some embodiments, organisms include those selected from the group consisting of golden algae (such as microorganisms of the kingdom Stramenopiles), green algae, diatoms, dinoflagellates (such as microorganisms of the order Dinophyceae including members of the genus *Crypthecodinium* such as, for example, *Crypthecodinium cohnii),* yeast, and fungi of the genera *Mucor* and *Mortierella,* including but not limited to *Mortierella alpina* and *Mortierella* sect. *schmuckeri.* Members of the microbial group Stramenopiles include microalgae and algae-like microorganisms, including the following groups of microorganisms: Hamatores, Proteromonads, Opalines, Develpayella, Diplophrys, Labrinthulids, Thraustochytrids, Biosecids, Oomycetes, Hypochytridiomycetes, Commation, Reticulosphaera, Pelagomonas, Pelagococcus, Ollicola, Aureococcus, Parmales, Diatoms, Xanthophytes, Phaeophytes (brown algae), Eustigmatophytes, Raphidophytes, Synurids, Axodines (including Rhizochromulinaales, Pedinellales, Dictyochales), Chrysomeridales, Sarcinochrysidales, Hydrurales, Hibberdiales, and Chromulinales. The Thraustochytrids include the genera Schizochytrium (species include *aggregatum, limnaceum, mangrovei, minutum, octosporum), Thraustochytrium* (species include *arudimentale, aureum,benthicola, globosum, kinnei, motivum, multirudimentale, pachydermum, proliferum,roseum, striatum), Ulkenia* (species include *amoeboidea, kerguelensis, minuta, profunda,radiate, sailens, sarkariana, schizochytrops, visurgensis, yorkensis), Aplanochytrium* (species include *haliotidis, kerguelensis, profunda, stocchinoi), Japonochytrium (species* include *marinum), Althornia* (species include *crouchii),* and *Elina* (species include *marisalba, sinorifica).* The Labrinthulids include the genera *Labyrinthula* (species include *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfi), Labyrinthomyxa* (species include *marina), Labyrinthuloides* (species include *haliotidis, yorkensis), Diplophrys* (species include *archeri), Pyrrhosorus** (species include *marinus), Sorodiplophrys** (species include *stercorea), Chlamydomyxa** (species include *labyrinthuloides, montana).* (* = there is no current general consensus on the exact taxonomic placement of these genera).

Having generally described this invention, a further understanding can be obtained by reference to the examples provided herein.

### EXAMPLE 1

### Isolation of Microorganisms

Samples were collected from intertidal habitats during low tide, including bays and estuaries along the West Coast of North America (California, Oregon, and Washington) and Hawaii. Water, sediment, living plant material, and decaying plant/animal debris were placed into sterile 50 ml tubes. Portions of each sample along with the water were spread onto solid agar plates of isolation media. Isolation media consisted of: 500 ml of artificial seawater, 500 ml of distilled water, 1 g of glucose, 1 g of glycerol, 13 g of agar, 1 g of glutamate, 0.5 g of yeast extract, 0.5 g casein hydrolysate, 1 ml of a vitamin solution (100 mg/L thiamine, 0.5 mg/L biotin, 0.5 mg B₁₂), 1 ml of a trace mineral solution (PII metals, containing per liter: 6.0 g FeCl₃6H₂O, 6.84 g H₃BO₃, 0.86 g MnCl₂4H₂O, 0.06 g ZnCl₂, 0.026 CoCl₂6H₂0, 0.052 g MSO₄H₂O, 0.002 g CuSO₄5H₂O and 0.005 g Na₂MoO₄2H₂O), and 500 mg each of penicillin G and streptomycin sulfate. The agar plates were incubated in the dark at 20-25°C. After 2-4 days the agar plates were examined under magnification, and colonies of cells were picked with a sterile toothpick and restreaked onto a fresh plate of media. Cells were repeatedly streaked onto fresh media until contaminated organisms were removed. Two of the isolated microorganisms were deposited under ATCC Accession Nos. PTA-10212 and PTA-10208.

### Taxonomic Characteristics of the Isolated Microorganism Deposited Under ATCC Accession No. PTA-10212

Cultures of the isolated microorganism deposited under ATCC Accession No. PTA-10212 ("PTA-10212") appeared as white, wet, smeared colonies without visible isolated sori.

PTA-10212 was grown on solid and liquid FFM, solid KMV, KMV slush (1%), KMV broth, and MH broth to further examine growth characteristics. PTA-10212 was observed to grow rapidly on KMV and MH. *See, e.g.,* Porter D., 1989. Phylum Labyrinthulomycota. In Margulis, L., Corliss, J.O., Melkonian, M., Chapman, D.J. (Eds.) Handbook of Protoctista, Jones and Bartlett, Boston, pp. 388-398 (KMV); Honda et al., Mycol. Res. 102:439-448 (1998) (MH); and U.S. Patent No. 5,130,242 (FFM), each of which is incorporated herein by reference in its entirety.

The following observations were made after growth of PTA-10212 over several days on solid FFM media, after 72 hours growth in KMV medias, and MH broth. Sporangia were not clumped in/on any media and were very small (5-10 µm). PTA-10212 did not demonstrate the copious tetrads characteristic of *Schizochytrium* cleavage patterns. Amoeboid cells appeared about 24 hours after transfer to fresh solid media. These amoeboid cells were gone after a few days and were not evident in liquid or slush media. Unlike *Aurantiochytrium,* described by Yokoyama, R. et al., Mycoscience 48(6): 329-341 (2007), as having the appearance of "small sandgrains on the bottom of the flask" when grown in liquid media, PTA-10212 did not settle at the bottom of the flask but was suspended in both KMV and MH liquid media. The sporangia were not as dense as typical of *Schizochytrium* or *Oligochytrium,* which also have robust ectoplasmic networks that were absent from PTA-10212. While most species undergo vegetative cleavage of small sporangia or assimilative cells by the division of a larger sporangium over several hours, PTA-10212 formed dumbbell-shaped elongated assimilative cells, which then formed a thin isthmus that pulled apart as the ends of the dumbbell separated. The resulting cells appeared to be small assimilative cells. Direct transformation of an amoeboid cell into the dumbbell shaped assimilative cell was not observed. Typical biflagellate zoospores were observed swimming but were relatively rare. PTA-10212 was non-prolific, dividing by vegetative cleavage. Direct release of zoospores was not observed, although zoospores were observed swimming. Vegetative cells were very small (2 µm to 5 µm).

PTA-10212 was further examined using the flow-through technique, in which microscopic slides were prepared by suspending a small portion of an agar-grown colony in a drop of half-strength sea water. With this technique, primary sporangia were observed to be globose and approximately 10 µm in diameter. Walls were very thin and remnants were not observed when binary division of the protoplast was initiated. Repeated binary division produced 8-16 smaller (4-5 µm in diameter) secondary sporangia. The secondary sporangia remained quiescent for several hours before again releasing an amorphous protoplast. The amorphous protoplast divided by pinching and pulling, initially producing typical dumbbell-shaped intermediate stages and finally resulting in 4-8 small globose bodies 2.5-2.8 µm in diameter. The latter rested for several minutes up to 1-2 hours, then changed shape (elongated) and turned into biflagellate zoospores, 2.3-2.5 x 3.7-3.9 µm. Zoospores were abundant and could be precisely measured when they came to rest. Zoospores then rounded off and started a new cycle of development. The zoospores were larger than *Sicyoidochytrium minutum* and smaller than *Ulkenia visurgensis.*

PTA-10212 was further characterized based on the similarity of its 18s rRNA gene to that of known species. Genomic DNA was prepared from PTA-10212 by standard procedures. *See,* for example, Sambrook J. and Russell D. 2001. Molecular cloning: A laboratory manual, 3rd edition. Cold Spring Harbor Laboratory Press, Cold Spring Harbor, New York. Briefly: (1) 500 µL of cells were centrifuged from mid-log culture. The cells were re-centrifuged, and all traces of liquid were removed from the cell pellet with a small-bore tip; (2) pellets were resuspended with 200 µL lysis buffer (20 mM Tris pH 8.0, 125 µg/mL Proteinase K, 50 mM NaCl, 10 mM EDTA pH 8.0, 0.5% SDS); (3) cells were lysed at 50°C for 1 hour; (4) the lysis mixture was pipetted into phase-lock gel (PLG-Eppendorf) 2 mL tubes; (5) equal volume of P:C:I was added and allowed to mix for 1.5 hours; (6) the tubes were centrifuged at 12,000 x g for 5 minutes; (7) the aqueous phase was removed from above the gel within the PLG tube and an equal volume of chloroform was added to the aqueous phase, and mixed for 30 minutes; (8) the tubes were centrifuged at 14,000 x g for approximately 5 minutes; (9) the top layer (aqueous phase) was pipetted away from the chloroform, and placed in a new tube; (10) 0.1 volume of 3 M NaOAC was added and mixed (inverted several times); (11) 2 volumes of 100% EtOH were added and mixed (inverted several times) with genomic DNA precipitant forming at this stage; (12) the tubes were centrifuged at 4°C in a microcentrifuge at 14,000 x g for approximately 15 minutes; (13) the liquid was gently poured off with genomic DNA remaining at the bottom of the tube; (14) the pellet was washed with 0.5 mL 70% EtOH; (15) the tubes were centrifuged at 4°C in a microcentrifuge at 14,000 x g for approximately 5 minutes; (16) the EtOH was gently poured off and the genomic DNA pellet was dried; and (17) a suitable volume of H₂O and RNase was added directly to the genomic DNA pellet. The PCR amplification of the 18s rRNA gene was carried out with primers previously described (Honda et. al., J. Euk. Micro. 46(6): 637-647 (1999). The PCR conditions with chromosomal DNA template were as follows: 0.2 µM dNTPs, 0.1 µM each primer, 8% DMSO, 200 ng chromosomal DNA, 2.5 U Herculase® II Fusion DNA Polymerase (Stratagene), and Herculase® buffer (Stratagene) in a 50 µL total volume. The PCR Protocol included the following steps: (1) 95°C for 2 minutes; (2) 95°C for 35 seconds; (3) 55°C for 35 seconds; (4) 72°C for 1 minute and 30 seconds; (5) repeat steps 2-4 for 30 cycles; (6) 72°C for 5 minutes; and (7) hold at 4°C.

PCR amplification yielded a distinct DNA product with the expected size using chromosomal template described above. The PCR product was cloned into the vector pJET1.2/blunt (Fermentas) according to the manufacturer's instructions, and the insert sequence was determined using supplied standard primers.

Phylogenetic analysis places PTA-10212 within the lineage that includes *Thraustochytrium pachydermum* and *Thraustochytrium aggregatum* with moderate support. The sporangia of *T. pachydermum* have very thick cell walls. *T. aggregatum* forms clearly visible clumps of sori that are opaque. PTA-10212 shows neither of these characteristics. The presence of many amoeboid cells has been described in other taxa, such as *Ulkenia, T. gaertnerium, A. limiacinum,* and *S. mangrovei;* however, the descriptions associated with those taxa differ from the observed characteristics of the isolate. Moreover, PTA-10212 did not show phylogenetic affinity towards any of these taxa.

Table 3 shows a comparison of the 18s rRNA sequence from the microorganism deposited under ATCC Accession No. PTA-10212 to DNA sequences in the National Center for Biotechnology Information (NCBI) electronic database. The percent identity was determined using two different calculations. "Calculation #1" takes into consideration any "gaps" that occur in the sequences, either from non-homologous regions or partial sequence (AlignX-VectorNTI default settings). "Calculation #2" does not include calculated penalties for gaps (AlignX-VectorNTI "IDENTITY" matrix setting).

**Table 3: Comparison of 18s rRNA Sequences**

| **Thraustochytrids** | **% Identity Calculation #1** | **% Identity Calculation #2** |
|---|---|---|
| *Thraustochytrium pachydermum* | 85% | 93% |
| *Thraustochytrium aggregatum* (p) | 83% | 92% |
| *Thraustochytrium gaertnerium* | 82% | 92% |
| *Ulkenia visurgensis* | 82% | 92% |
| *Schizochytrium sp. PTA-9695* | 80% | 92% |
| *Schizochytrium mangrovei* | 80% | 91% |
| *Schizochytrium sp. ATCC 20888* | 80% | 90% |
| *Aurantiochytrium limiacinum* | 79% | 90% |

| | | |
|---|---|---|
| (p): indicates partial sequence | | |

As shown in Table 3, it was found that, in terms of % identity, the 18s rRNA gene sequence (SEQ ID NO:1) from the microorganism deposited under ATCC Accession No. PTA-10212 is related, though not identical, to 18s rRNA gene sequences available in the NCBI database. It is generally recognized that organisms can have closely related 18s rRNA gene sequences while belonging to a different genus or species.

Based on the above characterization, the isolated microorganism deposited under ATCC Accession No. PTA-10212 is believed to represent a new *Thraustochytrium* species and is therefore also designated as *Thraustochytrium sp.* ATCC PTA-10212.

### Taxonomic Characteristics of the Isolated Microorganism Deposited Under ATCC Accession No. PTA-10208

The microorganism deposited under ATCC Accession No. PTA-10208 ("PTA-10208") was identified as a sub-isolate (an individual cell isolated from a culture and maintained as a new separate and distinct culture) of the microorganism deposited under ATCC Accession No. PTA-9695 ("PTA-9695"), described in U.S. Appl. No. 12/407,687 and PCT/US2009/001720, each of which is incorporated herein by reference in its entirety.

PTA-10208 shares taxonomic characteristics with PTA-9695. PTA-9695 was found to have biflagellate zoospores at discharge that swim actively away from the mature sporangium, wall remnants of which were clearly visible (in phase contrast) after spore release. PTA-9695 sporangia measured 12.5 µm to 25 µm in diameter, and zoospores were 2.5 µm to 2.8 µm x 4.5 µm to 4.8 µm in size. There were 8 to 24 spores per individual PTA-9695 sporangium. Settled PTA-9695 zoospores enlarged and rapidly underwent binary divisions leading to tetrads, octads, and finally to clusters of sporangia. Tetrad formation commenced at a very early stage prior to maturity of the sporangia. These characteristics are in agreement with the genus *Schizochytrium.* In terms of % identity, the PTA-9695 18s rRNA gene sequence (SEQ ID NO:2), which is shared by PTA-10208, was found to be closely related, though not identical, to the 18s rRNA gene sequence of *T. aggregatum* provided in Honda, D, et al., J. Euk. Micro. 46(6): 637-647 (1999). The 18s rRNA sequence published for *Thraustochytrium aggregatum* is a partial sequence, with an approximately 71 DNA nucleotide gap in the middle of the sequence. PTA-9695 is believed to represent a new *Schizochytrium* species. As such, the sub-isolate PTA-10208 is also designated as *Schizochytrium sp.* ATCC PTA-10208.

### EXAMPLE 2

### Growth Characteristics of the Isolated Microorganism Deposited Under ATCC Accession No. PTA-10212

The isolated microorganism deposited under ATCC Accession No. PTA-10212 was examined for growth characteristics in individual fermentation runs, as described below. Typical media and cultivation conditions are shown in Table 1.

In carbon (glycerol) and nitrogen-fed cultures with 1000 ppm Cl⁻ at 22.5°C with 20% dissolved oxygen at pH 7.3, PTA-10212 produced a dry cell weight of 26.2 g/L after 138 hours of culture in a 10 L fermentor volume. The lipid yield was 7.9 g/L; the omega-3 yield was 5.3 g/L; the EPA yield was 3.3 g/L; and the DHA yield was 1.8 g/L. The fatty acid content was 30.3% by weight; the EPA content was 41.4% of fatty acid methyl esters (FAME); and the DHA content was 26.2% of FAME. The lipid productivity was 1.38 g/L/day, and the omega-3 productivity was 0.92 g/L/day under these conditions, with 0.57 g/L/day EPA productivity and 0.31 g/L/day DHA productivity.

In carbon (glycerol) and nitrogen-fed cultures with 1000 ppm Cl⁻ at 22.5°C with 20% dissolved oxygen at pH 7.3, PTA-10212 produced a dry cell weight of 38.4 g/L after 189 hours of culture in a 10 L fermentor volume. The lipid yield was 18 g/L; the omega-3 yield was 12 g/L; the EPA yield was 5 g/L; and the DHA yield was 6.8 g/L. The fatty acid content was 45% by weight; the EPA content was 27.8% of FAME; and the DHA content was 37.9% of FAME. The lipid productivity was 2.3 g/L/day, and the omega-3 productivity was 1.5 g/L/day under these conditions, with 0.63 g/L/day EPA productivity and 0.86 g/L/day DHA productivity.

In carbon (glycerol) and nitrogen-fed cultures with 1000 ppm Cl⁻ at 22.5°C with 20% dissolved oxygen at pH 6.8-7.7, PTA-10212 produced a dry cell weight of 13 g/L after 189 hours of culture in a 10 L fermentor volume. The lipid yield was 5.6 g/L; the omega-3 yield was 3.5 g/L; the EPA yield was 1.55 g/L; and the DHA yield was 1.9 g/L. The fatty acid content was 38% by weight; the EPA content was 29.5% of FAME; and the DHA content was 36% of FAME. The lipid productivity was 0.67 g/L/day, and the omega-3 productivity was 0.4 g/L/day under these conditions, with 0.20 g/L/day EPA productivity and 0.24 g/L/day DHA productivity.

In carbon (glycerol) and nitrogen-fed cultures with 1000 ppm Cl⁻ at 22.5-28.5°C with 20% dissolved oxygen at pH 6.6-7.2, PTA-10212 produced a dry cell weight of 36.7 g/L - 48.7 g/L after 191 hours of culture in a 10 L fermentor volume. The lipid yield was 15.2 g/L - 25.3 g/L; the omega-3 yield was 9.3 g/L - 13.8 g/L; the EPA yield was 2.5 g/L - 3.3 g/L; and the DHA yield was 5.8 g/L - 11 g/L. The fatty acid content was 42.4% - 53% by weight; the EPA content was 9.8% - 22% of FAME; and the DHA content was 38.1% - 43.6% of FAME. The lipid productivity was 1.9 g/L/day - 3.2 g/L/day, and the omega-3 productivity was 1.2 g/L/day - 1.7 g/L/day under these conditions, with 0.31 g/L/day - 0.41 g/L/day EPA productivity and 0.72 g/L/day - 1.4 g/L/day DHA productivity.

### Growth Characteristics of the Isolated Microorganism Deposited Under ATCC Accession No. PTA-10208

The isolated microorganism deposited under ATCC Accession No. PTA-10208 was examined for growth characteristics in individual fermentation runs, as described below. Typical media and cultivation conditions are shown in Table 2.

In carbon (glucose) and nitrogen-fed cultures with 1000 ppm Cl⁻ at 22.5°C at pH 7.0 with 20% dissolved oxygen during the nitrogen feed and 10% dissolved oxygen thereafter, PTA-10208 produced a dry cell weight of 95 g/L after 200 hours of culture in a 10 L fermentor volume. The lipid yield was 53.7 g/L; the omega-3 yield was 37 g/L; the EPA yield was 14.3 g/L; and the DHA yield was 21 g/L. The fatty acid content was 57% by weight; the EPA content was 27.7% of FAME; and the DHA content was 39.1% of FAME. The lipid productivity was 6.4 g/L/day, and the omega-3 productivity was 4.4 g/L/day under these conditions, with 1.7 g/L/day EPA productivity and 2.5 g/L/day DHA productivity.

In carbon (glucose) and nitrogen-fed cultures with 1000 ppm Cl⁻ at 22.5°C at pH 7.5 with 20% dissolved oxygen during the nitrogen feed and 10% dissolved oxygen thereafter, PTA-10208 produced a dry cell weight of 56 g/L after 139 hours of culture in a 10 L fermentor volume. The lipid yield was 53 g/L; the omega-3 yield was 34 g/L; the EPA yield was 11.5 g/L; and the DHA yield was 22 g/L. The fatty acid content was 58% by weight; the EPA content was 21.7% of FAME; and the DHA content was 41.7% of FAME. The lipid productivity was 9.2 g/L/day, and the omega-3 productivity was 5.9 g/L/day under these conditions, with 2 g/L/day EPA productivity and 3.8 g/L/day DHA productivity.

In carbon (glucose) and nitrogen-fed cultures with 1000 ppm Cl⁻ at 22.5°C at pH 7.0 with 20% dissolved oxygen during the nitrogen feed and 10% dissolved oxygen thereafter, PTA-10208 produced a dry cell weight of 93.8 g/L after 167 hours of culture in a 2000 L fermentor volume. The lipid yield was 47.2 g/L; the omega-3 yield was 33.1 g/L; the EPA yield was 10.5 g/L; and the DHA yield was 20.4 g/L. The fatty acid content was 50.6% by weight; the EPA content was 23% of FAME; and the DHA content was 42.6% of FAME. The lipid productivity was 6.8 g/L/day, and the omega-3 productivity was 4.7 g/L/day under these conditions, with 1.5 g/L/day EPA productivity and 2.9 g/L/day DHA productivity.

In carbon (glucose) and nitrogen-fed cultures with 1000 ppm Cl⁻ at 22.5°C at pH 7.0 with 20% dissolved oxygen during the nitrogen feed and 10% dissolved oxygen thereafter, PTA-10208 produced a dry cell weight of 105 g/L after 168 hours of culture in a 2000 L fermentor volume. The lipid yield was 46.4g/L; the omega-3 yield was 33 g/L; the EPA yield was 10.7 g/L; and the DHA yield was 20.3 g/L. The fatty acid content was 43.9% by weight; the EPA content was 24% of FAME; and the DHA content was 43.7% of FAME. The lipid productivity was 6.6 g/L/day, and the omega-3 productivity was 4.7 g/L/day under these conditions, with 1.5 g/L/day EPA productivity and 2.9 g/L/day DHA productivity.

In carbon (glucose) and nitrogen-fed cultures with 1000 ppm Cl⁻ at 22.5°C at pH 7.0 with 20% dissolved oxygen during the nitrogen feed and 10% dissolved oxygen thereafter, PTA-10208 produced a dry cell weight of 64.8 g/L after 168 hours of culture in a 2000 L fermentor volume. The lipid yield was 38.7 g/L; the omega-3 yield was 29.9 g/L; the EPA yield was 8.5 g/L; and the DHA yield was 16.7 g/L. The fatty acid content was 59.6% by weight; the EPA content was 23% of FAME; and the DHA content was 42.3% of FAME. The lipid productivity was 5.53 g/L/day, and the omega-3 productivity was 3.8 g/L/day under these conditions, with 1.2 g/L/day EPA productivity and 2.3 g/L/day DHA productivity.

### EXAMPLE 3

### Fatty Acid Profiles of Microorganism Strains ATCC PTA-10208 and PTA-10212

Four samples of biomass (PTA-10208 Sample #1; PTA-10208 Sample #2; PTA-10212 Sample #1; and PTA-10212 Sample #2) were analyzed for total crude oil content by solvent extraction, lipid classes were determined by high performance liquid chromatography/evaporative light scattering detection (HPLC/ELSD), triacylglycerol (TAG) were analyzed by HPLC/mass spectrometry (HPLC/MS), and fatty acid (FA) profiles were determined by gas chromatography with flame ionization detection (GC-FID). The crude lipid content of each freeze dried biomass was determined using solvent grinding with hexane and compared to the sum of FAME (mg/g) generated by direct transesterification, and the resultant fatty acid methyl esters (FAME) were quantified by GC/FID analysis. Fatty acids in the extracted crude lipid were also quantified by transesterification and quantified using GC/FID analysis of the resultant FAME. The weight percent of all neutral lipids (NL) and free fatty acids (FFA) were determined in the extracted crude lipid using normal phase HPLC with ELSD and atmospheric pressure chemical ionization-MS (APCI-MS) identification. The method separates and quantifies sterol esters (SE), TAG, free fatty acids (FFA), 1,3-diacylglycerols (1,3-DAG), sterols, 1,2-diacylglycerols (1,2-DAG), and monoacylglycerols (MAG). Results are shown in Tables 4 and 5, below.

TAG and phospholipids (PL) were isolated from the crude oils extracted from the four samples of biomass (PTA-10208 Sample #1; PTA-10208 Sample #2; PTA-10212 Sample #1; and PTA-10212 Sample #2). TAG was isolated using low pressure flash chromatography and PL was isolated using solid phase extraction (SPE). The identity of each isolated fraction was confirmed by thin layer chromatography (TLC). The fatty acid profiles of the isolated TAG and PL fractions were determined following direct transesterification using GC-FID as FAME. Results are shown in Tables 6 and 7, below.

The total crude oil content and fatty acid profiles of isolated lipid classes were also determined for two additional samples of biomass from microorganism strain ATCC PTA-10212 (PTA-10212 Sample #3 and PTA-10212 Sample #4). Crude oil was obtained from each sample by hexane extraction, and individual lipid classes were isolated using low pressure flash chromatography. The fatty acid profiles of the biomass, crude oil, and isolated fractions were determined using GC-FID as FAME. Results are shown in Tables 8-11, below.

Individual lipid classes were isolated from a sample of crude oil from microorganism strain ATCC PTA-10212 (PTA-10212 Sample #5) previously extracted using the FRIOLEX® process, and the fatty acid profiles of each class were determined using GC-FID as FAME. Results are shown in Tables 12 and 13, below.

Individual lipid classes were isolated from a sample of crude oil from microorganism strain ATCC PTA-10208 (PTA-10208 Sample #3) using normal HPLC with ELSD and APCI-MS identification.

### Experimental Procedures

*Crude Oil Extraction* - Crude oil was extracted from samples of freeze-dried biomass using solvent grinding. For example, approximately 3 grams of biomass was weighed into a Swedish tube. Three ball bearings and 30 mL of hexane were added to the Swedish tube, which was sealed with a neoprene stopper and placed in a shaker for 2 hours. The resultant slurry was filtered using a Buchner funnel and Whatman filter paper. The filtered liquid was collected, the solvent removed under vacuum, and the amount of remaining crude lipid determined gravimetrically.

*Fatty Acid Analysis* - The samples of biomass, extracted crude lipid, and isolated lipid classes were analyzed for fatty acid composition as FAME. Briefly, freeze-dried biomass and isolated lipid classes were weighed directly into a screw cap test tubes, while samples of the crude oil were dissolved in hexane to give a concentration of approximately 2 mg/mL. Toluene, containing internal standard, and 1.5 N HCl in methanol was added to each tube. The tubes were vortexed, then capped and heated to 100° C for 2 hours. The tubes were allowed to cool, and saturated NaCl in water was added. The tubes were vortexed again and centrifuged to allow the layers to separate. A portion of the organic layer was then placed in a GC vial and analyzed by GC-FID. FAME was quantified using a 3-point calibration curve generated using Nu-Check-Prep GLC Reference Standard (NuCheck, Elysian, MN). Fatty acids present in the extract were expressed as mg/g and as a weight percent. Fat content in the samples was estimated assuming equal response to the internal standard when analyzed by GC-FID.

### HPLC/ELSD/MS Method-

| | | | |
|---|---|---|---|
| Instrument | Agilent 11 MSD 00 HPLC , Alltech 3300 ELSD, Agilent 1100 | | |
| Column | Phenomenex Luna Silica, 250 x 4.6 mm, | | |
| | 5 µm particle size w/ Guard Column | | |
| Mobile Phase | A - 99.5% Hexanes (Omnisolv) | | |
| | 0.4% Isopropyl alcohol (Omnisolv) | | |
| | 0.1% Acetic Acid | | |
| | B - 99.9% Ethanol (Omnisolv, 95:5 Ethanol:IPA) | | |
| | 0.1% Acetic Acid | | |
| Gradient | | | |
| | Time, min. | % A | % B |
| | 0 | 100 | 0 |
| | 5 | 100 | 0 |
| | 15 | 85 | 10 |
| | 20 | 0 | 100 |
| | 25 | 0 | 100 |
| | 26 | 100 | 0 |
| | 35 | 100 | 0 |
| Column Temp. | 30°C | | |
| Flow Rate | 1.5 mL/min | | |
| Injection Volume | 5 µL | | |
| ELSD Detection | Temperature 35°C, Gas flow 1.2 L/min | | |
| MSD | Mass Range 200 - 1200, Fragmentor 225 V | | |
| | Drying Gas Temperature 350°C | | |
| | Vaporizer Temperature 325°C | | |
| | Capillary Voltage 3500 V | | |
| | Corona Current 10 µA | | |

*Solid Phase Extraction* -PL fractions were separated from the crude lipid by solid phase extraction (SPE) using 2 g aminopropyl cartridges (Biotage, Uppsala, Sweden) placed in a Vac Elut apparatus (Varian Inc, Palo Alto, USA). The cartridge was conditioned with 15 mL of hexane, and ∼60 mg of each sample was dissolved in 1 mL CHCl₃ and applied to the cartridge. The column was washed with 15 mL of 2:1 CHCl₃:isopropyl alcohol to elute all the neutral lipids, which was discarded. The fatty acids were then eluted with 15 mL of 2% acetic acid (HOAc) in ether, which was discarded. The PL portion was eluted with 15 mL of 6:1 Methanol:Chloroform, which was collected, dried under nitrogen, and weighed.

*Flash Chromatography* - Flash chromatography was used to separate the lipid classes present in the crude oil. Approximately 200 mg of crude oil dissolved in hexane was injected onto the head of the column. The chromatography system utilized Silica Gel 60 (EMD Chemical, Gibbstown, NJ) with mobile phase composed of Petroleum Ether and Ethyl Acetate at 5 mL/min (Tables 6-7) or 3 mL/min (Tables 8-13). A step gradient was used to selectively elute each lipid class from the column. The mobile phase gradient started from 100% petroleum ether and finished with 50% ethyl acetate. Fractions were collected in 10 mL test tubes using a Gilson FC 204 large-bed fraction collector (Gilson, Inc., Middleton, WI). Each tube was analyzed by thin layer chromatography (TLC) and the tubes containing individual lipid classes (as judged by single spots on TLC plate with expected retention factor (Rf)) were pooled, concentrated to dryness, and weighed. The total fraction content was then determined gravimetrically.

*TLC Analysis* - Thin layer chromatography was conducted on silica gel plates. The plates were eluted using a solvent system consisting of petroleum ether : ethyl ether : acetic acid (80:20:1) and were visualized using iodine vapor. The Rf values of each spot were then compared with reported literature values for each lipid class.

*Analysis of TAG and PL fractions* - The isolated TAG and PL fractions were analyzed for fatty acid composition as fatty acid methyl esters (FAME). The TAG fractions were dissolved in hexane to give a concentration of approximately 1-2 mg/mL. 1 mL aliquots of the solutions were concentrated to dryness under nitrogen. Toluene, containing internal standard, and 1.5 N HCl in methanol was added to each tube. The tubes were vortexed, then capped and heated to 100° C for 2 hours. Internal standard and HCl methanol were added directly to the tubes containing the PL fraction and heated. The tubes were allowed to cool, and saturated NaCl in water was added. The tubes were vortexed again and centrifuged to allow the layers to separate. A portion of the organic layer was then placed in a GC vial and analyzed by GC-FID. FAMEs were quantified using a 3-point calibration curve generated using Nu-Check-Prep GLC 502B Reference Standard (NuCheck, Elysian, MN). Fatty acids present in the extract were expressed as mg/g and as a % of FAME.

### Results

### PTA-10208 Sample #1

The fatty acid profile of the biomass and extracted crude lipid for PTA-10208 Sample #1 was determined using GC/FID. Fatty acids in the biomass were transesterified *in situ* by weighing 28.6 mg of biomass directly into a FAME tube, while a sample of the extracted crude lipid was prepared by weighing 55.0 mg of crude lipid into a 50 mL volumetric flask and transferring 1 ml to a separate FAME tube. The estimated crude lipid content of the biomass was determined to be 53.2% (as SUM of FAME) using GC with FID detection, while 52.0% (wt/wt) lipid was extracted from the dry biomass, giving a 97.8% recovery of total lipid. The crude lipid was determined to be 91.9% fatty acids (as SUM of FAME) using GC/FID. The major fatty acids contained in the crude lipid were C16:0 (182.5 mg/g), C20:5 n-3 (186.8 mg/g), and C22:6 n-3 (423.1 mg/g).

The lipid class profile of the extracted crude lipid was determined by weighing 55.0 mg of crude lipid into a 50 mL volumetric flask and transferring an aliquot into an HPLC vial for HPLC/ELSD/MS analysis. According to the HPLC/ELSD/MS analysis, the crude lipid contained 0.2% sterol esters (SE), 95.1% TAG, 0.4% sterols, and 0.5% 1,2-diacylglycerol (DAG). 5% of the TAG fraction included a peak that eluted directly after the TAG peak, but did not give a recognizable mass spectrum.

Isolated TAG from this sample as determined by flash chromatography made up approximately 92.4% of the crude oil. PL was not detected by weight or TLC after SPE isolation. The major fatty acids (>50 mg/g) contained in the TAG were C16:0 (189 mg/g), C20:5 n-3 (197 mg/g), and C22:6 n-3 (441 mg/g).

### PTA-10208 Sample #2

The fatty acid profile of the biomass and extracted crude lipid for PTA-10208 Sample #2 was determined using GC/FID. Fatty acids in the biomass were transesterified *in situ* by weighing 32.0 mg of biomass directly into a FAME tube, while a sample of the extracted crude lipid was prepared by weighing 60.1 mg of crude lipid into a 50 mL volumetric flask and transferring 1 ml to a separate FAME tube. The estimated crude lipid content of the biomass was determined to be 52.4% (as SUM of FAME) using GC with FID detection, while 48.0% (wt/wt) lipid was extracted from the dry biomass, giving a 91.7% recovery of total lipid. The crude lipid was determined to be 95.3% fatty acids (as SUM of FAME) using GC/FID. The major fatty acids contained in the crude lipid were C16:0 (217.5 mg/g), C20:5 n-3 (169.3 mg/g), and C22:6 n-3 (444.1 mg/g).

The lipid class profile of the extracted crude lipid was determined by weighing 60.1 mg of crude lipid into a 50 mL volumetric flask and transferring an aliquot into an HPLC vial for HPLC/ELSD/MS analysis. According to the HPLC/ELSD/MS analysis, the crude lipid contained 0.2% SE, 95.7% TAG, 0.3% sterols, and 0.7% 1,2-DAG. 5.1% of the TAG fraction included a peak that eluted directly after the TAG peak, but did not give a recognizable mass spectrum.

Isolated TAG from this sample made up approximately 93.9% of the crude oil. PL was not detected by weight or TLC after SPE isolation. The major fatty acids (>50mg/g) contained in the TAG were C16:0 (218 mg/g), C20:5 n-3 (167 mg/g) and C22:6 n-3 (430 mg/g).

### PTA-10208 Sample #3

A sample of crude oil from the microorganism deposited under ATCC Accession No. PTA-10208 (Sample PTA-10208 #3) was analyzed using HPLC/ELSD/MS. A total of 98.38% of lipids were recovered, with the sterol ester (SE) fraction accounting for 0.32%, the TAG fraction accounting for 96.13%, the 1,3-diacylglycerol (DAG) fraction accounting for 0.22%, the 1,2-DAG fraction accounting for 0.78%, and the sterol fraction accounting for 0.93%.

### PTA-10212 Sample #1

The fatty acid profile of the biomass and extracted crude lipid for PTA-10212 Sample #1 was determined using GC/FID. Fatty acids in the biomass were transesterified *in situ* by weighing 27.0 mg of biomass directly into a FAME tube, while a sample of the extracted crude lipid was prepared by weighing 52.5 mg of crude lipid into a 50 mL volumetric flask and transferring 1 ml to a separate FAME tube. The estimated crude lipid content of the biomass was determined to be 38.3% (as SUM of FAME) using GC with FID detection, while 36.3% (wt/wt) lipid was extracted from the dry biomass, giving a 94.6% recovery of total lipid. The crude lipid was determined to be 83.2% fatty acids (as SUM of FAME) using GC/FID. The major fatty acids contained in the crude lipid were C16:0 (328.5 mg/g), C20:5 n-3 (90.08 mg/g), and C22:6 n-3 (289.3 mg/g).

The lipid class profile of the extracted crude lipid was determined by weighing 52.5 mg of crude lipid into a 50 mL volumetric flask and transferring an aliquot into an HPLC vial for HPLC/ELSD/MS analysis. According to the HPLC/ELSD/MS analysis, the crude lipid contained 0.2% SE, 64.2% TAG, 1.9% FFA, 2.8% 1,3-DAG, 1.4% sterols, 18.8% 1,2-DAG, and 0.5% MAG. 3.4% of the TAG fraction included a peak that eluted directly after the TAG peak, but did not give a recognizable mass spectrum.

Isolated TAG from this sample made up approximately 49.8% of the crude oil. Isolated PL made up approximately 8.1% of the crude oil. The major fatty acids (>50mg/g) contained in the TAG fraction are C16:0 (400 mg/g), C20:5 n-3 (91 mg/g), and C22:6 n-3 (273 mg/g). The major fatty acids (>50mg/g) contained in the PL fraction are C16:0 (98 mg/g), C20:5 n-3 (33 mg/g), and C22:6 n-3 (227 mg/g).

### PTA-10212 Sample #2

The fatty acid profile of the biomass and extracted crude lipid PTA-10212 Sample #2 was determined using GC/FID. Fatty acids in the biomass were transesterified *in situ* by weighing 29.5 mg of biomass directly into a FAME tube, while a sample of the extracted crude lipid was prepared by weighing 56.5 mg of crude lipid into a 50 mL volumetric flask and transferring 1 ml to a separate FAME tube. The estimated crude lipid content of the biomass was determined to be 40.0% (as SUM of FAME) using GC with FID detection, while 41.3% (wt/wt) lipid was extracted from the dry biomass, giving a 106.1% recovery of total lipid. The crude lipid was determined to be 82.8% fatty acids (as SUM of FAME) using GC/FID. The major fatty acids contained in the crude lipid were C16:0 (327.3 mg/g), C20:5 n-3 (92.5 mg/g), and C22:6 n-3 (277.6 mg/g).

The lipid class profile of the extracted crude lipid was determined by weighing 56.5 mg of crude lipid into a 50 mL volumetric flask and transferring an aliquot into an HPLC vial for HPLC/ELSD/MS analysis. According to the HPLC/ELSD/MS analysis, the crude lipid contained 0.2% SE, 58.2% TAG, 2.3% FFA, 3.4% 1,3-DAG, 1.7% sterols, 23.4% 1,2-DAG, and 0.6% MAG. 3.3% of the TAG fraction included a peak that eluted directly after the TAG peak, but did not give a recognizable mass spectrum.

Isolated TAG from this sample made up approximately 51.9% of the crude oil. Isolated PL made up approximately 8.8% of the crude oil. The major fatty acids (>50mg/g) contained in the TAG fraction are C16:0 (402 mg/g), C20:5 n-3 (92 mg/g), and C22:6 n-3 (245 mg/g). The major fatty acids (>50mg/g) contained in the PL fraction are C16:0 (121 mg/g), C20:5 n-3 (48 mg/g), and C22:6 n-3 (246 mg/g).

**Table 4: Fatty Acid Profiles of PTA-10208 and PTA-10212 Biomasses and Extracted Crude Lipids (mg/g)**

| | PTA-10208 Sample #1 | PTA-10208 Sample #1 | PTA-10208 Sample #2 | PTA-10208 Sample #2 | PTA-10212 Sample #1 | PTA-10212 Sample #1 | PTA-10212 Sample #2 | PTA-10212 Sample #2 |
|---|---|---|---|---|---|---|---|---|
| | Biomass | Crude Lipid | Biomass | Crude Lipid | Biomass | Crude Lipid | Biomass | Crude Lipid |
| Fatty Acid | FAME (mg/g) | FAME (mg/g) | FAME (mg/g) | FAME (mg/g) | FAME (mg/g) | FAME (mg/g) | FAME (mg/g) | FAME (mg/g) |
| C12:0 | 1.47 | 2.43 | 1.80 | 3.14 | 0.99 | 1.90 | 0.87 | 1.91 |
| C14:0 | 11.62 | 20.12 | 16.72 | 31.03 | 5.51 | 12.91 | 5.97 | 13.69 |
| C14:1 | 0.00 | 0.00 | 0.00 | 0.00 | 0.00 | 0.00 | 0.00 | 0.00 |
| C15:0 | 2.43 | 3.75 | 3.60 | 6.22 | 9.13 | 20.42 | 9.39 | 20.81 |
| C16:0 | 105.04 | 182.47 | 117.72 | 217.49 | 145.87 | 328.45 | 147.87 | 327.27 |
| C16:1 | 0.00 | 0.00 | 0.06 | 0.01 | 6.26 | 14.53 | 7.46 | 16.89 |
| C18:0 | 5.37 | 8.96 | 4.77 | 8.37 | 6.77 | 15.39 | 6.77 | 15.15 |
| C18:1 n-9 | 0.00 | 3.26 | 0.00 | 3.09 | 0.03 | 4.04 | 0.08 | 5.87 |
| C18:1 n-7 | 0.00 | 0.00 | 0.00 | 0.00 | 0.00 | 0.00 | 0.00 | 0.00 |
| C18:2 n-6 | 0.00 | 0.00 | 0.00 | 0.00 | 0.00 | 0.00 | 0.00 | 0.00 |
| C20:0 | 1.48 | 1.79 | 1.40 | 1.85 | 1.60 | 3.09 | 1.67 | 3.20 |
| C18:3 n-3 | 0.00 | 0.00 | 0.00 | 0.00 | 0.00 | 0.00 | 0.00 | 0.00 |
| C20:1 n-9 | 0.00 | 0.00 | 0.00 | 0.00 | 0.00 | 0.00 | 0.00 | 0.00 |
| C18:4 n-3 | 0.91 | 1.61 | 1.10 | 2.00 | 2.28 | 2.56 | 2.21 | 2.64 |
| C20:2 n-6 | 0.00 | 0.00 | 0.00 | 0.00 | 0.00 | 0.00 | 0.00 | 0.00 |
| C20:3 n-6 | 0.00 | 0.00 | 0.00 | 0.00 | 0.00 | 0.00 | 0.00 | 0.00 |
| C22:0 | 0.10 | 0.00 | 0.08 | 0.00 | 0.30 | 0.12 | 0.35 | 0.24 |
| C20:4 n-7 | 0.81 | 0.45 | 0.67 | 0.41 | 0.00 | 0.00 | 0.00 | 0.00 |
| C20:4 n-6 | 7.22 | 12.23 | 6.84 | 12.18 | 1.19 | 2.26 | 1.31 | 2.32 |
| C22:1 n-9 | 0.00 | 0.00 | 0.00 | 0.00 | 0.00 | 0.00 | 0.00 | 0.00 |
| C20:4 n-5 | 0.63 | 0.52 | 0.00 | 0.46 | 0.00 | 0.00 | 0.00 | 0.00 |
| C20:4 n-3 | 3.45 | 5.45 | 3.33 | 5.58 | 0.00 | 2.40 | 0.00 | 2.34 |
| C20:3 n-3 | 0.09 | 0.00 | 0.11 | 0.00 | 0.00 | 0.00 | 0.00 | 0.00 |
| C20:5 n-3 | 107.31 | 186.83 | 92.99 | 169.32 | 40.32 | 90.08 | 43.15 | 92.54 |
| C22:4 n-9 | 0.00 | 0.00 | 0.00 | 0.00 | 0.00 | 0.00 | 0.00 | 0.00 |
| C24:0 | 0.60 | 0.00 | 0.52 | 0.00 | 2.81 | 6.83 | 2.74 | 6.53 |
| C24:1 n-9 | 1.55 | 3.26 | 0.85 | 2.04 | 0.43 | 1.34 | 0.42 | 1.24 |
| C22:5 n-6 | 9.66 | 15.84 | 10.27 | 17.98 | 2.42 | 4.68 | 2.32 | 4.21 |
| C22:5 n-3 | 20.44 | 35.13 | 9.92 | 17.50 | 2.41 | 4.94 | 2.69 | 5.23 |
| C22:6 n-3 | 246.98 | 423.10 | 245.96 | 444.08 | 139.58 | 289.34 | 137.35 | 277.57 |
| Sum of FAME | 527.15 | 907.18 | 518.71 | 942.75 | 367.89 | 805.29 | 372.63 | 799.68 |

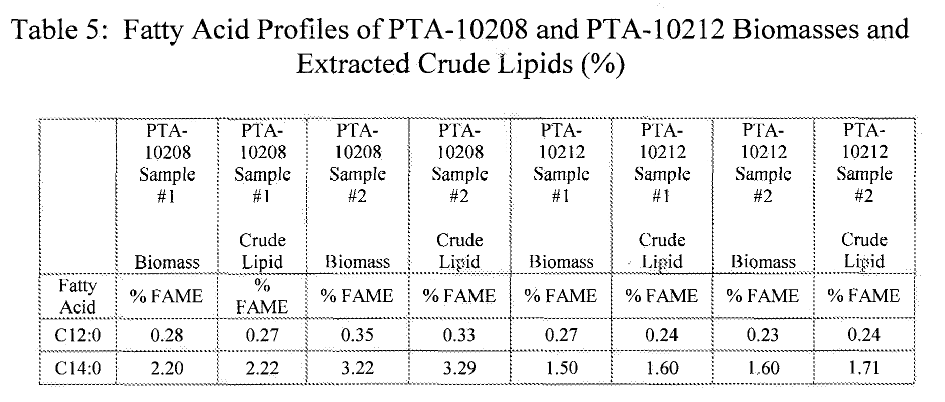

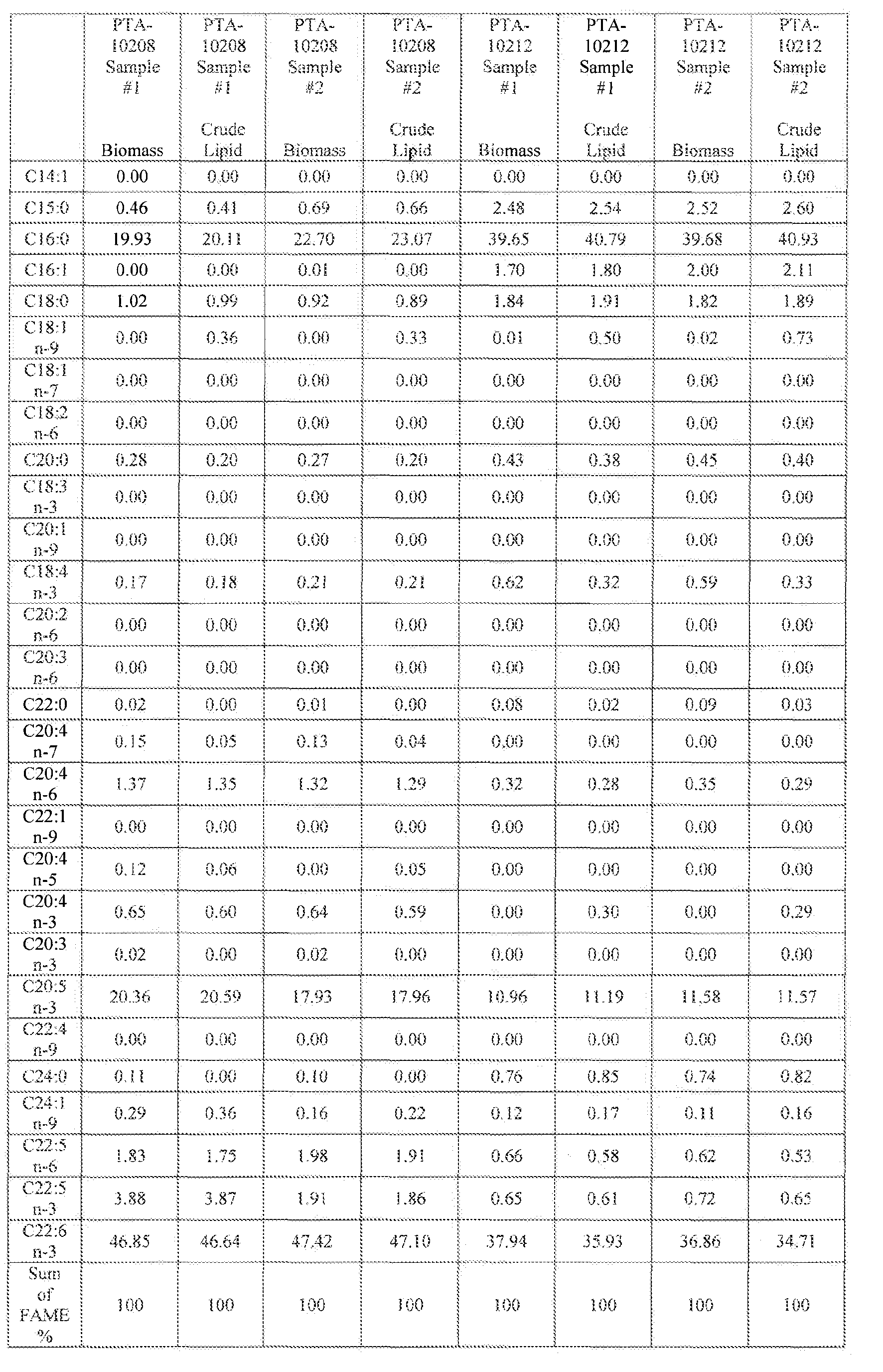

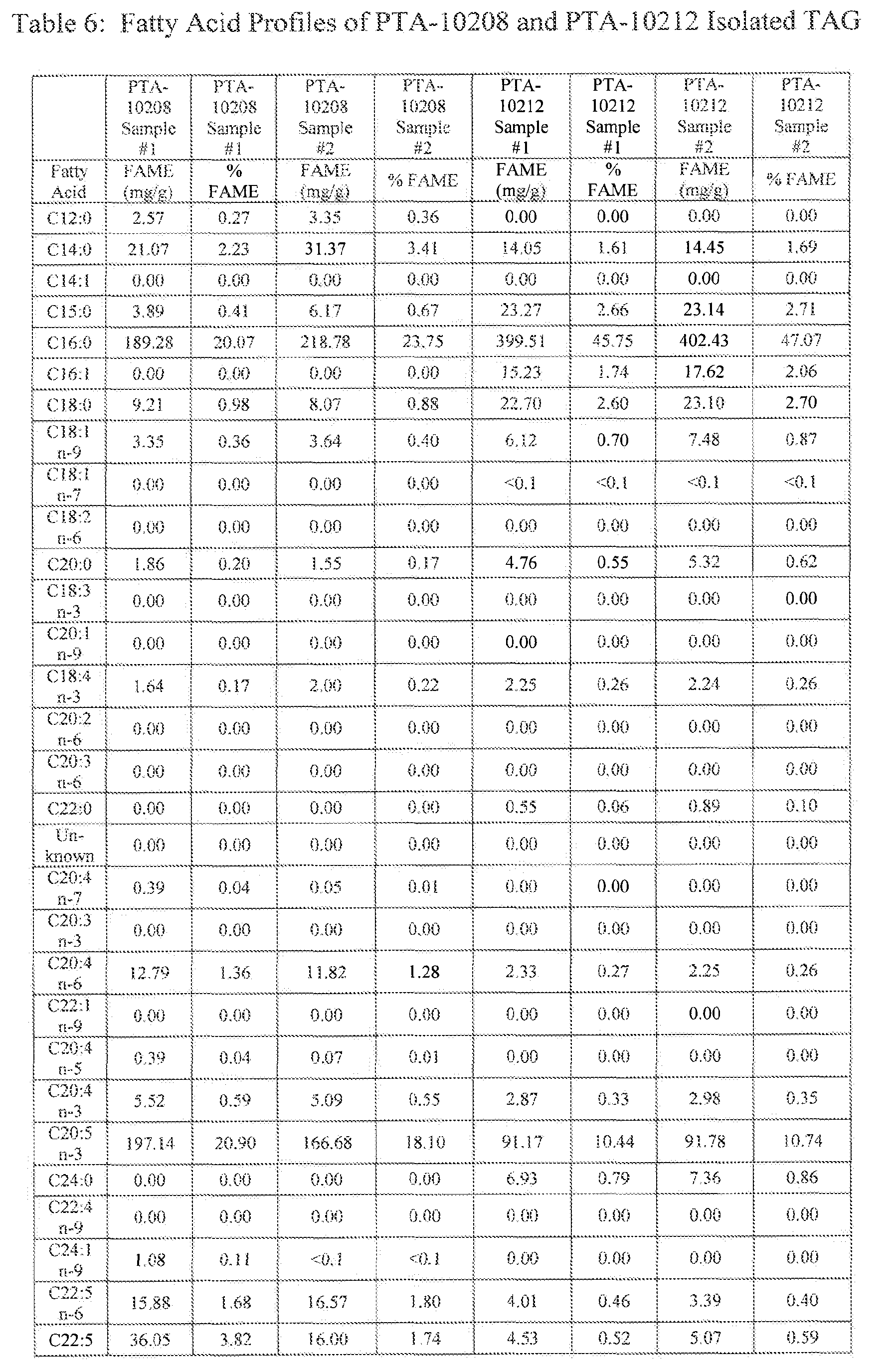

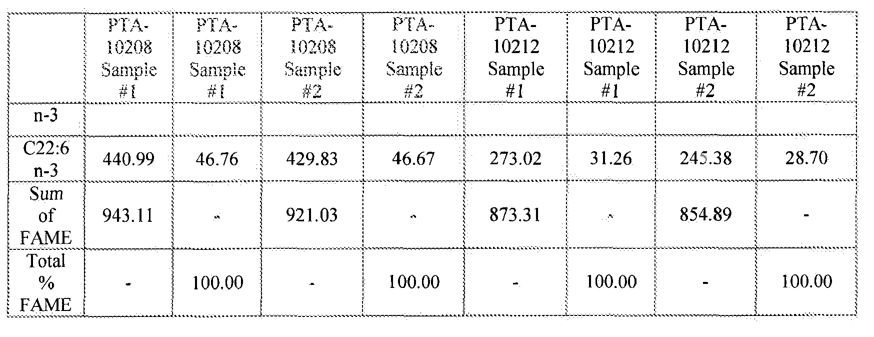

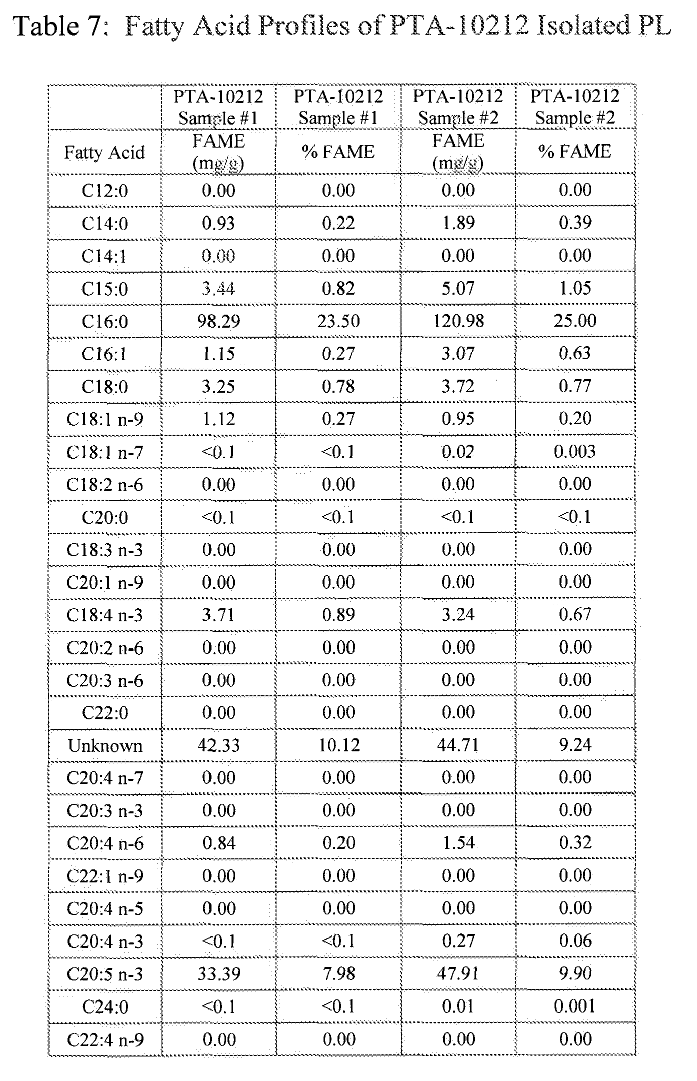

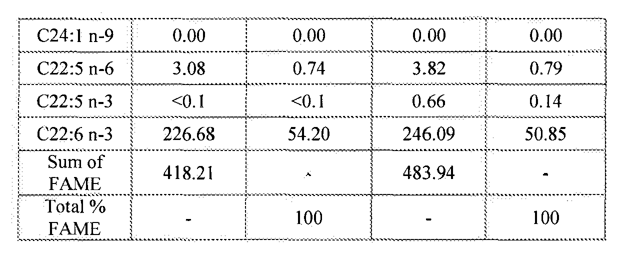

### PTA-10212 Sample #3

The lipid content of the biomass for PTA-10212 Sample #3 was estimated to be 34% as the sum of FAME, and the amount of crude oil obtained after solvent extraction was 37% by weight, giving a 109% recovery of fat present in the biomass. After fractionation using flash chromatography, approximately 46% of the crude oil was isolated as TAG, 28% was isolated as DAG, The crude oil contained 309 mg/g DHA and 264 mg/g EPA. The isolated TAG contained 341 mg/g DHA and 274 mg/g EPA. The isolated DAG fraction contained 262 mg/g DHA and 237 mg/g EPA. The total fatty acid profiles of the biomass, extracted crude oil, and isolated fractions are shown below in Table 8 and Table 9 calculated as mg/g and % FAME, respectively.

**Table 8: Fatty Acid Profiles of PTA-10212 Sample #3 Biomass and Extracted Crude Lipid (mg/g)**

| | Biomass | Crude Oil | TAG | DAG |
|---|---|---|---|---|
| Wt. % | NA | 37.2% | 46.0% | 27.9% |
| Fatty Acid | FAME (mg/g) | FAME (mg/g) | FAME (mg/g) | FAME (mg/g) |
| C12:0 | 0.0 | 0.0 | 0.0 | 0.0 |
| C14:0 | 3.6 | 10.3 | 11.5 | 9.4 |
| C14:1 | 0.0 | 0.0 | 0.0 | 0.0 |
| C15:0 | 4.1 | 10.6 | 9.8 | 6.6 |
| C16:0 | 70.5 | 181.8 | 231.7 | 111.3 |
| C16:1 | 6.7 | 19.1 | 18.7 | 17.1 |
| C18:0 | 2.4 | 10.2 | 14.2 | 0.0 |
| C18:1 n-9 | 0.0 | 6.7 | 0.0 | 0.0 |
| C18:1 n-7 | 0.0 | 1.2 | 0.0 | 0.0 |
| C18:2 n-6 | 0.0 | 1.8 | 0.0 | 0.0 |
| C20:0 | 0.0 | 2.4 | 0.0 | 0.0 |
| C18:3 n-3 | 0.0 | 0.0 | 0.0 | 0.0 |
| C20:1 n-9 | 0.0 | 0.3 | 0.0 | 1.7 |
| C18:4 n-3 | 1.9 | 3.4 | 3.2 | 4.4 |
| C20:2 n-6 | 0.0 | 0.0 | 0.0 | 0.0 |
| C20:3 n-6 | 0.0 | 0.0 | 0.0 | 0.0 |
| C22:0 | 3.3 | 0.0 | 0.0 | 0.0 |
| C20:4 n-7 | 0.0 | 2.1 | 1.5 | 0.0 |
| C20:3 n-3 | 0.0 | 0.0 | 0.0 | 0.0 |
| C20:4 n-6 | 6.8 | 17.9 | 21.4 | 13.8 |
| C22:1 n-9 | 0.0 | 0.0 | 0.0 | 0.0 |
| C20:4 n-5 | 0.0 | 1.3 | 1.3 | 0.0 |
| C20:4 n-3 | 3.0 | 8.5 | 10.9 | 6.4 |
| C20:5 n-3 | 102.0 | 263.6 | 274.2 | 237.4 |
| C24:0 | 0.0 | 1.7 | 3.9 | 0.0 |
| C22:4 n-9 | 0.0 | 0.0 | 0.0 | 0.0 |
| C24:1 n-9 | 0.0 | 0.0 | 4.2 | 0.0 |
| C22:5 n-6 | 3.2 | 8.3 | 10.7 | 6.1 |
| C22:5 n-3 | 3.8 | 10.4 | 15.1 | 6.6 |
| C22:6 n-3 | 131.2 | 309.4 | 341.1 | 261.9 |
| Sum of FAME | 342.4 | 871.1 | 973.2 | 682.6 |

**Table 9: Fatty Acid Profiles of PTA-10212 Sample #3 Biomass and Extracted Crude Lipid (%)**

| | Biomass | Crude Oil | TAG | DAG |
|---|---|---|---|---|
| Wt. % | NA | 37.2% | 46.0% | 27.9% |
| Fatty Acid | FAME (mg/g) | FAME (mg/g) | FAME (mg/g) | FAME (mg/g) |
| C12:0 | 0.0 | 0.0 | 0.0 | 0.0 |
| C14:0 | 1.1 | 1.2 | 1.2 | 1.4 |
| C14:1 | 0.0 | 0.0 | 0.0 | 0.0 |
| C15:0 | 1.2 | 1.2 | 1.0 | 1.0 |
| C16:0 | 20.6 | 20.9 | 23.8 | 16.3 |
| C16:1 | 2.0 | 2.2 | 1.9 | 2.5 |
| C18:0 | 0.7 | 1.2 | 1.5 | 0.0 |
| C18:1 n-9 | 0.0 | 0.8 | 0.0 | 0.0 |
| C18:1 n-7 | 0.0 | 0.1 | 0.0 | 0.0 |
| C18:2 n-6 | 0.0 | 0.2 | 0.0 | 0.0 |
| C20:0 | 0.0 | 0.3 | 0.0 | 0.0 |
| C18:3 n-3 | 0.0 | 0.0 | 0.0 | 0.0 |
| C20:1 n-9 | 0.0 | 0.0 | 0.0 | 0.2 |
| C18:4 n-3 | 0.6 | 0.4 | 0.3 | 0.6 |
| C20:2 n-6 | 0.0 | 0.0 | 0.0 | 0.0 |
| C20:3 n-6 | 0.0 | 0.0 | 0.0 | 0.0 |
| C22:0 | 1.0 | 0.0 | 0.0 | 0.0 |
| C20:4 n-7 | 0.0 | 0.2 | 0.2 | 0.0 |
| C20:3 n-3 | 0.0 | 0.0 | 0.0 | 0.0 |
| C20:4 n-6 | 2.0 | 2.1 | 2.2 | 2.0 |
| C22:1 n-9 | 0.0 | 0.0 | 0.0 | 0.0 |
| C20:4 n-5 | 0.0 | 0.1 | 0.1 | 0.0 |
| C20:4 n-3 | 0.9 | 1.0 | 1.1 | 0.9 |
| C20:5 n-3 | 29.8 | 30.3 | 28.2 | 34.8 |
| C24:0 | 0.0 | 0.2 | 0.4 | 0.0 |
| C22:4 n-9 | 0.0 | 0.0 | 0.0 | 0.0 |
| C24:1 n-9 | 0.0 | 0.0 | 0.4 | 0.0 |
| C22:5 n-6 | 0.9 | 1.0 | 1.1 | 0.9 |
| C22:5 n-3 | 1.1 | 1.2 | 1.6 | 1.0 |
| C22:6 n-3 | 38.3 | 35.5 | 35.1 | 38.4 |
| Total % FAME | 100.0 | 100.0 | 100.0 | 100.0 |

### PTA-10212 Sample #4

PTA-10212 Sample #4 contained approximately 23% lipid determined as the sum of FAME, of which 107% was recovered using hexane extraction. After fractionation using flash chromatography, approximately 42% of the crude oil was isolated as TAG, 18% was isolated as DAG. The crude oil contained 275 mg/g DHA and 209 mg/g EPA. The isolated TAG contained 296 mg/g DHA and 205 mg/g EPA. The isolated DAG fraction contained 245 mg/g DHA and 219 mg/g EPA. The total fatty acid profiles of the biomass, extracted crude oil, and isolated fractions are shown below in Table 10 (mg/g) and Table 11 (%FAME).

**Table 10: Fatty Acid Profiles of PTA-10212 Sample #4 Biomass and Extracted Crude Lipid (mg/g)**

| | Biomass | Crude Oil | TAG | DAG |
|---|---|---|---|---|
| Wt. % | NA | 24.7% | 42.2% | 18.4% |
| Fatty Acid | FAME (mg/g) | FAME (mg/g) | FAME (mg/g) | FAME (mg/g) |
| C12:0 | 0.0 | 0.0 | 2.1 | 2.4 |
| C14:0 | 2.0 | 8.3 | 9.8 | 9.6 |
| C14:1 | 0.0 | 0.0 | 0.0 | 0.0 |
| C15:0 | 4.8 | 16.8 | 0.4 | 0.9 |
| C16:0 | 63.3 | 210.6 | 285.7 | 138.0 |
| C16:1 | 1.6 | 6.7 | 7.4 | 7.5 |
| C18:0 | 2.8 | 12.2 | 19.9 | 4.6 |
| C18:1 n-9 | 0.0 | 3.7 | 0.7 | 1.1 |
| C18:1 n-7 | 0.0 | 0.0 | 0.3 | 1.2 |
| C18:2 n-6 | 0.0 | 0.0 | 0.0 | 0.0 |
| C20:0 | 0.0 | 3.3 | 6.0 | 1.5 |
| C18:3 n-3 | 0.0 | 0.0 | 0.0 | 0.0 |
| C20:1 n-9 | 0.0 | 0.0 | 0.7 | 1.2 |
| C18:4 n-3 | 1.4 | 3.8 | 3.6 | 5.0 |
| C20:2 n-6 | 0.0 | 0.0 | 0.0 | 0.0 |
| C20:3 n-6 | 0.0 | 0.0 | 0.4 | 0.0 |
| C22:0 | 1.5 | 0.0 | 1.9 | 0.0 |
| C20:4 n-7 | 0.0 | 0.0 | 0.9 | 0.6 |
| C20:3 n-3 | 0.0 | 0.0 | 0.0 | 0.0 |
| C20:4 n-6 | 2.5 | 10.1 | 13.0 | 10.3 |
| C22:1 n-9 | 0.0 | 0.0 | 0.0 | 0.0 |
| C20:4 n-5 | 0.0 | 0.0 | 0.8 | 0.7 |
| C20:4 n-3 | 1.4 | 6.3 | 8.6 | 6.0 |
| C20:5 n-3 | 57.6 | 209.1 | 205.4 | 219.0 |
| C24:0 | 0.0 | 2.6 | 0.8 | 0.0 |
| C22:4 n-9 | 0.1 | 0.0 | 0.0 | 0.0 |
| C24:1 n-9 | 0.0 | 0.0 | 1.1 | 0.5 |
| C22:5 n-6 | 1.4 | 6.1 | 7.9 | 4.5 |
| C22:5 n-3 | 4.0 | 15.8 | 20.8 | 12.9 |
| C22:6 n-3 | 87.7 | 275.0 | 296.4 | 244.8 |
| Sum of FAME | 232.2 | 790.1 | 894.8 | 672.4 |

**Table 11: Fatty Acid Profiles of PTA-10212 Sample #4 Biomass and Extracted Crude Lipid (%)**

| | Biomass | Crude Oil | TAG DAG | |
|---|---|---|---|---|
| Wt. % | NA | 24.7% | 42.2% | 18.4% |
| Fatty Acid | FAME (mg/g) | FAME (mg/g) | FAME (mg/g) | FAME (mg/g) |
| C12:0 | 0.0 | 0.0 | 0.2 | 0.4 |
| C14:0 | 0.9 | 1.0 | 1.1 | 1.4 |
| C14:1 | 0.0 | 0.0 | 0.0 | 0.0 |
| C15:0 | 2.1 | 2.1 | 0.0 | 0.1 |
| C16:0 | 27.3 | 26.7 | 31.9 | 20.5 |
| C16:1 | 0.7 | 0.8 | 0.8 | 1.1 |
| C18:0 | 1.2 | 1.5 | 2.2 | 0.7 |
| C18:1 n-9 | 0.0 | 0.5 | 0.1 | 0.2 |
| C18:1 n-7 | 0.0 | 0.0 | 0.0 | 0.2 |
| C18:2 n-6 | 0.0 | 0.0 | 0.0 | 0.0 |
| C20:0 | 0.0 | 0.4 | 0.7 | 0.2 |
| C18:3 n-3 | 0.0 | 0.0 | 0.0 | 0.0 |
| C20:1 n-9 | 0.0 | 0.0 | 0.1 | 0.2 |
| C18:4 n-3 | 0.6 | 0.5 | 0.4 | 0.7 |
| C20:2 n-6 | 0.0 | 0.0 | 0.0 | 0.0 |
| C20:3 n-6 | 0.0 | 0.0 | 0.0 | 0.0 |
| C22:0 | 0.6 | 0.0 | 0.2 | 0.0 |
| C20:4 n-7 | 0.0 | 0.0 | 0.1 | 0.1 |
| C20:3 n-3 | 0.0 | 0.0 | 0.0 | 0.0 |
| C20:4 n-6 | 1.1 | 1.3 | 1.5 | 1.5 |
| C22:1 n-9 | 0.0 | 0.0 | 0.0 | 0.0 |
| C20:4 n-5 | 0.0 | 0.0 | 0.1 | 0.1 |
| C20:4 n-3 | 0.6 | 0.8 | 1.0 | 0.9 |
| C20:5 n-3 | 24.8 | 26.5 | 23.0 | 32.6 |
| C24:0 | 0.0 | 0.3 | 0.1 | 0.0 |
| C22:4 n-9 | 0.0 | 0.0 | 0.0 | 0.0 |
| C24:1 n-9 | 0.0 | 0.0 | 0.1 | 0.1 |
| C22:5 n-6 | 0.6 | 0.8 | 0.9 | 0.7 |
| C22:5 n-3 | 1.7 | 2.0 | 2.3 | 1.9 |
| C22:6 n-3 | 37.8 | 34.8 | 33.1 | 36.4 |
| Total % FAME | 100.0 | 100.0 | 100.0 | 100.0 |

### PTA-10212 Sample #5

A sample of crude oil was extracted from a biomass of PTA-10212 using the FRIOLEX® process (GEA Westfalia Separator UK Ltd., Milton Keynes, England) to yield microbial oil PTA-10212 Sample #5. Individual lipid classes were isolated from PTA-10212 Sample #5 using low pressure flash chromatography, and the weight percent of each class was determined. The fatty acid profile of each class was determined using GC-FID.

Briefly, the sample was prepared by dissolving 240 mg of crude oil in 600 µL of hexane and applying to the head of the column. After fractionation of the sample using flash chromatography, the combined weights of all the fractions was 240 mg giving a 100% recovery. The sterol ester fraction accounted for 0.9%, the TAG fraction accounted for 42.6%, the free fatty acid (FFA) fraction accounted for 1.3%, the sterol fraction accounted for 2.2%, the DAG fraction accounted for 41.6%. The total fatty acid profiles of the FRIOLEX® crude oil and isolated fractions are shown below in Table 12 and Table 13 calculated as mg/g and % FAME, respectively.

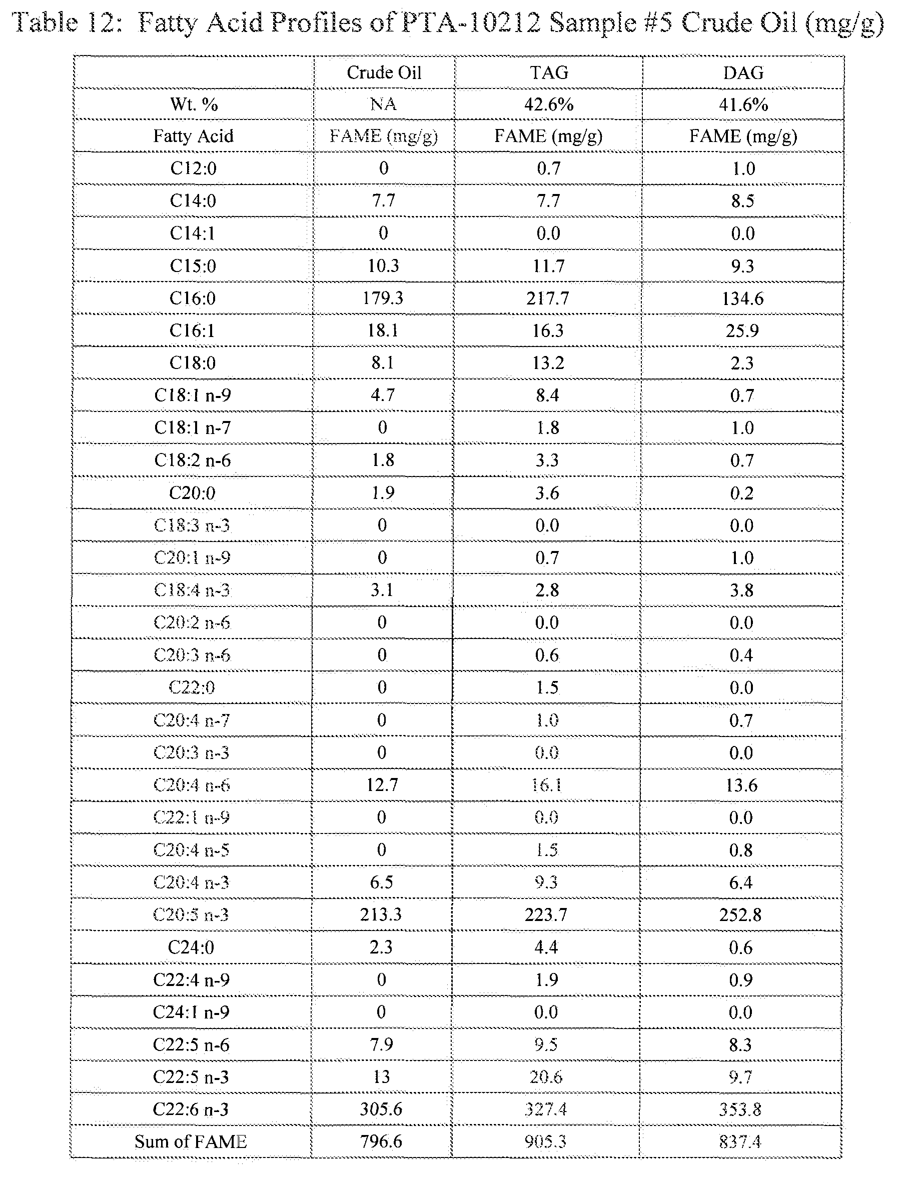

**Table 13: Fatty Acid Profiles of PTA-10212 Sample #5 Crude Oil (%)**

| | Crude Oil | TAG | DAG |
|---|---|---|---|
| Fatty Acid | % FAME | % FAME | % FAME |
| C12:0 | 0 | 0.1 | 0.1 |
| C14:0 | 1 | 0.9 | 1.0 |
| C14:1 | 0 | 0.0 | 0.0 |
| C15:0 | 1.3 | 1.3 | 1.1 |
| C16:0 | 22.5 | 24.0 | 16.1 |
| C16:1 | 2.3 | 1.8 | 3.1 |
| C18:0 | 1 | 1.5 | 0.3 |
| C18:1 n-9 | 0.6 | 0.9 | 0.1 |
| C18:1 n-7 | 0 | 0.2 | 0.1 |
| C18:2 n-6 | 0.2 | 0.4 | 0.1 |
| C20:0 | 0.2 | 0.4 | 0.0 |
| C18:3 n-3 | 0 | 0.0 | 0.0 |
| C20:1 n-9 | 0 | 0.1 | 0.1 |
| C18:4 n-3 | 0.4 | 0.3 | 0.5 |
| C20:2 n-6 | 0 | 0.0 | 0.0 |
| C20:3 n-6 | 0 | 0.1 | 0.0 |
| C22:0 | 0 | 0.2 | 0.0 |
| C20:4 n-7 | 0 | 0.1 | 0.1 |
| C20:3 n-3 | 0 | 0.0 | 0.0 |
| C20:4 n-6 | 1.6 | 1.8 | 1.6 |
| C22:1 n-9 | 0 | 0.0 | 0.0 |
| C20:4 n-5 | 0 | 0.2 | 0.1 |
| C20:4 n-3 | 0.8 | 1.0 | 0.8 |
| C20:5 n-3 | 26.8 | 24.7 | 30.2 |
| C24:0 | 0.3 | 0.5 | 0.1 |
| C22:4 n-9 | 0 | 0.2 | 0.1 |
| C24:1 n-9 | 0 | 0.0 | 0.0 |
| C22:5 n-6 | 1 | 1.1 | 1.0 |
| C22:5 n-3 | 1.6 | 2.3 | 1.2 |
| C22:6 n-3 | 38.4 | 36.2 | 42.3 |
| Total % FAME | 100 | 100 | 100 |

### EXAMPLE 4

The relative amount and fatty acid composition of each TAG isomer present in the extracted crude lipid was determined for each of samples PTA-10208 Sample #1, PTA-10208 Sample #2, PTA-10212 Sample #1, PTA-10212 Sample #2, and PTA-10212 Sample #3, PTA-10212 Sample #4, and PTA-10212 Sample #5 from Example 3 using non-aqueous reversed phase HPLC separation and APCI-MS detection.

### TAG Method -

| | | | |
|---|---|---|---|
| Instrument | Agilent 1100 HPLC | | |
| | Agilent 1100 MSD | | |
| Column(s) | Two Phenomenex Luna C18 (2), 150 x 4.6 mm, 3 µm particle size connected in series | | |
| Mobile Phase | A - Acetonitrile | | |
| Gradient | B - IPA w/ 0.1% Ammonium Acetate | | |
| | Time, min. | % A | % B |
| | 0 | 80 | 20 |
| | 120 | 20 | 80 |
| | 125 | 20 | 80 |
| | 126 | 80 | 20 |
| | 140 | 80 | 20 |
| Column Temp. | 20°C | | |
| Flow Rate | 0.5 mL/min | | |
| Injection Volume | 5 µL | | |
| MSD | Mass Range 350 - 1150 | | |
| | Fragmentor 225 V | | |
| | Drying Gas Temperature 350°C, | | |
| | Vaporizor Temperature 325°C | | |
| | Capillary Voltage 3500 V | | |
| | Corona Current 10 µA | | |

### PTA-10208 Sample #1

The crude lipid isolated from PTA-10208 Sample #1 was prepared for TAG analysis prepared for TAG analysis by weighing 5.5 mg of oil into an HPLC vial and diluting with 1 mL of hexane.

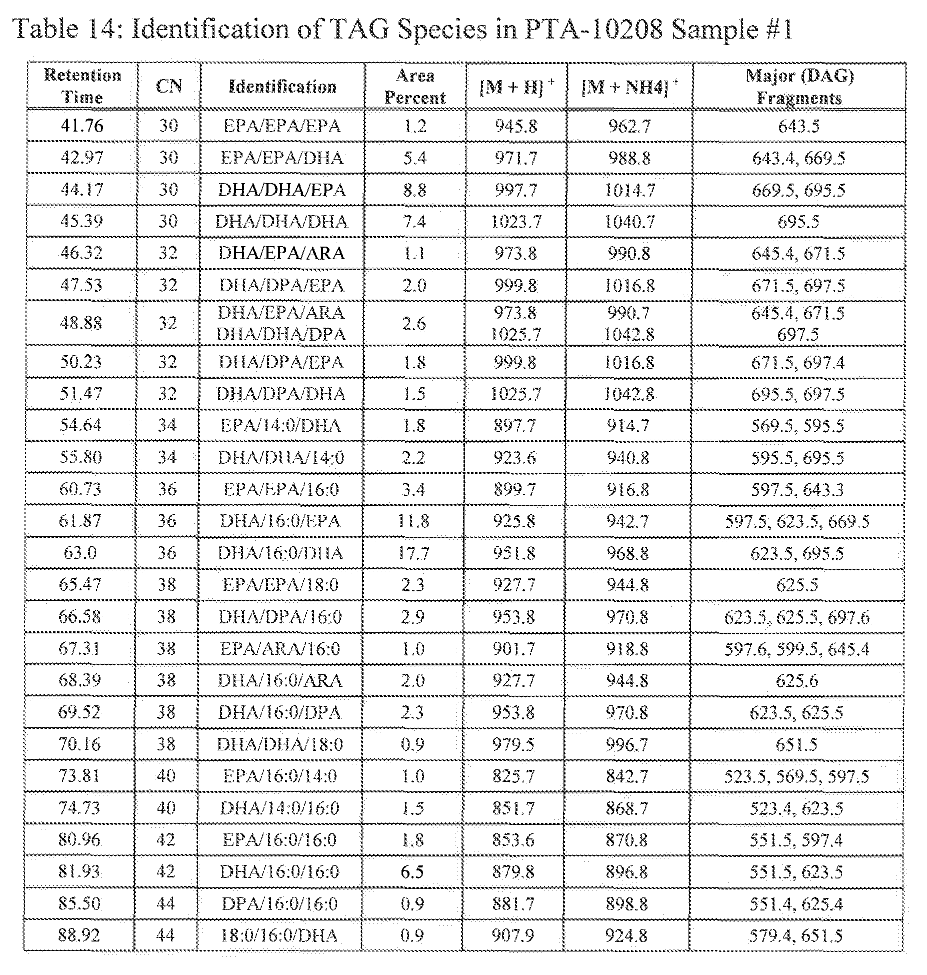

### PTA-10208 Sample #2

The crude lipid isolated from PTA-10208 Sample #2 was prepared for TAG analysis prepared for TAG analysis by weighing 5.3 mg of oil into an HPLC vial and diluting with 1 mL of hexane.

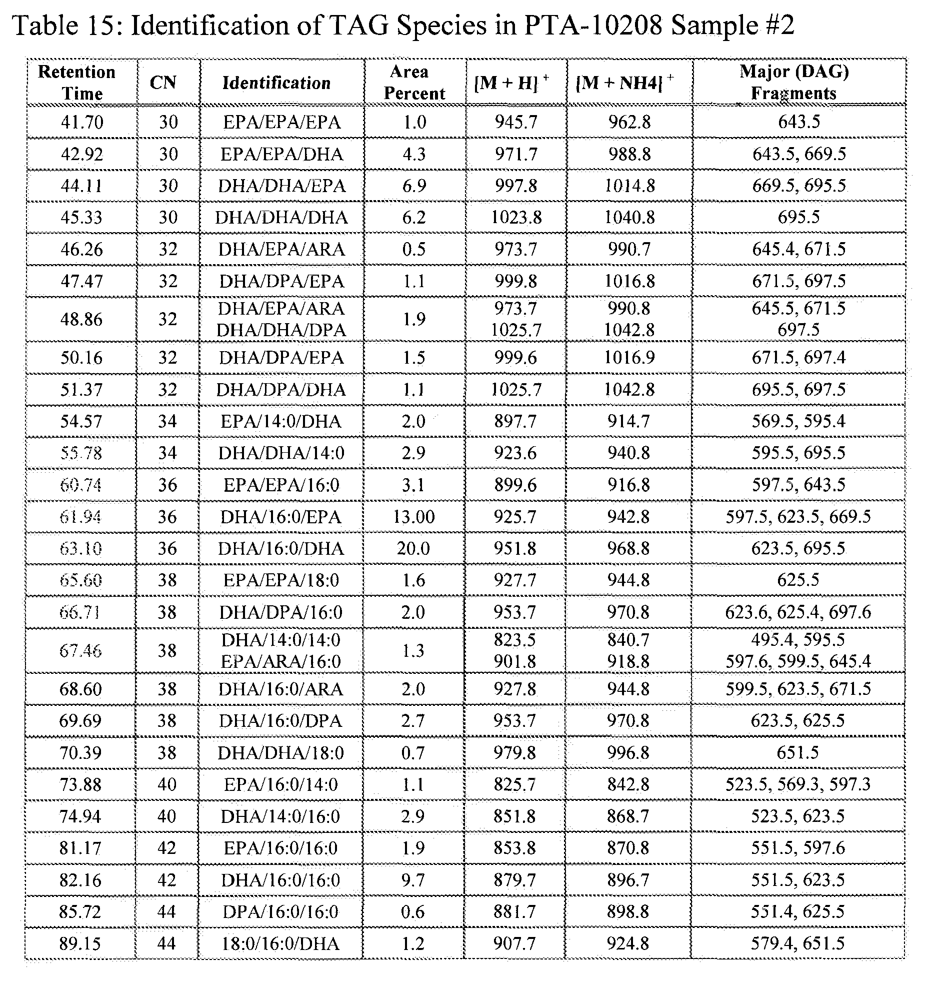

### PTA-10212 Sample #1

The crude lipid isolated from PTA-10212 Sample #1 was prepared for TAG analysis prepared for TAG analysis by weighing 5.3 mg of oil into an HPLC vial and diluting with 1 mL of hexane.

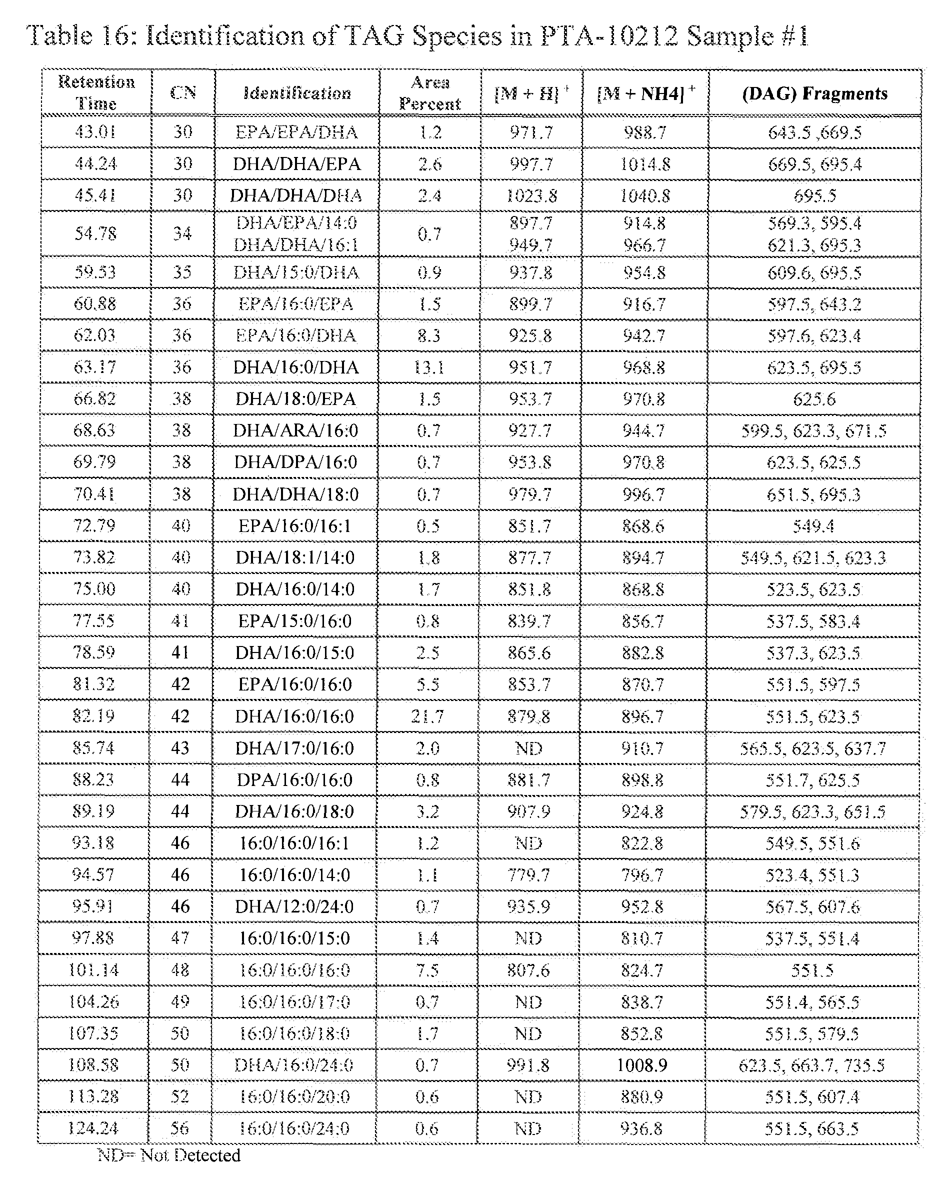

### PTA-10212 Sample #2

The crude lipid isolated from PTA-10212 Sample #2 was prepared for TAG analysis prepared for TAG analysis by weighing 3.6 mg of oil into an HPLC vial and diluting with 1 mL of hexane.

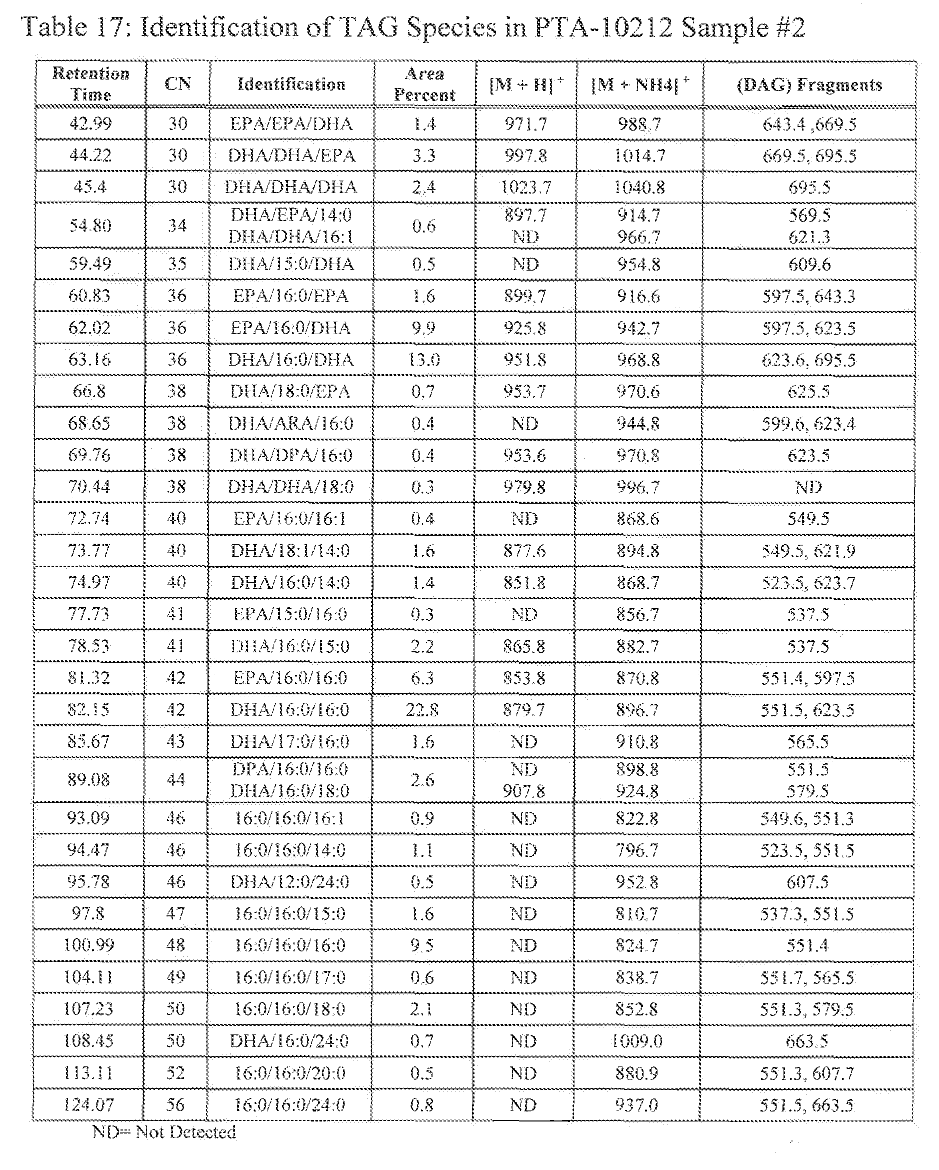

### PTA-10212 Sample #3

A sample of the TAG fraction of PTA-10212 Sample #3 was prepared in hexane and analyzed by HPLC/APCI/MS to determine the identities of individual TAG isomers.

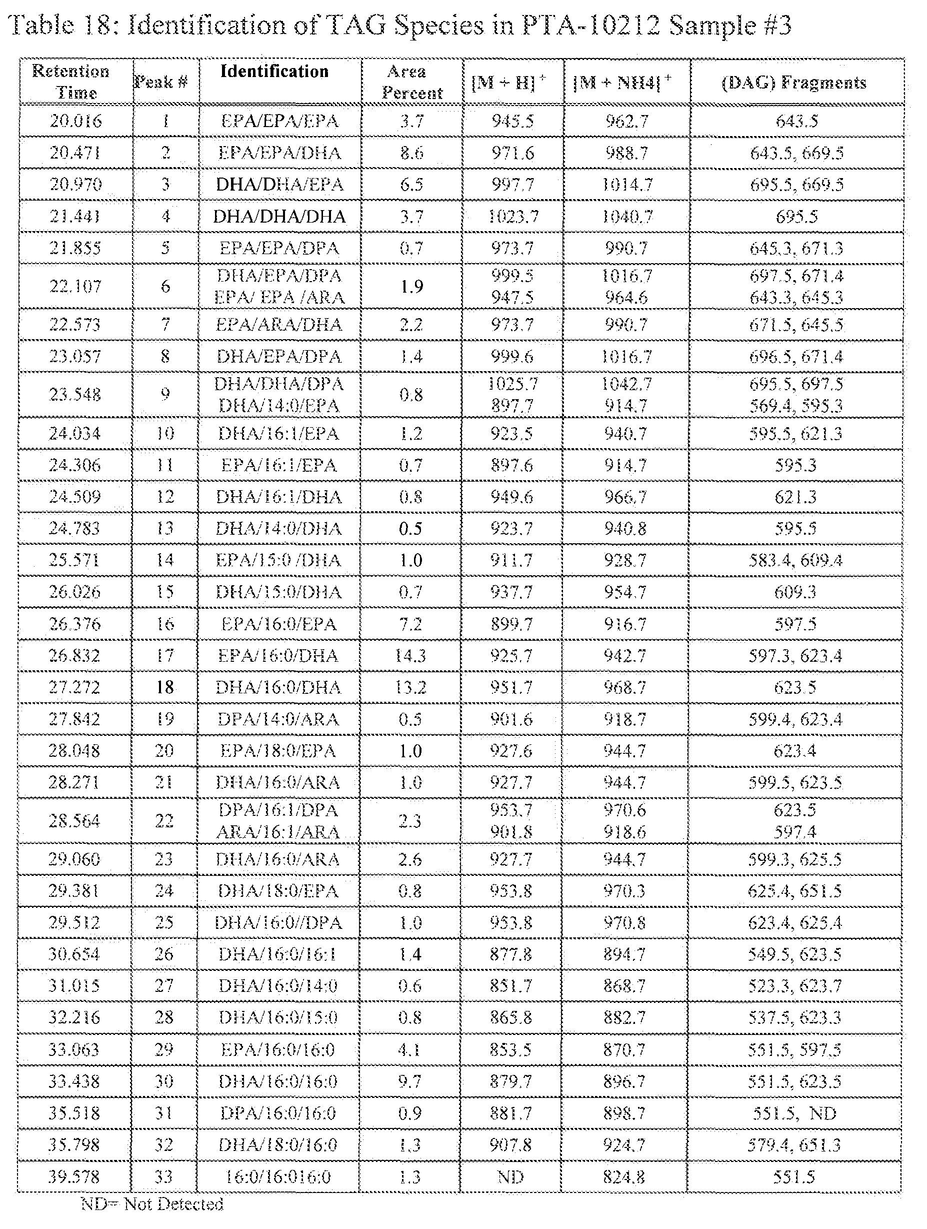

### PTA-10212 Sample #4

A sample of the TAG fraction of PTA-10212 Sample #4 was prepared in hexane and analyzed by HPLC/APCI/MS to determine the identities of individual TAG isomers.

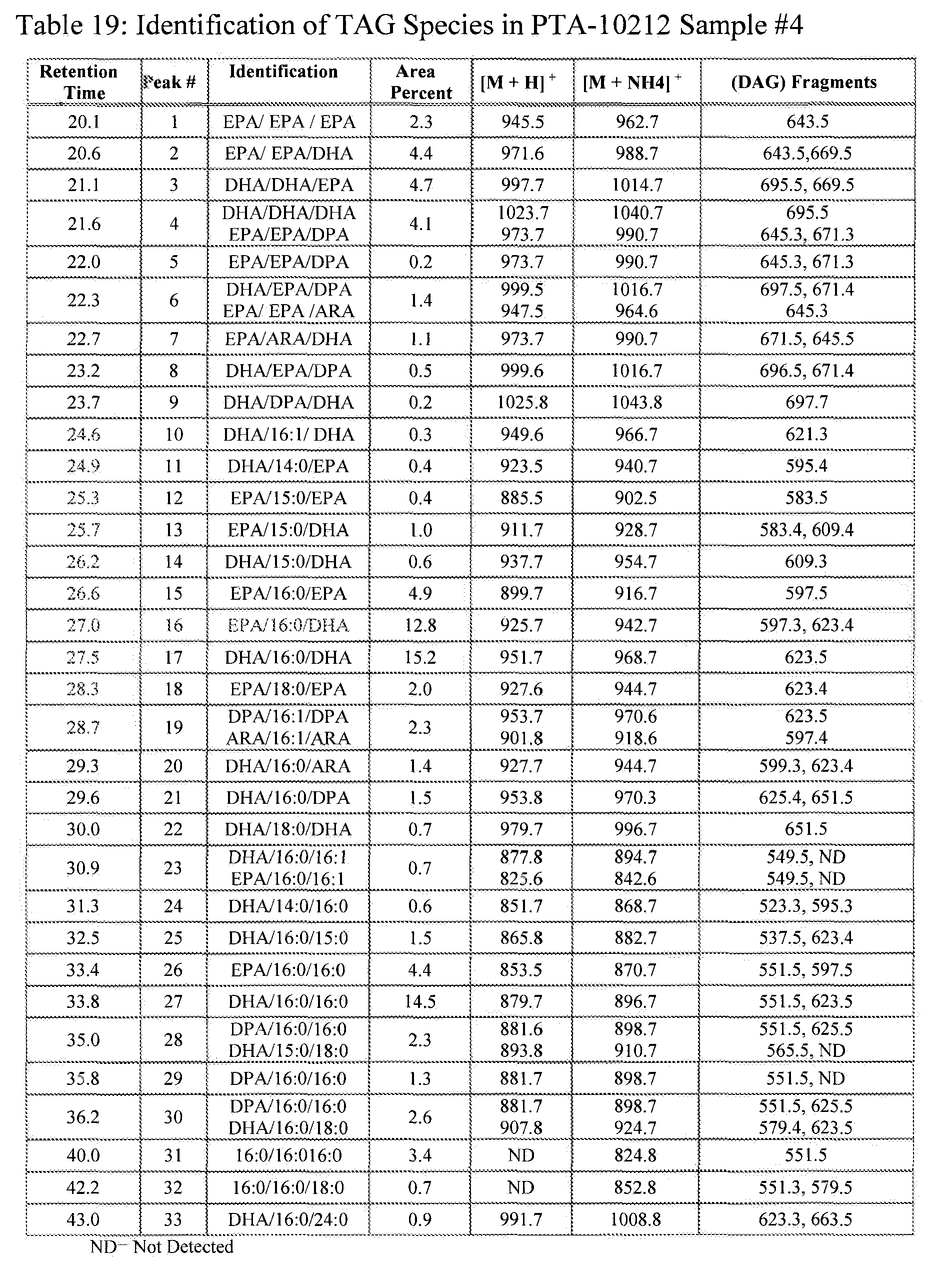

### PTA-10212 Sample #5

A sample of the TAG fraction of PTA-10212 Sample #5 was prepared in hexane and analyzed by HPLC/APCI/MS to determine the identities of individual TAG isomers.

**Table 20: Identification of TAG Species in PTA-10212 Sample #5**

| **Retention Time** | **Peak #** | **Identification** | **Area Percent** | **[M + H]⁺** | **[M + NH4]⁺** | **(DAG) Fragments** |
|---|---|---|---|---|---|---|
| 21.0 | 1 | EPA/EPA/EPA | 1.7 | 945.7 | 962.8 | 643.5 |
| 21.5 | 2 | EPA/EPA/DHA | 5.5 | 971.6 | 988.7 | 643.5 ,669.5 |
| 22.0 | 3 | DHA/DHA/EPA | 7.6 | 997.7 | 1014.7 | 695.5, 669.5 |
| 22.5 | 4 | DHA/DHA/DHA | 4.5 | 1023.8 | 1040.7 | 695.5 |
| 23.0 | 5 | EPA/EPA/DPA | 0.5 | 973.8 | 990.8 | 645.5, 671.5 |
| 23.3 | 6 | DHA/EPA/DPA EPA/EPA /ARA | 1.5 | 999.7 947.6 | 1016.7 964.8 | 697.5, 671.5 645.5 |
| 23.7 | 7 | EPA/ARA/DHA DHA/DPA/DHΔ | 1.9 | 973.6 1023.7 | 990.7 1042.7 | 671.5, 645.4 695.5, 697.5 |
| 24.2 | 8 | DHA/EPA/DPA | 1.1 | 999.7 | 1016.7 | 669.5, 671.4, 696.5 |
| 24.8 | 9 | DHA/DHA/DPA DHA/14:0/EPA | 0.6 | 1025.7 897.7 | 1042.9 914.7 | 695.5, 697.5 595.5 |
| 25.3 | 10 | DHA/16:1/EPA | 0.7 | 923.8 | 940.8 | 595.5, 621.3 |
| 25.6 | 11 | DHA/14:0/EPA | 0.6 | 897.7 | 914.7 | 569.3, 595.4 |
| 25.8 | 12 | DHN16:1/DHA | 0.5 | 949.7 | 966.7 | 621.3, 695.5 |
| 26.0 | 13 | DHA/16:1/EPA | 0.4 | 923.7 | 940.8 | 595.5, 621.3 |
| 26.9 | 14 | EPA/15:0/DHA | 0.7 | 911.7 | 928.7 | 583.5, 609.3 |
| 27.4 | 15 | DHA/15:0/DHA | 0.7 | 937.6 | 954.8 | 609.3 |
| 27.8 | 16 | EPA/16:0/EPA | 4.9 | 899.7 | 916.7 | 597.5 |
| 28.2 | 17 | EPA/16:0/DHA | 14.3 | 925.7 | 942.8 | 597.5, 623.5 |
| 28.7 | 18 | DHA/16:0/DHA | 12.2 | 951.7 | 968.8 | 623.5 |
| 29.3 | 19 | DPA/14:0/ARA | 0.6 | 901.7 | 918.8 | 597.3 |
| 29.5 | 20 | EPA/18:0/EPA | 1.5 | 927.7 | 944.8 | 625.5 |
| 30.0 | 21 | DHA/16:0/ARA | 3.4 | 953.7 | 970.8 | 623.4 |
| 30.6 | 22 | EPA/EPA/18:0 | 2.1 | 927.7 | 944.7 | 599.5, 625.5, 669.3 |
| 31.0 | 23 | DHA/18:0/EPA | 1.7 | 953.8 | 970.8 | 625.4, 651.5 |
| 31.3 | 24 | DHA/18:0/DHA | 0.9 | 979.7 | 996.8 | 651.5, 695.3 |
| 31.9 | 26 | 16:0/DHA/14:0 | 0.8 | 851.7 | 868.7 | 595.5, 623.5 |
| 32.3 | 27 | 18:1/14:0/DHA | 1.7 | 877.7 | 894.7 | 549.5, 595.5 |
| 32.6 | 28 | DHA/16:0/14:0 | 0.9 | 851.8 | 868.7 | 523.4, 623.5 |
| 33.5 | 29 | EPA/15:0/16:0 DHA/20:0/EPA | 0.7 | 839.7 981.7 | 856.7 998.8 | 537.5, 583.3 653.5, 679.6 |
| 33.9 | 30 | DHA/16:0/15:0 | 1.2 | 865.7 | 882.7 | 537.5, 623.5 |
| 34.8 | 31 | EPA/16:0/16:0 | 3.9 | 853.8 | 870.7 | 551.5, 597.5 |
| 35.2 | 32 | DHA/16:0/16:0 | 10.6 | 879.7 | 896.7 | 551.5, 623.5 |
| 36.4 | 33 | DPA/16:0/16:0 | 1.5 | 881.7 | 898.7 | 551.5, 625.5 |
| 37.4 | 33 | DPA/16:0/16:0 | 1.2 | 881.7 | 898.7 | 551.5, 625.5 |
| 37.7 | 34 | DHA/16:0/18:0 | 1.9 | 907.7 | 924.7 | 579.4 |
| 38.4 | 35 | EPA/24:0/DHA | 0.5 | 1037.8 | 1054.8 | 709.5, 735.6 |
| 38.8 | 36 | DHA/24:0/DHA | 1.0 | 1064.8 | 1081.8 | 735.7 |

### EXAMPLE 5

Crude oils were further processed via refining, bleaching, and deodorizing to obtain refined oils. The refined oils were diluted with high oleic sunflower oil to obtain final oils with a DHA content of approximately 400 mg/g. Individual lipid classes were isolated and the fatty acid profiles of each class was determined using GC-FID as FAME.

### PTA-10208 Final Oils

The fatty acid profiles for PTA-10208 Final Oils #1-5 are summarized in Tables 21-22, including profiles associated within the isolated TAG fraction (Tables 23-24) and the isolated sterols/DAG fraction (Tables 24-26).

Individual lipid classes in the final oils were also determined using flash chromatography (Table 27) and normal HPLC with ELSD and APCI-MS confirmation (Table 28).

**Table 21: Fatty Acid Profiles of PTA-10208 Final Oils (mg/g)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | FAME (mg/g) | FAME (mg/g) | FAME (mg/g) | FAME (mg/g) | FAME (mg/g) |
| C12:0 | 2.5 | 2.4 | 2.8 | 2.7 | 2.7 |
| C14:0 | 16.1 | 14.9 | 21.0 | 18.4 | 17.5 |
| C14:1 | 0.0 | 0.0 | 0.0 | 0.0 | 0.0 |
| C15:0 | 3.8 | 3.6 | 4.4 | 3.9 | 3.9 |
| C16:0 | 192.1 | 179.1 | 193.1 | 184.3 | 194.6 |
| C16:1 | 0.4 | 0.5 | 0.5 | 0.5 | 0.5 |
| C17:0 | 0.6 | 0.5 | 0.9 | 0.8 | 2.1 |
| C18:0 | 12.8 | 13.9 | 11.5 | 12.3 | 12.9 |
| C18:1 n-9 | 23.5 | 82.0 | 25.7 | 26.0 | 29.5 |
| C18:1 n-7 | 0.2 | 0.7 | 0.1 | 0.1 | 0.1 |
| C18:2 n-6 | 3.7 | 8.1 | 4.0 | 4.1 | 4.3 |
| C20:0 | 4.3 | 4.1 | 3.7 | 4.0 | 4.0 |
| C18:3 n-3 | 0.0 | 0.0 | 0.0 | 0.0 | 0.0 |
| C20:1 n-9 | <0.1 | 0.1 | <0.1 | <0.1 | <0.1 |
| C18:4 n-3 | 2.4 | 2.5 | 2.8 | 2.7 | 2.8 |
| C20:2 n-6 | 0.0 | 0.0 | 0.0 | 0.0 | 0.0 |
| C20:3 n-6 | 0.2 | 0.1 | 0.1 | 0.1 | 0.1 |
| C22:0 | 1.2 | 1.8 | 1.0 | 1.1 | 1.1 |
| C20:4 n-7 | 1.7 | 1.6 | 1.7 | 1.8 | 1.6 |
| C20:3 n-3 | 0.0 | 0.0 | 0.0 | 0.0 | 0.0 |
| C20:4 n-6 | 12.9 | 12.1 | 13.5 | 13.5 | 13.3 |
| C22:1 n-9 | 0.0 | 0.0 | 0.0 | 0.0 | 0.0 |
| C20:4 n-5 | 1.6 | 1.4 | 1.5 | 1.7 | 1.5 |
| C20:4 n-3 | 6.0 | 5.7 | 6.0 | 6.0 | 6.1 |
| C20:5 n-3 | 173.8 | 163.3 | 196.4 | 209.6 | 197.9 |
| C24:0 | 1.4 | 1.6 | 1.3 | 1.3 | 1.0 |
| C22:4 \n-9 | 0.0 | +0.0 | 0.0 | 0.0 | 0.0 |
| C24:1 n-9 | 3.4 | 3.2 | 2.3 | 2.6 | 2.3 |
| C22:5 n-6 | 14.9 | 14.0 | 14.4 | 13.0 | 12.9 |
| C22:5 n-3 | 43.9 | 41.3 | 32.8 | 40.3 | 36.9 |
| C22:6 n-3 | 394.8 | 373.7 | 373.2 | 374.3 | 364.2 |
| Sum of FAME | 918.1 | 932.2 | 914.7 | 925.1 | 914.1 |

**Table 22: Fatty Acid Profiles of PTA-10208 Final Oils (%)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | % FAME | % FAME | % FAME | % FAME | % FAME |
| C12:0 | 0.3 | 0.3 | 0.3 | 0.3 | 0.3 |
| C14:0 | 1.8 | 1.6 | 2.3 | 2.0 | 1.9 |
| C14:1 | 0.0 | 0.0 | 0.0 | 0.0 | 0.0 |
| C15:0 | 0.4 | 0.4 | 0.5 | 0.4 | 0.4 |
| C16:0 | 20.9 | 19.2 | 21.1 | 19.9 | 21.3 |
| C16:1 | <0.1 | <0.1 | <0.1 | <0.1 | 0.1 |
| C17:0 | 0.1 | 0.1 | 0.1 | 0.1 | 0.2 |
| C18:0 | 1.4 | 1.5 | 1.3 | 1.3 | 1.4 |
| C18:1 n-9 | 2.6 | 8.8 | 2.8 | 2.8 | 3.2 |
| C18:1 n-7 | <0.1 | 0.1 | <0.1 | <0.1 | <0.1 |
| C18:2 n-6 | 0.4 | 0.9 | 0.4 | 0.4 | 0.5 |
| C20:0 | 0.5 | 0.4 | 0.4 | 0.4 | 0.4 |
| C18:3 n-3 | 0.0 | 0.0 | 0.0 | 0.0 | 0.0 |
| C20:1 n-9 | <0.1 | <0.1 | <0.1 | <0.1 | <0.1 |
| C18:4 n-3 | 0.3 | 0.3 | 0.3 | 0.3 | 0.3 |
| C20:2 n-6 | 0.0 | 0.0 | 0.0 | 0.0 | 0.0 |
| C20:3 n-6 | <0.1 | <0.1 | <0.1 | <0.1 | <0.1 |
| C22:0 | 0.1 | 0.2 | 0.1 | 0.1 | 0.1 |
| C20:4 n-7 | 0.2 | 0.2 | 0.2 | 0.2 | 0.2 |
| C20:3 n-3 | 0.0 | 0.0 | 0.0 | 0.0 | 0.0 |
| C20:4 n-6 | 1.4 | 1.3 | 1.5 | 1.5 | 1.5 |
| C22:1 n-9 | 0.0 | 0.0 | 0.0 | 0.0 | 0.0 |
| C20:4 \n-5 | 0.2 | 0.2 | 0.2 | 0.2 | 0.2 |
| C20:4 \n-3 | 0.7 | 0.6 | 0.7 | 0.7 | 0.7 |
| C20:5 n-3 | 18.9 | 17.5 | 21.5 | 22.7 | 21.6 |
| C24:0 | 0.1 | 0.2 | 0.1 | 0.1 | 0.1 |
| C22:4 n-9 | 0.0 | 0.0 | 0.0 | 0.0 | 0.0 |
| C24:1 n-9 | 0.4 | 0.3 | 0.2 | 0.3 | 0.2 |
| C22:5 n-6 | 1.6 | 1.5 | 1.6 | 1.4 | 1.4 |
| C22:5 n-3 | 4.8 | 4.4 | 3.6 | 4.4 | 4.0 |
| C22:6 n-3 | 43.0 | 40.1 | 40.8 | 40.5 | 39.9 |

**Table 23: Isolated TAG Fatty Acid Profiles: PTA-10208 Final Oils (mg/g)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | FAME (mg/g) | FAME (mg/g) | FAME (mg/g) | FAME (mg/g) | FAME (mg/g) |
| C12:0 | 2.5 | 2.3 | 2.7 | 2.5 | 2.6 |
| C14:0 | 16.3 | 15.1 | 21.3 | 18.6 | 18.1 |
| C14:1 | 0.0 | 0.0 | 0.0 | 0.0 | 0.0 |
| C15:0 | 3.9 | 3.6 | 4.4 | 4.0 | 4.0 |
| C16:0 | 194.2 | 181.9 | 196.1 | 186.1 | 199.8 |
| C16:1 | 0.4 | 0.4 | 0.6 | 0.5 | 0.7 |
| C17:0 | 0.6 | 0.5 | 0.9 | 0.8 | 0.8 |
| C18:0 | 12.9 | 14.2 | 11.7 | 12.5 | 13.2 |
| C18:1 n-9 | 24.3 | 84.0 | 26.8 | 26.1 | 34.0 |
| C18:1 n-7 | 0.1 | 0.7 | 0.1 | 0.1 | 0.3 |
| C18:2 n-6 | 3.2 | 7.7 | 3.4 | 3.5 | 4.0 |
| C20:0 | 4.4 | 4.2 | 3.8 | 4.0 | 4.2 |
| C18:3 n-3 | 0.0 | 0.0 | 0.0 | 0.0 | 0.0 |
| C20:1 n-9 | <0.1 | 0.2 | <0.1 | <0.1 | 0.1 |
| C18:4 n-3 | 2.5 | 2.4 | 2.8 | 2.6 | 2.7 |
| C20:2 n-6 | 0.0 | 0.0 | 0.0 | 0.0 | 0.0 |
| C20:3 n-6 | 0.2 | 0.2 | 0.1 | 0.1 | 0.1 |
| C22:0 | 1.2 | 1.9 | 1.0 | 1.1 | 1.1 |
| C20:4 n-7 | 1.7 | 1.6 | 1.8 | 1.8 | 1.7 |
| C20:3 n-3 | 0.0 | 0.0 | 0.0 | 0.0 | 0.0 |
| C20:4 n-6 | 13.2 | 12.3 | 13.8 | 13.7 | 13.8 |
| C22:1 n-9 | 0.0 | 0.0 | 0.0 | 0.0 | 0.0 |
| C20:4 n-5 | 1.6 | 1.5 | 1.6 | 1.7 | 1.5 |
| C20:4 n-3 | 6.1 | 5.7 | 6.1 | 5.9 | 6.2 |
| C20:5 n-3 | 176.0 | 166.1 | 199.0 | 211.2 | 204.2 |
| C24:0 | 1.2 | 1.3 | 1.0 | 1.1 | 1.2 |
| C22:4 n-9 | 0.0 | 0.0 | 0.0 | 0.0 | 0.0 |
| C24:1 n-9 | 3.3 | 3.2 | 2.2 | 2.5 | 2.4 |
| C22:5 n-6 | 15.0 | 14.2 | 14.7 | 13.2 13.5 | |
| C22:5 n-3 | 44.4 | 42.0 | 33.3 | 40.5 | 38.3 |
| C22:6 n-3 | 397.9 | 378.4 | 376.4 | 375.5 | 375.5 |
| Sum of FAME | 926.9 | 945.7 | 925.5 | 929.6 | 944.1 |

**Table 24: Isolated TAG Fatty Acid Profiles: PTA-10208 Final Oils (%)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | % FAME | % FAME | % FAME | % FAME | % FAME |
| C12:0 | 0.3 | 0.2 | 0.3 | 0.3 | 0.3 |
| C14:0 | 1.8 | 1.6 | 0.3 | 0.3 | 0.3 |
| C14:1 | 0.0 | 0.0 | 0.0 | 0.0 | 0.0 |
| C15:0 | 0.4 | 0.4 | 0.5 | 0.4 | 0.4 |
| C16:0 | 20.9 | 19.2 | 21.2 | 20.0 | 21.2 |
| C16:1 | <0.1 | <0.1 | 0.1 | 0.1 | 0.1 |
| C17:0 | 0.1 | 0.1 | 0.1 | 0.1 | 0.1 |
| C18:0 | 1.4 | 1.5 | 1.3 | 1.3 | 1.4 |
| C18:1 n-9 | 2.6 | 8.9 | 2.9 | 2.8 | 3.6 |
| C18:1 n-7 | <0.1 | 0.1 | <0.1 | <0.1 | <0.1 |
| C18:2 n-6 | 0.3 | 0.8 | 0.4 | 0.4 | 0.4 |
| C20:0 | 0.5 | 0.4 | 0.4 | 0.4 | 0.4 |
| C18:3 n-3 | 0.0 | 0.0 | 0.0 | 0.0 | 0.0 |
| C20:1 n-9 | <0.1 | <0.1 | <0.1 | <0.1 | <0.1 |
| C18:4 n-3 | 0.3 | 0.3 | 0.3 | 0.3 | 0.3 |
| C20:2 n-6 | 0.0 | 0.0 | 0.0 | 0.0 | 0.0 |
| C20:3 n-6 | <0.1 | <0.1 | <0.1 | <0.1 | <0.1 |
| C22:0 | 0.1 | 0.2 | 0.1 | 0.1 | 0.1 |
| C20:4 n-7 | 0.2 | 0.2 | 0.2 | 0.2 | 0.2 |
| C20:3 n-3 | 0.0 | 0.0 | 0.0 | 0.0 | 0.0 |
| C20:4 n-6 | 1.4 | 1.3 | 1.5 | 1.5 | 1.5 |
| C22:1 n-9 | 0.0 | 0.0 | 0.0 | 0.0 | 0.0 |
| C20:4 n-5 | 0.2 | 0.2 | 0.2 | 0.2 | 0.2 |
| C20:4 n-3 | 0.7 | 0.6 | 0.7 | 0.6 | 0.7 |
| C20:5 n-3 | 19.0 | 17.6 | 21.5 | 22.7 | 21.6 |
| C24:0 | 0.1 | 0.1 | 0.1 | 0.1 | 0.1 |
| C22:4 n-9 | 0.0 | 0.0 | 0.0 | 0.0 | 0.0 |
| C24:1 n-9 | 0.4 | 0.3 | 0.2 | 0.3 | 0.3 |
| C22:5 n-6 | 1.6 | 1.5 | 1.6 | 1.4 | 1.4 |
| C22:5 n-3 | 4.8 | 4.4 | 3.6 | 4.4 | 4.1 |
| C22:6 n-3 | 42.9 | 40.0 | 40.7 | 40.4 | 39.8 |

**Table 25: Isolated Sterols/DAG Fatty Acid Profiles: PTA-10208 Final Oils (mg/g)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | FAME (mg/g) | FAME (mg/g) | FAME (mg/g) | FAME (mg/g) | FAME (mg/g) |
| C12:0 | 1.9 | 2.1 | 2.9 | 2.1 | 1.9 |
| C14:0 | 9.9 | 9.5 | 9.7 | 10.3 | 8.0 |
| C14:1 | 0.0 | 0.0 | 0.0 | 0.0 | 0.0 |
| C15:0 | 2.4 | 2.3 | 2.2 | 2.3 | 2.0 |
| C16:0 | 132.6 | 128.6 | 110.1 | 116.8 | 106.4 |
| C16:1 | 0.2 | 0.3 | <0.1 | 0.3 | 0.4 |
| C17:0 | 0.3 | 0.2 | 0.3 | 0.3 | 0.3 |
| C18:0 | 7.3 | 8.1 | 6.4 | 6.8 | 6.1 |
| C18:1 n-9 | 15.0 | 55.1 | 47.4 | 19.0 | 30.1 |
| C18:1 n-7 | 0.4 | 0.7 | 0.1 | <0.1 | 0.2 |
| C18:2 n-6 | 13.1 | 16.7 | 21.6 | 13.5 | 18.4 |
| C20:0 | 2.0 | 2.1 | 1.2 | 1.8 | 1.4 |
| C18:3 n-3 | 0.0 | 0.0 | 0.0 | 0.0 | 0.0 |
| C20:1 n-9 | <0.1 | <0.1 | <0.1 | <0.1 | <0.1 |
| C18:4 n-3 | 2.3 | 2.4 | 2.4 | 2.4 | 2.0 |
| C20:2 n-6 | 0.0 | 0.0 | 0.0 | 0.0 | 0.0 |
| C20:3 n-6 | <0.1 | <0.1 | <0.1 | <0.1 | <0.1 |
| C22:0 | 0.6 | 1.0 | 0.5 | 0.6 | 0.5 |
| C20:4 n-7 | 0.8 | 0.9 | 2.1 | 0.9 | 0.7 |
| C20:3 n-3 | 0.0 | 0.0 | 0.0 | 0.0 | 0.0 |
| C20:4 n-6 | 5.7 | 5.8 | 4.8 | 6.1 | 4.5 |
| C22:1 n-9 | 0.0 | 0.0 | 0.0 | 0.0 | 0.0 |
| C20:4 n-5 | <0.1 | <0.1 | <0.1 | 0.6 | <0.1 |
| C20:4 n-3 | 2.7 | 2.7 | 2.1 | 2.7 | 2.0 |
| C20:5 n-3 | 92.9 | 94.5 | 91.9 | 111.6 | 84.8 |
| C24:0 | 1.2 | 1.3 | 1.1 | 1.1 | 1.3 |
| C22:4 n-9 | 0.0 | 0.0 | 0.0 | 0.0 | 0.0 |
| C24:1 n-9 | 1.9 | 2.0 | 1.2 | 1.5 | 1.2 |
| C22:5 n-6 | 7.8 | 8.0 | 6.7 | 7.0 | 5.5 |
| C22:5 n-3 | 22.2 | 22.9 | 13.9 | 20.7 | 14.2 |
| C22:6 n-3 | 246.3 | 252.7 | 223.5 | 240.3 | 196.3 |
| Sum of FAME | 569.3 | 619.8 | 552.1 | 568.7 | 488.2 |

**Table 26: Isolated Sterols/DAG Fatty Acid Profiles: PTA-10208 Final Oils (%)**

| | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| Fatty Acid | % FAME | % FAME | % FAME | % FAME | % FAME |
| C12:0 | 0.3 | 0.3 | 0.5 | 0.4 | 0.4 |
| C14:0 | 1.7 | 1.5 | 1.8 | 1.8 | 1.6 |
| C14:1 | 0.0 | 0.0 | 0.0 | 0.0 | 0.0 |
| C15:0 | 0.4 | 0.4 | 0.4 | 0.4 | 0.4 |
| C16:0 | 23.3 | 20.8 | 19.9 | 20.5 | 21.8 |
| C16:1 | <0.1 | <0.1 | <0.1 | <0.1 | 0.1 |
| C17:0 | 0.0 | 0.0 | 0.1 | 0.1 | 0.1 |
| C18:0 | 1.3 | 1.3 | 1.2 | 1.2 | 1.2 |
| C18:1 n-9 | 2.6 | 8.9 | 8.6 | 3.3 | 6.2 |
| C18:1 n-7 | 0.1 | 0.1 | <0.1 | <0.1 | <0.1 |
| C18:2 n-6 | 2.3 | 2.7 | 3.9 | 2.4 | 3.8 |
| C20:0 | 0.4 | 0.3 | 0.2 | 0.3 | 0.3 |
| C18:3 n-3 | 0.0 | 0.0 | 0.0 | 0.0 | 0.0 |
| C20:1 n-9 | <0.1 | <0.1 | <0.1 | <0.1 | <0.1 |
| C18:4 n-3 | 0.4 | 0.4 | 0.4 | 0.4 | 0.4 |
| C20:2 n-6 | 0.0 | 0.0 | 0.0 | 0.0 | 0.0 |
| C20:3 n-6 | <0.1 | <0.1 | <0.1 | <0.1 | <0.1 |
| C22:0 | 0.1 | 0.2 | 0.1 | 0.1 | 0.1 |
| C20:4 n-7 | 0.1 | 0.1 | 0.4 | 0.2 | 0.1 |
| C20:3 n-3 | 0.0 | 0.0 | 0.0 | 0.0 | 0.0 |
| C20:4 n-6 | 1.0 | 0.9 | 0.9 | 1.1 | 0.9 |
| C22:1 n-9 | 0.0 | 0.0 | 0.0 | 0.0 | 0.0 |
| C20:4 n-5 | <0.1 | <0.1 | <0.1 | 0.1 | <0.1 |
| C20:4 n-3 | 0.5 | 0.4 | 0.4 | 0.5 | 0.4 |
| C20:5 n-3 | 16.3 | 15.2 | 16.6 | 19.6 | 17.4 |
| C24:0 | 0.2 | 0.2 | 0.2 | 0.2 | 0.3 |
| C22:4 n-9 | 0.0 | 0.0 | 0.0 | 0.0 | 0.0 |
| C24:1 n-9 | 0.3 | 0.3 | 0.2 | 0.3 | 0.2 |
| C22:5 n-6 | 1.4 | 1.3 | 1.2 | 1.2 | 1.1 |
| C22:5 n-3 | 3.9 | 3.7 | 2.5 | 3.6 | 2.9 |
| C22:6 n-3 | 43.3 | 40.8 | 40.5 | 42.3 | 40.2 |

**Table 27: Lipid class separation by flash chromatography (wt %)**

| Lipid Class Separation | PTA-10208 Final Oil #1 | PTA-10208 Final Oil #2 | PTA-10208 Final Oil #3 | PTA-10208 Final Oil #4 | PTA-10208 Final Oil #5 |
|---|---|---|---|---|---|
| TAG | 93.4 | 95.4 | 94.0 | 95.7 | 95.1 |
| Sterols/DAG | 3.1 | 2.9 | 2.6 | 3.0 | 2.9 |
| Recovery (%) | 96.5 | 98.3 | 96.6 | 98.7 | 98.0 |

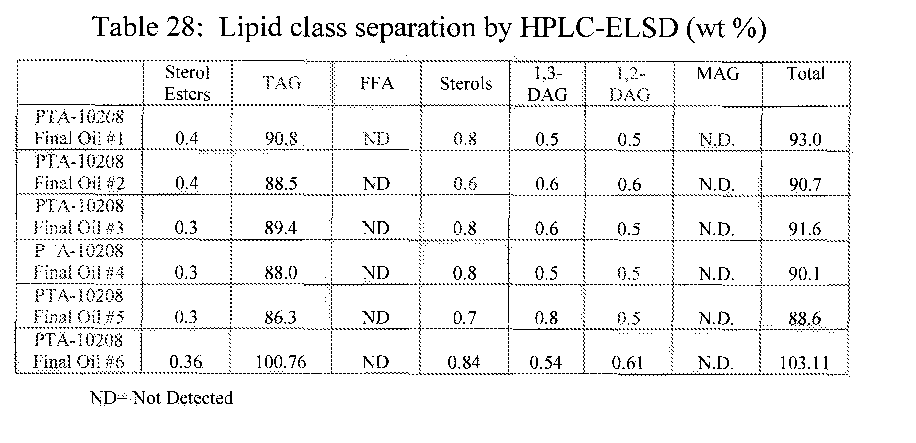

### PTA-10212 Final Oil

DHA was present in a PTA-10212 Final Oil at 41.63% and 366.9 mg/g, while EPA was present at 16.52%. Individual fatty acid profiles were determined and are summarized in Table 29.

**Table 29: Fatty Acid Profiles of PTA-10212 Final Oil (% FAME)**

| Fatty Acid | % FAME |
|---|---|
| C6:0 | ND |
| C7:0 | ND |
| C8:0 | ND |
| C9:0 | ND |
| C10:0 | ND |
| C11:0 | ND |
| C12:0 | ND |
| C13:0 | ND |
| C14:0 | 0.84 |
| C14:1 | ND |
| C15:0 | 1.33 |
| C16:0 | 27.09 |
| C16:1 | 1.03 |
| C17:0 | 0.34 |
| C17:1 | ND |
| C18:0 | 1.26 |
| C18:1 n-9 | 2.14 |
| C18:1 n-7 | 0.18 |
| C19:0 | ND |
| C18:2 n-6 | 0.58 |
| C20:0 | 0.32 |
| C18:3 n-3 | ND |
| C20:1 n-9 | ND |
| C18:3 n-6 | ND |
| C20:2 n-6 | 0.26 |
| C20:3 n-6 | ND |
| C22:0 | 0.14 |
| C20:3 n-3 | ND |
| C20:4 n-6 | 1.34 |
| C22:1 n-9 | ND |
| C23:0 | ND |
| C20:5 n-3 | 16.53 |
| C24:0 | 0.53 |
| C24:1 n-9 | ND |
| C22:5 n-6 | 1.50 |
| C22:5 n-3 | 1.30 |
| C22:6 n-3 | 41.63 |
| Unknown | 0.87 |

| | |
|---|---|
| ND Not Detected | |

### EXAMPLE 6

An analysis of the triacylglycerides (TAGs) of the PTA-10208 final oils described in Example 5 was performed using techniques described in Example 4. The identification of each fatty acid moiety was made, as summarized in Table 30 below.

**Table 30: Identification of TAG Species in PTA-10208 Final Oil**

| **Identification** | PTA-10208 Final Oil #1 **Area %** | PTA-10208 Final Oil #2 **Area %** | PTA-10208 Final Oil #3 **Area %** | PTA-10208 Final Oil #4 **Area %** | PTA-10208 Final Oil #5 **Area %** |
|---|---|---|---|---|---|
| EPA/EPA/EPA | 1.3 | 1.0 | 1.9 | 1.7 | 1.4 |
| EPA/EPA/DHA | 8.2 | 6.0 | 7.3 | 6.8 | 6.4 |
| DHA/DHA/EPA | 14.2 | 11.1 | 10.6 | 9.5 | 8.7 |
| DHA/DHA/DHA | 10.2 | 8.3 | 7.6 | 6.1 | 5.7 |
| DPA/EPA/EPA | 1.2 | 0.9 | 1.0 | 1.1 | 1.1 |
| DHA/DPA/EPA | 3.0 | 2.4 | 2.5 | 2.3 | 2.9 |
| DHA/EPA/ARA DHA/DPA/DHA | 3.8 | 3.0 | 3.0 | 3.0 | 2.3 |
| DHA/DPA/EPA | 2.3 | 1.9 | 1.5 | 1.6 | 1.7 |
| DHA/DPA/DHA | 1.7 | 1.2 | 1.1 | 1.2 | 1.2 |
| EPA/14:0/DHA | 1.1 | 1.0 | 1.8 | 1.8 | 1.5 |
| DHA/DHA/14:0 | 1.1 | 1.0 | 1.4 | 1.3 | 1.3 |
| EPA/EPA/16:0 | 2.3 | 2.3 | 3.4 | 3.9 | 3.3 |
| DHA/16:0/EPA | 12.1 | 12.5 | 12.9 | 14.0 | 13.4 |
| DHA/16:0/DHA | 16.1 | 16.8 | 17.5 | 14.8 | 17.2 |
| EPA/EPA/18:0 | 2.1 | 2.0 | 1.7 | 2.1 | 2.7 |
| DHA/DPA/16:0 | 3.0 | 3.3 | 2.3 | 2.9 | 2.7 |
| DHA/16:0/ARA | 1.6 | 1.6 | 1.8 | 2.0 | 2.2 |
| DHA/16:0/DPA | 1.3 | 2.1 | 1.4 | 1.5 | 2.5 |
| DHA/18:0/DHA | 0.8 | 0.8 | 0.7 | 0.8 | 1.0 |
| DHA/14:0/16:0 | 0.6 | 1.0 | 1.3 | 1.4 | 1.3 |
| EPA/16:0/16:0 | 0.9 | 1.1 | 1.5 | 1.7 | 2.0 |
| DHA/16:0/16:0 | 3.6 | 4.8 | 5.5 | 5.7 | 6.5 |
| 18:0/16:0/DHA | 0.4 | 0.8 | 0.7 | 0.8 | 1.2 |
| 18:1/18:1/18:1 | 0.6 | 4.0 | 1.0 | 1.4 | 1.6 |

### EXAMPLE 7

A two-day old inoculum flask of the isolated microorganisms deposited under ATCC Accession Nos. PTA-10208 and 10212 was prepared as a carbon and nitrogen-fed culture in media according to Tables 1 and 2.

Mutagenesis was carried out according to following procedure:
A sterile T=2 day old flask, approximately 50 ml, was poured into a sterile 40 ml glass homogenizer. The culture received 50 plunges in the homogenizer. The culture was pipeted out and filtered through a sterile 50 micron mesh filter, which was placed in a 50 ml sterile tube (the mesh was used as a means of retaining the larger clumps of colonies while letting the smaller clusters and single cells pass through the 50 micron mesh.). The entire concentrated macerate was collected in a sterile 50 ml tube. The macerated culture was vortexed and dilutions at levels up to 1:100 fold were made. The diluted macerate samples were vortexed prior to adding 200 µl of inoculum to a media agar plate, 100 x 15 mm, containing 4-5 glass beads (3 mm glass beads). Each plate was gently agitated in an effort to have the beads spread the inoculum evenly around the plate. Beads were dumped off of plates and plates were left to sit with covers on for approximately 5 minutes to dry. Lights in both the sterile hood and adjoining areas were turned off as the procedure was performed in dim light. There was minimal light available to be able to run the procedure but only indirect and dim.

Five replicate plates were placed on the floor of the XL crosslinker (Spectronics Corporation, New York) with the lids off while the samples were irradiated. The crosslinker delivered power in terms of microjoules and a level was sought that achieved a 90%-95% Kill. Five replicate control plates were inoculated with un-mutagenized cells using the same protocol. These cell counts were used to calculate the % Kill. Once the irradiation was finished the plates were taken out, the lids were replaced, and the plates were wrapped in parafilm followed by a wrap in aluminum foil. It was imperative that the plates grew for the first week in the dark so that they were not able to repair the damaged genes.

Plates were placed in a 22.5°C room for about 10 days prior to counting the colonies. When final counts were made, individual colonies were picked with a sterile inoculating loop and re-streaked on new media plates. Each colony was plated on an individual plate. As plates grew dense a sample was taken, using a inoculating loop, and inoculated into a sterile 250 ml shake flask containing 50 ml of media. This flask was placed on a shaker at 200 rpm in a 22.5°C room. On T=7 days the shake flask culture was harvested into a 50 ml sterile tube. The pH was taken and the sample was centrifuged to collect the biomass pellet. Each sample was rinsed and re-suspended in a 50:50 mixture of isopropyl alcohol and distilled water prior to being re-centrifuged. The collected pellet was freeze dried, weighed, and a FAME analysis was performed. The data in Tables 31 and 32 represents mutants produced with the above process from strains PTA-10208 and PTA-10212, respectively.

**Table 31: PTA-10208 Mutants**

| Fatty Acids | control PTA-10208 | Mutant 1 PTA-10209 | Mutant 2 PTA-10210 | Mutant 3 PTA-10211 |
|---|---|---|---|---|
| % 08:0 | 0.00 | 0.00 | 0.00 | 0.00 |
| % 09:0 | 0.00 | 0.00 | 0.00 | 0.00 |
| % 10:0 | 0.00 | 0.00 | 0.00 | 0.00 |
| % 11:0 | 0.00 | 0.00 | 0.00 | 0.00 |
| % 11:1 | 0.00 | 0.00 | 0.00 | 0.00 |
| % 12:0 | 0.11 | 0.10 | 0.22 | 0.19 |
| % 12:1 | 0.00 | 0.00 | 0.00 | 0.00 |
| % 13:0 | 0.19 | 0.19 | 0.15 | 0.16 |
| % 13:1 | 0.00 | 0.00 | 0.00 | 0.00 |
| % 14:0 | 1.94 | 1.82 | 2.98 | 2.59 |
| % 14:1 | 0.00 | 0.00 | 0.00 | 0.00 |
| % 15:1 | 2.66 | 2.22 | 1.76 | 1.66 |
| % 16:0 | 24.87 | 24.97 | 23.71 | 25.01 |
| % 16:1 | 0.20 | 0.25 | 0.07 | 0.07 |
| % 16:2 | 0.00 | 0.00 | 0.00 | 0.00 |
| % 16:3 | 0.00 | 0.00 | 0.00 | 0.00 |
| % 17:0 | 1.49 | 1.21 | 0.62 | 0.66 |
| % 18:0 | 1.13 | 1.14 | 0.91 | 1.01 |
| % 18:1 n-9 | 0.07 | 0.07 | 0.06 | 0.06 |
| % 18:1 n-7 | 0.00 | 0.00 | 0.00 | 0.00 |
| % 18:2 | 0.00 | 0.00 | 0.00 | 0.00 |
| % 18:3 n-6 | 0.00 | 0.00 | 0.05 | 0.04 |
| % 18:3 n-3 | 0.09 | 0.08 | 0.17 | 0.14 |
| % 18:4 n-3 | 0.00 | 0.00 | 0.00 | 0.00 |
| % 20:0 | 0.31 | 0.33 | 0.24 | 0.30 |
| % 20:1 n-9 | 0.00 | 0.04 | 0.00 | 0.00 |
| % 20:2 | 0.00 | 0.00 | 0.05 | 0.00 |
| % 20:3 n-9 | 0.00 | 0.00 | 0.00 | 0.00 |
| % 20:3 n-6 : | 0.12 | 0.13 | 0.08 | 0.04 |
| % 20:3 n-3 | 0.42 | 0.42 | 0.08 | 0.06 |
| % 20:4 ARA | 0.68 | 0.67 | 1.44 | 1.11 |
| % 20:5 n-3 EPA | 6.56 | 6.47 | 11.99 | 9.87 |
| % 22:0 | 0.07 | 0.07 | 0.06 | 0.07 |
| % 22:1 | 0.00 | 0.00 | 0.00 | 0.00 |
| % 22:2 | 0.11 | 0.09 | 0.10 | 0.08 |
| % 22:3 | 0.00 | 0.00 | 0.00 | 0.00 |
| % 22:4 n-6 | 0.00 | 0.00 | 0.00 | 0.00 |
| % 22:5 n-6 . | 2.32 | 2.36 | 2.36 | 2.36 |
| % 22:5 n-3 | 0.48 | 0.66 | 0.66 | 0.52 |
| % 22:6 n-3 DHA | 51.58 | 52.27 | 48.17 | 49.35 |
| % 24:0 | 0.00 | 0.00 | 0.00 | 0.00 |
| % 24:1 | 0.00 | 0.00 | 0.00 | 0.00 |
| % Fat | 47.87 | 49.41 | 66.00 | 63.12 |
| % Unknown | 4.61 | 4.45 | 4.07 | 4.64 |

**Table 32: PTA-10212 Mutants**

| Fatty Acids | Control PTA-10212 | Mutant 1 PTA-10213 | Mutant 2 PTA-10214 | Mutant 3 PTA-10215 |
|---|---|---|---|---|
| % 08:0 | 0.00 | 0.00 | 0.00 | 0.00 |
| % 09:0 | 0.00 | 0.00 | 0.00 | 0.00 |
| % 10:0 | 0.00 | 0.00 | 0.00 | 0.00 |
| % 11:0 | 0.00 | 0.00 | 0.00 | 0.00 |
| % 11:1 | 0.00 | 0.00 | 0.00 | 0.00 |
| % 12:0 | 0.00 | 0.00 | 0.00 | 0.00 |
| % 12:1 | 0.00 | 0.00 | 0.00 | 0.00 |
| % 13:0 | 0.00 | 0.00 | 0.21 | 0.20 |
| % 13:1 | 0.00 | 0.00 | 0.00 | 0.00 |
| % 14:0 | 0.68 | 0.77 | 0.62 | 0.97 |
| % 14:1 | 0.00 | 0.00 | 0.00 | 0.00 |
| % 15:1 | 0.00 | 0.00 | 0.00 | 0.00 |
| % 16:0 | 17.36 | 19.94 | 15.27 | 23.61 |
| % 16:1 | 1.45 | 2.33 | 1.40 | 2.57 |
| % 16:2 | 0.00 | 0.00 | 0.00 | 0.00 |
| % 16:3 | 0.00 | 0.00 | 0.00 | 0.00 |
| % 17:0 | 0.20 | 0.21 | 0.18 | 0.27 |
| % 18:0 | 0.78 | 0.82 | 0.79 | 0.81 |
| % 18:1 n-9 | 0.00 | 0.00 | 0.00 | 0.00 |
| % 18:1 n-7 | 0.18 | 0.27 | 0.20 | 0.19 |
| % 18:2 | 0.00 | 0.00 | 0.00 | 0.00 |
| % 18:3 n-6 | 0.00 | 0.00 | 0.00 | 0.00 |
| % 18:3 n-3 | 0.00 | 0.00 | 0.00 | 0.00 |
| % 18:4 n-3 | 0.00 | 0.00 | 0.00 | 0.00 |
| % 20:0 | 0.00 | 0.00 | 0.00 | 0.00 |
| % 20:1 n-9 | 0.00 | 0.00 | 0.00 | 0.00 |
| % 20:2 | 0.00 | 0.00 | 0.00 | 0.00 |
| % 20:3 n-9 | 0.00 | 0.00 | 0.00 | 0.00 |
| % 20:3 n-6 | 0.00 | 0.00 | 0.00 | 0.00 |
| % 20:3 n-3 | 0.90 | 0.77 | 0.99 | 0.66 |
| % 20:4 ARA | 1.43 | 1.32 | 1.65 | 0.72 |
| % 20:5 n-3 EPA | 13.33 | 14.93 | 14.14 | 8.54 |
| % 22:0 | 0.00 | 0.00 | 0.00 | 0.00 |
| % 22:1 | 0.00 | 0.00 | 0.00 | 0.00 |
| % 22:2 | 0.00 | 0.00 | 0.00 | 0.00 |
| % 22:3 | 0.00 | 0.00 | 0.00 | 0.00 |
| % 22:4 n-6 | 0.00 | 0.00 | 0.00 | 0.00 |
| % 22:5 n-6 | 2.39 | 1.95 | 2.59 | 2.18 |
| % 22:5 n-3 | 0.73 | 0.79 | 0.80 | 0.68 |
| % 22:6 n-3 DHA | 59.18 | 54.31 | 59.89 | 56.39 |
| % 24:0 | 0.00 | 0.00 | 0.00 | 0.00 |
| % 24:1 | 0.00 | 0.00 | 0.00 | 0.00 |
| % Fat | 45.69 | 38.08 | 42.88 | 48.48 |
| % Unknown | 1.38 | 1.58 | 1.27 | 2.19 |

### Examples 8-15 below are prophetic examples.

### EXAMPLE 8

A 1000 mL three neck flask equipped with a mechanical stirrer, reflux condenser, addition funnel, thermowell, heating mantle, and nitrogen atmosphere is charged with 10 grams of a microbial oil that contains 30% by weight of triglycerides that contain approximately 60% by weight of DHA bonded via ester linkages thereto, 193 grams of ethanol, and 28 grams of water. Sodium hydroxide (80 grams of 50% solution) is added through the addition funnel. The resulting mixture is heated at 64°C for 145 minutes. Hydrochloric acid (84 mL of 12 N) is added over five minutes. An additional 14 mL of HCl is added in small portions until a pH of 2 is attained. Stirring is stopped and reaction solution is poured into a separatory funnel. The reaction solution is extracted with ethyl acetate (3x) and the organic fractions are combined and dried with magnesium sulfate. The magnesium sulfate is filtered away and the solvent is removed by rotary evaporation. A product oil is obtained that contains DHA in its free fatty acid form. The DHA product yield is 78% by weight of theory.

### EXAMPLE 9

One gram of a microbial oil that contains 40% by weight of triglycerides that contain 50% by weight of EPA bonded via ester linkages thereto is placed in a 10 mL borosilicate glass test tube. The test tube is flooded with nitrogen gas, stoppered, and is cooled to -80°C. The test tube is allowed to stay at -80°C for 5 hours. At the end of the 5 hour period, a precipitate is formed and a separate oil layer is also present. The test tube is centrifuged for 2 minutes at 1000 g, and the oil is decanted off. The decanted product oil is isolated in 0.25 g and is found to contain 70% by weight of the triglycerides that contain approximately 50% by weight of EPA bonded via ester linkages thereto.

### EXAMPLE 10

One gram of an oil that contains 50 % by weight of the ethyl ester of EPA is placed in a 50 mL Erlenmeyer flask. 30 mL of methanol is added to the flask, and the resulting solution is heated to 67°C. The oil dissolves, and 6 g of urea is added to the solution. The urea also dissolves. The flask is then cooled to 5°C and allowed to remain at this temperature for 6 hours. A crystalline precipitate is visible in the flask. The methanol is removed from the flask by decantation and the precipitate is extracted 3x each with 25 mL of hexane. The hexane extracts are combined and the hexane is removed by rotary evaporation. A product oil is yielded in an amount of 0.3 g. The product oil contains 75% of the ethyl ester of EPA.

### EXAMPLE 11

0.5 g of an oil containing 50% by weight all-cis-4,7,10,13,16,19-docosahexaenoic alcohol is placed in a 75 mL 3 neck flask equipped with an addition funnel and a stir bar. The flask is placed under nitrogen and 20 mL of dichloromethane is added via syringe. Stirring is initiated and the oil dissolves. Solid Dess-Martin periodinane (DMP) is then added in an amount corresponding to 1.45 equivalents of (DMP) to 1 equivalent of all-cis-4,7,10,13,16,19-docosahexaenoic alcohol. Water is slowly added over the course of half of an hour via a syringe that is actuated by a syringe pump. The reaction is stopped after 1 hour and the resulting solution and solids contained therein are placed in a separatory funnel. Additional dichloromethane (30 mL) is added, and extraction is performed 3x with water containing 0.1 N NaOH. The dichloromethane is collected from the separatory funnel, dried over sodium sulfate, and filtered. Rotary evaporation of the dichloromethane yields 0.35 g of an oil containing 50% by weight of all-cis-4,7,10,13,16,19-docosahexaenoic aldehyde.

### EXAMPLE 12

An oil containing 50% tri-acyl glycerides where the tri-acyl glycerides contain 0.1 mol of an equimolar mixture of EPA and DHA in ester form is placed in a 500 mL round bottom flask equipped with a condenser. Ethanol (50 mL) is added and stirring is initiated. A NaOH solution is added such that the total amount of NaOH is equivalent to 0.11 mol. Water is added. The reaction is heated to 70°C and is kept in this state for 20 hours. The reaction is cooled. The flask is separated from reflux condenser, and the stir bar is removed from the flask. The flask is attached to a rotary evaporator and the water and ethanol are removed with heating and under reduced pressure. The resulting material is found to contain EPA and DHA in the sodium salt forms.

### EXAMPLE 13

100 g of an oil containing 50% by weight of triglycerides which contain DHA in 80% of the ester linkages therein is added to a 1000 mL flask equipped with a condenser. 500 mL of a sodium ethoxide ethanol solution is added, such that the molar ratio of sodium ethoxide to the ester bonds in the triglyceride is 30: 1. The reaction is heated to 77°C and allowed to stay at this temperature for 24 hours. The reaction is allowed to cool. A neutralizing amount of a 0.1 N HCl solution is added. The flask is separated from the condenser, the stir bar is removed, and the ethanol is removed under reduced pressure. The resulting water solution is extracted (3x) with ethyl acetate, and the ethyl acetate fractions are dried over sodium sulfate. The dried ethyl acetate fractions are filtered and subjected to rotary evaporation under reduced pressure. 18 g of an oil product are isolated. The oil product is found to be 93% pure and to contain the ethyl ester of DHA as its major component.

### EXAMPLE 14

80 g of a recycled urea adduct (containing approximately 3.5% by weight of fat - where the recycled urea adduct is previously extracted with supercritical CO₂ at a temperature of about 75 °C, a pressure of approximately 250 bar, and a flow rate of approximately 0.7 kg/hr, for about three hours), is dissolved in 88 g of ethanol and refluxed for 30 min. Then, 40 g of a sample containing fatty acids in the relative amounts listed in Table 4, PTA-10208 Sample #1, Crude Lipid, in their ethyl ester forms, is added. The resulting solution is heated at reflux for an additional 2 hours. The solution is subsequently cooled to 10 °C, and a urea adduct precipitates from a product oil-solvent combination. The urea adduct is removed by filtration and the solvent is evaporated from the product oil. The product oil weighs 18 g. The product oil is analyzed by gas chromatography and found to contain 85% of a combination of EPA and DHA in their ethyl ester forms, where the concentration of the ethyl ester of DHA in the product oil is 65% and the concentration of the ethyl ester of EPA in the product oil is 20%.

### EXAMPLE 15

A crude microbial oil ("CMO") is obtained as a starting oil. The CMO contains 95.1% by weight tri-acyl glycerides, based on the total weight of the crude microbial oil. The major fatty acids contained in the CMO include: EPA (19.7% by weight of the total fatty acids) and DHA (44.1% by weight of the total fatty acids). 3.8 g of the CMO is dissolved in 100 ml of an n-hexane/ethanol (10:90; v:v) solvent mixture to form a solvent-CMO solution. The solvent-CMO solution is then frozen for 8 hours at -85 °C, and subsequently is centrifuged for 2 minutes, at 3,600 rpm, at 0 °C. After centrifuging, supernatant is taken off using a Pasteur pipette, and the solvents (e.g., n-hexane and ethanol) are removed. Solvent removal produces 174.9 mg of a clear yellow oil product and 3.62 g of a turbid yellow oil residue. GC analysis (employing the method disclosed in U.S. Patent No. 7,588,791, at column 9, lines 4-17) for the product oil, gives an EPA content of 26.6% by weight of the total fatty acids, and a DHA content of 67.0 % by weight of the total fatty acids.

### EXAMPLE 16

This example illustrates a method of the present invention for extracting ethyl polyunsaturated fatty acids from the biomass of *Schizochytrium sp.,* ATCC Accession No. PTA-10208.

A biomass obtained from *Schizochytrium sp.* broth by centrifugation after mixing with ethanol was used directly without any further processing.

640 g of wet biomass (about 70% solids on weight basis), obtained by precipitation of whole broth, was mixed with 3000 mL of ethanol and stirred under nitrogen. 13 g of powdered potassium dydroxide was added to the stirring mixture and heated to reflux at about 90°C under nitrogen. The heating was continued for 8 hours. The reaction was monitored by TLC. No triglycerides (oil) was observed, only esters were observed. The reaction mixture was cooled and filtered to separate the ethanol solution of esters from spent biomass (biomeal). The ethanol solution was concentrated *in vacuo* to yield crude esters 268 g (>90% recovery based on the oil content of biomass). The left-over biomeal was dried to obtain 171 g spent biomeal. The crude esters were further enriched by either by fractional molecular distillation or urea adduction. A GC analysis of the biomass indicated the present of about 41% DHA ethyl ester, about 20% EPA ethyl ester, and about 4% DPA ethyl ester. The remaining materials were predominantly lower molecular weight ethyl esters (see **Table 33**)**.**

**Table 33. GC Analysis of Crude Ethly Esters**

| Sample | Crude Esters After Transesterifaction |
|---|---|
| % 22:6 (n-3) DHA | 41.5 |
| % 20:5 (n-3) EPA | 20.3 |
| % 22:5 (n-3) DPA | 4.2 |
| % Additional components | 34 |

### EXAMPLE 17

This example illustrates a method of the present invention for enriching crude esters by urea adduct crystallization.

60 g urea was dissolved in 500 mL of methanol while heating to reflux under nitrogen. 100 g of crude esters, obtained from direct esterification of biomass, were added to urea / methanol solution under nitrogen. The mixture was refluxed for about 2 hours and allowed to cool to room temperature with stirring. The mixture was further cooled to about 5 °C to complete crystallization. The solids were separated by filtration. The filter cake was washed with cold methanol to recover most of the suspended product on the urea adduct. The methanolic filtrate was concentrated *in vacuo* to obtain PUFA enriched concentrate with over 88% PUFAs (see **Table 34**)

**Table 34. GC Analysis of ethyl ester products after urea adduct crystallization**

| Sample | Crude Esters After Transesterifaction | Enriched DHA / DPA / EPA Ethyl Esters after Urea Adduction |
|---|---|---|
| % 22:6 (n-3) DHA | 41.5 | 54.9 |
| % 20:5 (n-3) EPA | 20.3 | 26.8 |
| % 22:5 (n-3) DPA | 4.2 | 5.3 |
| % Additional components | 34 | 13 |

### EXAMPLE 18

This example illustrates a method of the present invention for enriching crude esters by Vacuum Molecular Fractional Distillation.

The crude esters, obtained from direct esterification of biomass, were subjected to vacuum fractional distillation. The lower molecular weight ethyl esters were collected at temperatures between 100-150 °C and at a pressure of 0.8 to 0.9 mm Hg. The major components of this fraction were saturated C-14, and C-16 ethyl esters. The DHA ethyl ester / EPA ethyl ester / DPA ethyl ester mixture was collected at temperatures between 145-170 °C and at a pressure of about 0.5 mm Hg. A GC analysis of the DHA/DPA/EPA ethyl ester fraction showed a combined purity of about 90% of all ethyl esters (see **Table 35**).

**Table 35. GC Analysis of GC Analysis of ethyl ester products after vacuum molecular fractional distillation**

| Sample | Crude Esters After Transesterifaction | Enriched DHA / DPA / EPA Ethyl Esters after Vacuum Molecular Fractional Distillation |
|---|---|---|
| % 22:6 (n-3) DHA | 41.5 | 55.6 |
| % 20:5 (n-3) EPA | 20.3 | 27.3 |
| % 22:5 (n-3) DPA | 4.2 | 5.25 |
| % Additional components | 34 | 12 |

All of the various aspects, embodiments, and options described herein can be combined in any and all variations.

## Claims

1. An enriched microbial oil comprising at least 70% by weight of a combination of DHA and EPA, wherein the oil comprises at least 10% by weight of EPA and wherein
(i) the oil comprises at least 50% by weight DHA and at least 20% by weight EPA; or
(ii) the oil comprises a triacylglycerol fraction, wherein at least 50% by weight of the fatty acids in the triacylglycerol fraction is DHA and at least 20% by weight of the fatty acids in the triacylglycerol fraction is EPA.

2. The oil according to claim 1, comprising at least 50% by weight DHA and at least 20% by weight EPA.

3. The oil according to claim 1, wherein the oil comprises a triacylglycerol fraction, wherein at least 50% by weight of the fatty acids in the triacylglycerol fraction is DHA and at least 20% by weight of the fatty acids in the triacylglycerol fraction is EPA.

4. The oil of any preceding claim, wherein the DHA and EPA are in an ester form.

5. The oil according to claim 4wherein the DHA ester and the EPA ester are ethyl esters.

6. The oil of any preceding claim, which is obtainable from
(i) an isolated microorganism of the species deposited under ATCC Accession No. PTA-10208, wherein the total fatty acids produced by the microorganism comprise more than 10% by weight EPA;
(ii) an isolated microorganism having the characteristics of the species deposited under ATCC Accession No. PTA-10208, wherein the total fatty acids produced by the microorganism comprise more than 10% by weight EPA;
(iii) an isolated microorganism that produces a triacylglycerol fraction, wherein EPA content of the triacylglycerol fraction is at least 12% by weight;
(iv) an isolated microorganism deposited under ATCC Accession No. PTA-10208;
(v) an isolated microorganism deposited under ATCC Accession No. PTA-10209;
(vi) an isolated microorganism deposited under ATCC Accession No. PTA-10210; or
(vii) from an isolated microorganism deposited under ATCC Accession No. PTA-10211.

7. A method for obtaining a food or supplement, said method comprising incorporating the oil of any one of claims 1-6 in said food or supplement.

8. A method for obtaining a pharmaceutical composition, said method comprising incorporating the oil of any one of claims 1-6 and a pharmaceutically acceptable carrier in said pharmaceutical composition.

9. A method for obtaining an oral dosage form, said method comprising incorporating the oil of any one of claims 1-6 in said oral dosage form, preferably wherein the oral dosage form comprises 100 mg to 3000 mg of omega-3 polyunsaturated fatty acids, preferably 500 mg to 1000 mg of a combination of DHA and EPA.

10. The method according to claim 9, wherein said oral dosage form is a capsule or a vegetarian capsule.

11. An oil of any one of claims 1 to 6, or a mixture thereof, and a pharmaceutically acceptable carrier, for use in treating a heart disease or a condition related thereto in a subject in need thereof.

12. An oil of any one of claims 1 to 6, or mixtures thereof, for use in improving a heart condition or a condition related thereto, or maintaining a healthy heart condition or a condition related thereto.

13. A method for producing the enriched microbial oil of claim 1 from the biomass of a microorganism, comprising:
a) heating and reacting the biomass in the presence of an alcohol and a base to produce an ester of a polyunsaturated fatty acid from the biomass;
b) separating the biomass into a substantially liquid phase, enriched with polyunsaturated fatty acid, and a substantially solid phase; and
c) purifying the substantially liquid phase by recovering a fraction comprising the ester of polyunsaturated fatty acid.

14. The method of claim 13, wherein the microorganism is (i) an isolated microorganism of a *Schizochytrium* species or (ii) any one of isolated microorganisms of the species deposited under ATCC Accession Nos.: PTA-10212, PTA-10213, PTA-10214, PTA-10215, PTA-10208, PTA-10209, PTA-10210, PTA-10211, a mutant strain thereof, or a mixture thereof.

15. The method of any one of claims 13-14, wherein
(i) the heating step a) is conducted from 80 °C to 100 °C, preferably at 90 °C;
(ii) the base is potassium hydroxide;
(iii) the alcohol is ethanol;
(iv) the ester is an ethyl ester of the polyunsatured fatty acid;
(v) the polyunsatured fatty acid is a combination of DHA and EPA;
(vi) the separating step b) is conducted by centrifugation or filtration;
(vii) the purifying step c) is conducted by urea adduction crystallization in ethanol; and/or
(viii) the purifying step c) is conducted by vacuum molecular fractional distillation.

## Patentansprüche

1. Angereichertes mikrobielles Öl, umfassend wenigstens 70 Gew.-% an einer Kombination von DHA und EPA, wobei das Öl wenigstens 10 Gew.-% EPA umfasst und wobei
(i) das Öl wenigstens 50 Gew.-% DHA und wenigstens 20 Gew.-% EPA umfasst; oder
(ii) das Öl eine Triacylglycerolfraktion umfasst, wobei wenigstens 50 Gew.-% der Fettsäuren in der Triacylglycerolfraktion DHA sind und wenigstens 20 Gew.-% der Fettsäuren in der Triacylglycerolfraktion EPA sind.

2. Öl gemäß Anspruch 1, umfassend wenigstens 50 Gew.-% DHA und wenigstens 20 Gew.-% EPA.

3. Öl gemäß Anspruch 1, wobei das Öl eine Triacylglycerolfraktion umfasst, wobei wenigstens 50 Gew.-% der Fettsäuren in der Triacylglycerolfraktion DHA sind und wenigstens 20 Gew.-% der Fettsäuren in der Triacylglycerolfraktion EPA sind.

4. Öl gemäß einem der vorstehenden Ansprüche, wobei das DHA und EPA in einer Esterform vorliegen.

5. Öl gemäß Anspruch 4, wobei der DHA-Ester und der EPA-Ester Ethylester sind.

6. Öl gemäß einem der vorstehenden Ansprüche, das erhältlich ist von
(i) einem isolierten Mikroorganismus der Spezies, die unter der ATCC-Hinterlegungs-Nr. PTA-10208 hinterlegt ist, wobei die gesamten von dem Mikroorganismus erzeugten Fettsäuren mehr als 10 Gew.-% EPA umfassen;
(ii) einem isolierten Mikroorganismus mit den Eigenschaften der Spezies, die unter der ATCC-Hinterlegungs-Nr. PTA-10208 hinterlegt ist, wobei die gesamten von dem Mikroorganismus erzeugten Fettsäuren mehr als 10 Gew.-% EPA umfassen;
(iii) einem isolierten Mikroorganismus, der eine Triacylglycerolfraktion erzeugt, wobei der EPA-Gehalt der Triacylglycerolfraktion wenigstens 12 Gew.-% beträgt;
(iv) einem isolierten Mikroorganismus, der unter der ATCC-Hinterlegungs-Nr. PTA-10208 hinterlegt ist;
(v) einem isolierten Mikroorganismus, der unter der ATCC-Hinterlegungs-Nr. PTA-10209 hinterlegt ist;
(vi) einem isolierten Mikroorganismus, der unter der ATCC-Hinterlegungs-Nr. PTA-10210 hinterlegt ist; oder
(vii) von einem isolierten Mikroorganismus, der unter der ATCC-Hinterlegungs-Nr. PTA-10211 hinterlegt ist.

7. Verfahren zur Herstellung eines Lebensmittels oder Ergänzungsmittels, wobei das Verfahren Einverleiben des Öls gemäß einem der Ansprüche 1-6 in das Lebensmittel oder Ergänzungsmittel umfasst.

8. Verfahren zur Herstellung einer pharmazeutischen Zusammensetzung, wobei das Verfahren Einverleiben des Öls gemäß einem der Ansprüche 1-6 und eines pharmazeutisch verträglichen Trägers in die pharmazeutische Zusammensetzung umfasst.

9. Verfahren zur Herstellung einer oralen Darreichungsform, wobei das Verfahren Einverleiben des Öls gemäß einem der Ansprüche 1-6 und in die orale Darreichungsform umfasst, wobei die orale Darreichungsform vorzugsweise 100 mg bis 3000 mg mehrfach ungesättigte Omega-3-Fettsäuren umfasst, vorzugsweise 500 mg bis 1000 mg einer Kombination von DHA und EPA.

10. Verfahren gemäß Anspruch 9, wobei die orale Darreichungsform eine Kapsel oder eine vegetarische Kapsel ist.

11. Öl gemäß einem der Ansprüche 1 bis 6 oder ein Gemisch davon und ein pharmazeutisch verträglicher Träger zur Verwendung bei der Behandlung einer Herzerkrankung oder eines damit verbundenen Zustands bei einem Subjekt mit Bedarf daran.

12. Öl gemäß einem der Ansprüche 1 bis 6 oder Gemische davon zur Verwendung zum Verbessern eines Herzzustands oder eines damit verbundenen Zustands oder Aufrechterhalten eines gesunden Herzzustands oder eines damit verbundenen Zustands.

13. Verfahren zur Herstellung des angereicherten mikrobiellen Öls gemäß Anspruch 1 aus der Biomasse eines Mikroorganismus, umfassend:
a) Erhitzen und Umsetzen der Biomasse in Gegenwart eines Alkohols und einer Base, um einen Ester einer mehrfach ungesättigten Fettsäure aus der Biomasse zu erzeugen;
b) Trennen der Biomasse in eine im Wesentlichen flüssige Phase, die mit mehrfach ungesättigter Fettsäure angereichert ist, und eine im Wesentlichen feste Phase; und
c) Reinigen der im Wesentlichen flüssigen Phase durch Gewinnen einer Fraktion, die den Ester von mehrfach ungesättigter Fettsäure umfasst.

14. Verfahren gemäß Anspruch 13, wobei der Mikroorganismus (i) ein isolierter Mikroorganismus einer *Schizochytrium-*Spezies oder (ii) einer von isolierten Mikroorganismen der Spezies, die unter den ATCC-Hinterlegungs-Nrn. PTA-10212, PTA-10213, PTA-10214, PTA-10215, PTA-10208, PTA-10209, PTA-10210, PTA-10211 hinterlegt sind, ein Mutantenstamm davon oder ein Gemisch davon ist.

15. Verfahren gemäß einem der Ansprüche 13-14, wobei
(i) der Schritt des Erhitzens a) auf 80 °C bis 100 °C durchgeführt wird, vorzugsweise 90 °C;
(ii) die Base Kaliumhydroxid ist;
(iii) der Alkohol Ethanol ist;
(iv) der Ester ein Ethylester der mehrfach ungesättigten Fettsäure ist;
(v) die mehrfach ungesättigte Fettsäure eine Kombination von DHA und EPA ist;
(vi) der Schritt des Trennens b) durch Zentrifugation oder Filtration durchgeführt wird;
(vii) der Reinigungsschritt c) durch Harnstoffadduktionskristallisation in Ethanol durchgeführt wird; und/oder
(viii) der Reinigungsschritt c) durch fraktionelle Vakuummolekulardestillation durchgeführt wird.

## Revendications

1. Huile microbienne enrichie, comprenant au moins 70% en poids d'une combinaison de DHA et d'EPA, l'huile comprenant au moins 10% en poids d'EPA et où
(i) l'huile comprend au moins 50% en poids de DHA et au moins 20% en poids d'EPA ; ou
(ii) l'huile comprend une fraction de triacylglycérol, où au moins 50% en poids des acides gras dans la fraction de triacylglycérol sont constitués de DHA et au moins 20% en poids des acides gras dans la fraction de triacylglycérol sont constitués d'EPA.

2. Huile selon la revendication 1, comprenant au moins 50% en poids de DHA et au moins 20% en poids d'EPA.

3. Huile selon la revendication 1, l'huile comprenant une fraction de triacylglycérol, où au moins 50% en poids des acides gras dans la fraction de triacylglycérol sont constitués de DHA et au moins 20% en poids des acides gras dans la fraction de triacylglycérol sont constitués d'EPA.

4. Huile selon l'une quelconque des revendications précédentes, dans laquelle le DHA et l'EPA se trouvent sous une forme d'ester.

5. Huile selon la revendication 4, dans laquelle l'ester de DHA et l'ester d'EPA sont des éthylesters.

6. Huile selon l'une quelconque des revendications précédentes, qui peut être obtenue à partir
(i) d'un microorganisme isolé de l'espèce déposée sous le numéro d'accès ATCC PTA-10208, où les acides gras totaux produits par le microorganisme comprennent plus de 10% en poids d'EPA ;
(ii) d'un microorganisme isolé ayant les caractéristiques de l'espèce déposée sous le numéro d'accès ATCC PTA-10208, où les acides gras totaux produits par le microorganisme comprennent plus de 10% en poids d'EPA ;
(iii) d'un microorganisme isolé qui produit une fraction de triacylglycérol, où la teneur en EPA de la fraction de triacylglycérol est d'au moins 12% en poids ;
(iv) d'un microorganisme isolé déposé sous le numéro d'accès ATCC PTA-10208 ;
(v) d'un microorganisme isolé déposé sous le numéro d'accès ATCC PTA-10209 ;
(vi) d'un microorganisme isolé déposé sous le numéro d'accès ATCC PTA-10210 ; ou
(vii) d'un microorganisme isolé déposé sous le numéro d'accès ATCC PTA-10211.

7. Méthode d'obtention d'un aliment ou d'un complément, ladite méthode comprenant l'incorporation de l'huile selon l'une quelconque des revendications 1-6 dans ledit aliment ou complément.

8. Méthode d'obtention d'une composition pharmaceutique, ladite méthode comprenant l'incorporation de l'huile selon l'une quelconque des revendications 1-6 et d'un véhicule pharmaceutiquement acceptable dans ladite composition pharmaceutique.

9. Méthode d'obtention d'une forme de dosage oral, ladite méthode comprenant l'incorporation de l'huile selon l'une quelconque des revendications 1-6 dans ladite forme de dosage oral, préférablement où la forme de dosage oral comprend de 100 mg à 3000 mg d'acides gras polyinsaturés oméga-3, préférablement de 500 mg à 1000 mg d'une combinaison de DHA et d'EPA.

10. Méthode selon la revendication 9, dans laquelle ladite forme de dosage oral est une capsule ou une capsule végétarienne.

11. Huile selon l'une quelconque des revendications 1 à 6, ou un mélange de celle-ci, et un véhicule pharmaceutiquement acceptable, pour une utilisation destinée au traitement d'une maladie cardiaque ou d'une condition y étant apparentée, chez un sujet en ayant besoin.

12. Huile selon l'une quelconque des revendications 1 à 6, ou des mélanges de celle-ci, pour une utilisation destinée à l'amélioration d'une condition cardiaque, ou d'une condition y étant apparentée, ou au maintien d'une condition cardiaque en bonne santé, ou d'une condition y étant apparentée.

13. Méthode de production de l'huile microbienne enrichie selon la revendication 1 à partir de la biomasse d'un microorganisme, comprenant :
a) le chauffage et la réaction de la biomasse en présence d'un alcool et d'une base, afin de produire un ester d'un acide gras polyinsaturé à partir de la biomasse ;
b) la séparation de la biomasse en une phase sensiblement liquide, enrichie en un acide gras polyinsaturé, et une phase sensiblement solide ; et
c) la purification de la phase sensiblement liquide par la récupération d'une fraction comprenant l'ester d'acide gras polyinsaturé.

14. Méthode selon la revendication 13, dans laquelle le microorganisme est (i) un microorganisme isolé d'une espèce de *Schizochytrium* ou (ii) l'un quelconque parmi les microorganismes isolés des espèces déposées sous les numéros d'accès ATCC PTA-10212, PTA-10213, PTA-10214, PTA-10215, PTA-10208, PTA-10209, PTA-10210, PTA-10211, une souche mutante de celles-ci, ou un mélange de celles-ci.

15. Méthode selon l'une quelconque des revendications 13-14, dans laquelle
(i) l'étape de chauffage a) est effectuée à une température allant de 80°C à 100°C, préférablement à 90°C ;
(ii) la base est constituée d'hydroxyde de potassium ;
(iii) l'alcool est constitué d'éthanol ;
(iv) l'ester est constitué d'un éthylester de l'acide gras polyinsaturé ;
(v) l'acide gras polyinsaturé est constitué d'une combinaison de DHA et d'EPA ;
(vi) l'étape de séparation b) est effectuée par centrifugation ou filtration ;
(vii) l'étape de purification c) est effectuée par cristallisation par adduction d'urée dans l'éthanol ; et/ou
(viii) l'étape de purification c) est effectuée par distillation fractionnée sous vide moléculaire.


### SEQUENCE LISTING

<110> RAMAN, Krishna
<120> Microbial Oils Enriched in Polyunsaturated Fatty Acids
<130> 28744-EP-EPT
<140> PCT/US2012/047730
   <141> 2012-07-20
<150> 61/510,454
   <151> 2011-07-21
<160> 2
<170> PatentIn version 3.3
<210> 1
   <211> 1705
   <212> DNA
   <213> Thraustochytrium sp.
<400> 1
   aagatacctg gttgatcctg ccagtagtca tacgctcgtc tcaaagatta agccatgcat      60
   gtgtcagtat aaatactttt actttgaaac tgcgaacggc tcattaaatc agtaattatc     120
   tacatggtaa cgaaaattat atggataacc gtagtaattc tagggctaat acatgcgtaa     180
   aatctgggta actggatgca tttattggat tgaagccaac attaaaaggt gattcacgat     240
   aactaagcgg agcgttttag gacgctgaat cattcgagtt tctgccctat cagctgtcga     300
   tggtaaggta ttggcttacc atggcgttaa cgggtaacgg agaattaggg ttcgattccg     360
   gagagggagc ctgagagacg gctaccacat ccaaggaagg cagcaggcgc gtaaattgcc     420
   caatgagaac ttctcgaggc agtgacaaga aatatcaaag tgatgccgtt aggtattgca     480
   tttgaaatga gaacgatgta caacttctaa cgatgatcaa ttggagggca agtctggtgc     540
   cagcagccgc ggtaattcca gctccaatag cgtatactaa cgttgctgca gttaaaacgc     600
   ccgtagttga attagtatca tggtatttta accttattcg atgaatttga gttgaaagct     660
   aggatatata ggaagcgatt cctcatttac tgtaaaaaaa ttagagtgtt tcacacagat     720
   cgtaagatcg ggatatatta gtatggaata ataagatagg actttggtgc tattttgttg     780
   gtttgcacac caaagtaatg attaataggg acagttgggg gtattcgtat ttaattgtca     840
   gaggtgaaat tcttggattt atgaaagacg aactactgcg aaagcattta ccaaggatgt     900
   tttcattaat caaggacgaa agttagggga tcgaagatga ttagatacca tcgtagtctt     960
   aaccataaac tatgccgact gaggattctt gaaatttgta aatgaattta agagcactcc    1020
   atgagaaatc aaagtctttg ggttccgggg ggagtatggt cgcaagtctg aaacttaaag    1080
   gaattgacgg aagggcacca ccaggagtgg agcctgcggc ttaatttgac tcaacacggg    1140
   aaaacttacc aggtccagac atagtgagga ttgacagatt gatagctctt tcttgattct    1200
   atgggtggtg gtgcatggcc gttcttagtt ggtggtttga actgtctgct taattgcgtt    1260
   aacgaacgag acctcagcct actaaatagt atgttgttta gtaataaatg atatgacttc    1320
   ttagagggac atttcgggtt taccggaagg aagtttgagg caataacagg tctgtgatgc    1380
   ccttagatgt tctgggccgc acgcgcgcta cactgacgag ctcaacaagt aatatttggt    1440
   tgtctggaag gattgcctaa tcttttaaat actcgtcgtg atggggctag attcttgtaa    1500
   ttattaatct ccaacgagga attcctagta aacgcaagtc atcagcttgc attgattacg    1560
   tccctgccct ttgtacacac cgcccgtcgc acctaccgat tggatggtcc ggtgaaatct    1620
   tcggatgttt ttttacaata gtagagagac aaaagttgag taaaccttac catctagagg    1680
   aaggtgaagt cgtaacaagg atctt                                          1705
<210> 2
   <211> 1713
   <212> DNA
   <213> Schizochytrium sp.
<400> 2
   acctggttga tcctgccagc tgtcatttgc tcgtctaaaa gattaagcca tgcatgtcta      60
   agtataaaca aattatacgg tgaaactgcg aacggctcat tatatcagtt atagtttctt     120
   tgatagtgta tttctatatc tatttggata actgtggcaa ttctagagct aacacatgct     180
   ttcgagtggg actttttggt accactgcat ttattagatt ttgaagccaa cgtaaaattg     240
   gtgattcatg ataactttgc gaatcgcagt agcgtcttgt acgcggcgat gaatcattca     300
   agtttctgcc ccatcagctg tcgatggtac ggtattggcc taccatggct ttcacgggtg     360
   acggagaatt agggtttgat tccggagagg acgcttgaga gacggcgacc acatccaagg     420
   aaggcagcag gcgcgtaaat tacccaatgg ggactccccg aggtagtgac aagaaataaa     480
   aatgaggagc gctttgcgtt tttcaatttg aatgagagaa tcgtacaatc ctcatcgagg     540
   atcaattgga gggcaagtct ggtgccagca gccgcggtaa ctccagctcc aatagcaaat     600
   attagagttg ttgcagttaa aaagctcgta gttgaatttc cgatagtctt tggccgtgtc     660
   cttggtctcg tatcatgggt ttattgtgcc aagatgatcg tcctctatgg ttagtgatag     720
   tcatagtcgt ttactgtaaa aaaactggag tgtttaaagc atttctttgg gaaaggtaca     780
   tattagtata ggataattag ataggacctg tgattcttat ttggttggtt tgtgagtcat     840
   ggtaatgatt aatagggaca atcgggggta ttcgaattta attgtcagag gtgaaattct     900
   tggatttaag aaagtcgaac tactgcgaag gcatttacca aggatgtttt cattaataaa     960
   gaacgaaagt taggggatcg aagatgatta gataccatcg tagtcttaac tgtaaactat    1020
   gccgacttgc gattgtccgt cgttgttttt tcaaaaaaga gacctgggca gcagcacatg    1080
   agaaatcaaa gtttttgggt tccgggggga gtatggtcgc aaggctgaaa cttaaaggaa    1140
   ttgacggaag ggcaccacca ggagtggagc ctgcggctta attcgactca acacgggaaa    1200
   acttaccagg tccagacata gtaaggattg acagattgag agctctttct tgattctatg    1260
   ggtggtggtg catggccgtt cttagttggt ggagtgattt gtctggttaa ttccgttaac   1320
   gaacgagacc tcagcctact aaatagtggt gcatattgtg agatatgtga caaaaatcgc   1380
   ttcttagagg gacatttcgg gtttaccgga aggaagtttg aggcaataac aggtctgtga   1440
   tgcccctaga tgttctgggc cgcacgcgcg ctacaatgac agattcaaca agtccggtag   1500
   tggagctttt gcttctctat tattactttt ccgagaggaa tggttaatct tctaaatgtc   1560
   tgtcgtgatg gggctagatt tttgcaatta ttaatctcca acgaggaatt cctagtaaac   1620
   gcaagtcatc agcttgcatt gattacgtcc ctgccctttg tacacaccgc ccgtcgcacc   1680
   taccgattga acggtcctat gaaatcttcg gat                                1713